# EUROPEAN PATENT SPECIFICATION

(11) **EP 2 447 250 B1**
(45) Date of publication and mention of the grant of the patent: **18.09.2013**
(21) Application number: 11183421.4
(22) Date of filing: 30.09.2011
(51) Int. Cl.: C07D 209/80, C07D 401/10, C07D 401/12, C07D 401/14, C07D 403/12, C07D 405/12, C07D 409/04, C07D 409/10, C07D 409/12, H01L 51/50

(54) **Organic light-emitting device**
Organische lichtemittierende Vorrichtung
Dispositif électroluminescent organique

(30) Priority: 26.10.2010 KR 20100104736
(43) Date of publication of application: 02.05.2012
(73) Proprietor: Samsung Display Co., Ltd., Yongin-City, Gyeonggi-Do, 446-711 (KR)
(72) Inventor: Kim, Young-Kook, 446-711 Yongin-City (KR); Hwang, Seok-Hwan, 446-711 Yongin-city (KR); Jung, Hye-Jin, 446-711 Yongin-city (KR); Lim, Jin-O, 446-711 Yongin-city (KR); Han, Sang-Hyun, 446-711 Yongin-city (KR); Kwak, Yoon-Hyun, 446-711 Yongin-city (KR); Lee, Sun-Young, 446-711 Yongin-city (KR); Lee, Jong-Hyuk, 446-711 Yongin-city (KR); Kim, Sung-Chul, 446-711 Yongin-city (KR)
(74) Representative: Gulde Hengelhaupt Ziebig & Schneider

(56) References cited:
- WO-A1-2007/004799
- WO-A2-2010/074520
- WO-A2-2010/114264
- JP-A- 2010 073 987
- US-A1- 2006 177 691
- US-A1- 2008 124 455

## Description

### BACKGROUND OF THE INVENTION

### Field of the Invention

The present invention relates to a heterocyclic compound represented by Formula 1 and an organic light-emitting device including the heterocyclic compound.

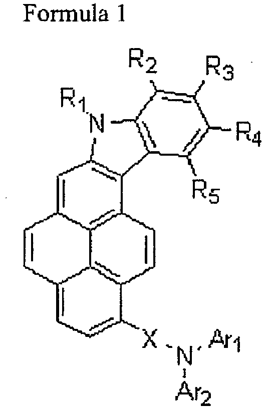

### Description of the Related Art

Light-emitting devices are self-emitting display devices and have a wide viewing angle, a high contrast ratio, and a short response time. Due to these characteristics, light-emitting devices (OLEDs) are drawing more attention.

Documents WO 2007/004799 A, US 2006/177691 A, WO 2010/114264 A, US 2008/0124455 A, WO 2010/074520 A and JP 2010/073987 A disclose OLED compositions which contain pyrene derivatives. In WO 2007/004799 A, the pyrene is fused to two pyrrole rings, each of which is N-substituted by phenyl and substituted by naphthyl at the 2-position of the indole rings. US 2006/177691 A discloses carbazoles which bear two pyrene substituents and which are N-substituted by phenyl. WO 2010/114264 A discloses an example of pyrene fused to an indole ring which is N-substituted by (4,6-diphenyl)-pyrimidin-2-yl. US 2008/0124455 A discloses pyrene fused to an indole moiety which is N-substituted by 1,1'-biphenyl-4-yl. WO 2010/074520 A discloses pyrene which is fused to two indole moieties. JP 2010/073987 A discloses pyrene fused to an indole moiety which is N-substituted by phenyl and further substituted on the indole ring by diphenylamino.

### SUMMARY OF THE INVENTION

The present invention provides heterocyclic compounds having improved electrical characteristics, charge transporting capabilities, light-emission capabilities, and a high glass-transition temperature that is high enough to prevent crystallization.

The present invention provides an organic light-emitting device including the heterocyclic compound.

The present invention provides a flat panel display device including the organic light-emitting device.

According to an aspect of the present invention, there is provided a heterocyclic compound represented by Formula 1 below: 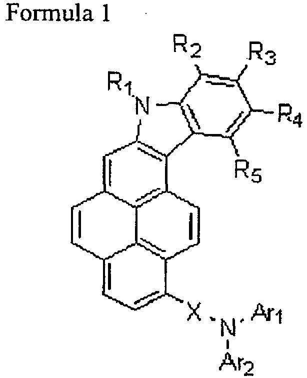 wherein, in Formula 1, R₁ through R₅ are each independently a hydrogen atom, a deuterium atom, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C3-C60 cycloalkyl group, a substituted or unsubstituted C₁-C₆₀ alkoxy group, a substituted or unsubstituted C₅-C₆₀ aryloxy group, a substituted or unsubstituted C₅-C₆₀ arylthio group, a substituted or unsubstituted C₅-C₆₀ aryl group, a amino group substituted with a C₅-C₆₀ aryl or C₃-C₆₀ heteroaryl group, a substituted or unsubstituted C₃-C₆₀ heteroaryl group, a substituted or unsubstituted C₆-C₆₀ condensed polycyclic group, a halogen atom, a cyano group, a nitro group, a hydroxyl group, or a carboxyl group;

Ar₁ and Ar₂ are each independently a substituted or unsubstituted C₁-C₆₀ alkyl group, a substituted or unsubstituted C₃-C₅₀ cycloalkyl group, a substituted or unsubstituted C₅-C₆₀ aryloxy group, a substituted or unsubstituted C₅-C₆₀ arylthio group, a substituted or unsubstituted C₅-C₆₀ aryl group, an amino group substituted with a C₅-C₆₀ aryl or C₃-C₆₀ heteroaryl group, a substituted or unsubstituted C₃-C₆₀ heteroaryl group, or a substituted or unsubstituted C₆-C₆₀ condensed polycyclic group; and

X is a divalent linking group represented by -(Ar₃)ₙ- where Ar₃ is a substituted or unsubstituted C₅-C₆₀ arylene group, a substituted or unsubstituted C₃-C₆₀ heteroarylene group, or a substituted or unsubstituted C₆-C₆₀ condensed polycyclic group, and n is an integer from 1 to 10, wherein *n* groups of Ar₃ are identical to or different from each other, and at least two adjacent groups of the *n* Ar₃ groups are fused or linked to each other by a single bond,
wherein the term "substituted" refers to the substitution of at least one hydrogen atom of the respective moiety with heavy hydrogen atom, a halogen atom, a hydroxyl group, a nitro group, a cyano group, an amino group, an amidino group, hydrazine, hydrazone, a carboxyl group or a salt thereof, a sulfonic acid group or a salt thereof, a phosphoric acid group or a salt thereof, a C₁-C₁₀alkyl group, a C₁-C₁₀alkoxy group, a C₂-C₁₀alkenyl group, a C₂-C₁₀alkynyl group, a C₆-C₁₆aryl group, or a C₃-C₁₆heteroaryl group.

In Formula 1 above, R₁ to R₅ may be each independently a hydrogen atom, a deuterium atom, a cyano group, a halogen atom, an unsubstituted C₁-C₂₀ alkyl group, a substituted C₁-C₂₀ alkyl group with at least one fluorine (-F) substituent, a substituted or unsubstituted C₅-C₂₀ aryl group, or a substituted or unsubstituted C₃-C₂₀ heteroaryl group.

In Formula 1 above, R₁ to R₅ may be each independently a hydrogen atom, a deuterium atom, a halogen atom, a cyano group, a substituted or unsubstituted C₁-C₂₀ alkyl group, or groups represented by Formulae 2a to 2f below: 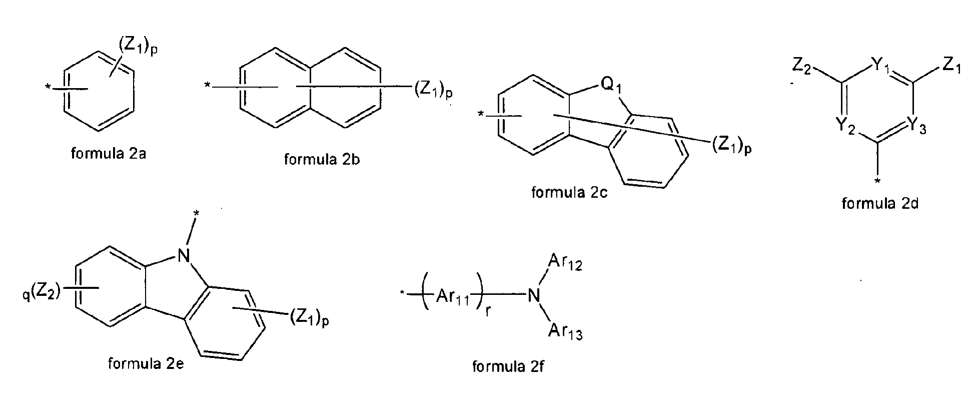 wherein, in Formulae 2a to 2f, Q₁ is a linking group represented by -C(R₆)(R₇)-, -N(R₆)-, -S-, or -O-;
Y₁, Y₂, and Y₃ are each independently a linking group represented by -N= or -C(R₈)=;
Z₁, Z₂, Ar₁₂, Ar₁₃, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
Ar₁₁ is a substituted or unsubstituted C₁-C₂₀ alkylene group, a substituted or unsubstituted C₅-C₂₀ arylene group, or a substituted or unsubstituted C₃-C₂₀ heteroarylene group;
p is an integer from 1 to 12;
q is an integer from 1 to 12;
r is an integer from 0 to 5; and
* indicates a binding site.

In Formula 1 above, R₁ to R₅ may be each independently a hydrogen atom, a deuterium atom, a halogen atom, a cyano group, a methyl group, an ethyl group, a propyl group, a butyl group, a pentyl group, or groups represented by Formulae 3a to 3h below: 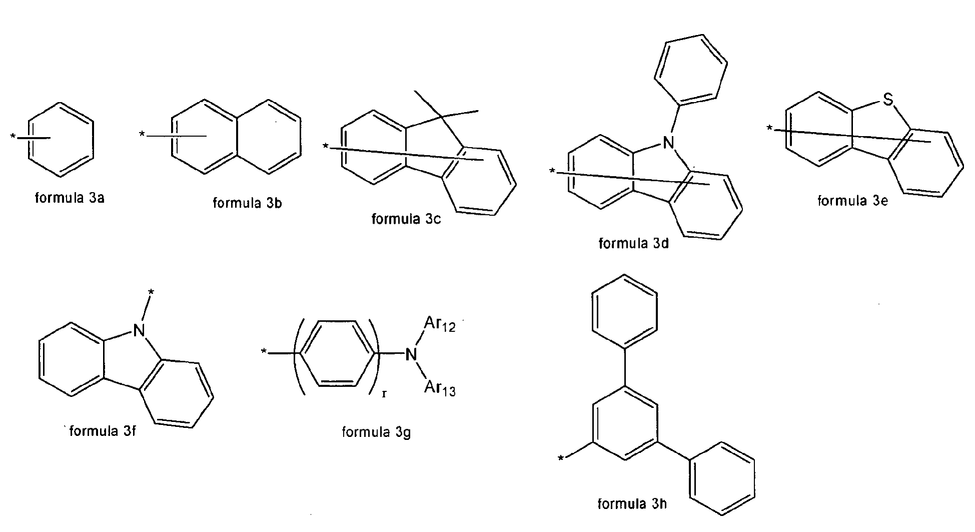 wherein in Formula 3a to 3h, Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group;
r is an integer from 0 to 2; and
* indicates a binding site.

In Formula 1 above, R₂ and R₅ may be hydrogen atoms; and R₁, R₃, and R₄ may be each independently a hydrogen atom, a deuterium atom, a halogen atom, a cyano group, a methyl group, an ethyl group, a propyl group, a butyl group, a pentyl group, or groups represented by Formulae 3a to 3h below: 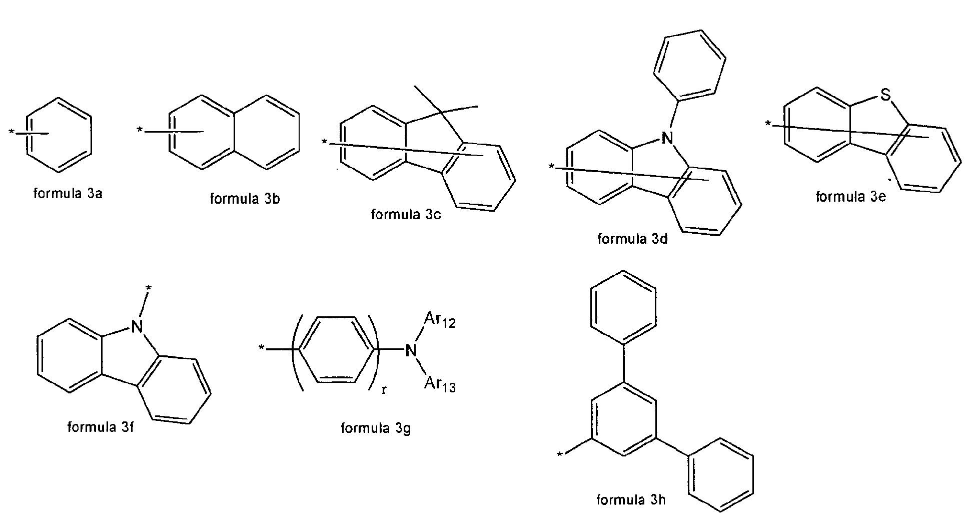 wherein, in Formula 3a to 3h, Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group; r is an integer from 0 to 2; and * indicates a binding site.

In Formula 1 above, R₂ and R₅ may be hydrogen atoms; and R₁, R₃ and R₄ may be each independently a substituted or unsubstituted C₁-C₂₀ alkyl group, a substituted or unsubstituted C₆-C₂₀ aryl group, or a substituted or unsubstituted C₃-C₂₀ heteroaryl group.

In Formula 1 above, Ar₁ and Ar₂ may be each independently a substituted or unsubstituted C₁-C₂₀ alkyl group, a substituted or unsubstituted C₅-C₂₀ aryl group, or a substituted or unsubstituted C₃-C₂₀ heteroaryl group.

In Formula 1 above, Ar₁ and Ar₂ may be each independently a group represented by one of Formulae 4a to 4d below: 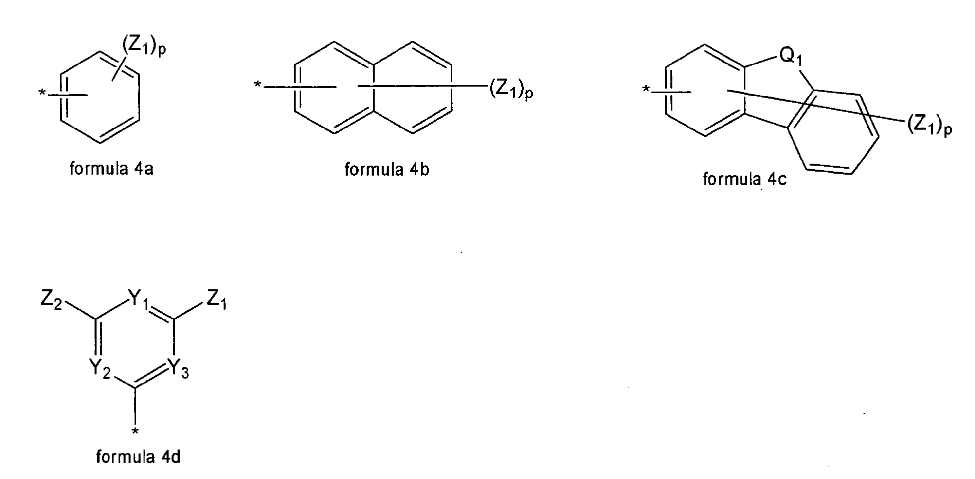 wherein, in Formula 4a to 4d, Q₁ is a linking group represented by -C(R₆)(R₇)-, -N(R₆)-, -S-, or -O-;
Y₁, Y₂, and Y₃ are each independently a linking group represented by -N= or -C(R₈)=;
Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
p is an integer from 1 to 8; and
* indicates a binding site.

In Formula 1 above, Ar₁ and Ar₂ may be each independently a group represented by one of Formulae 5a to 5i below: 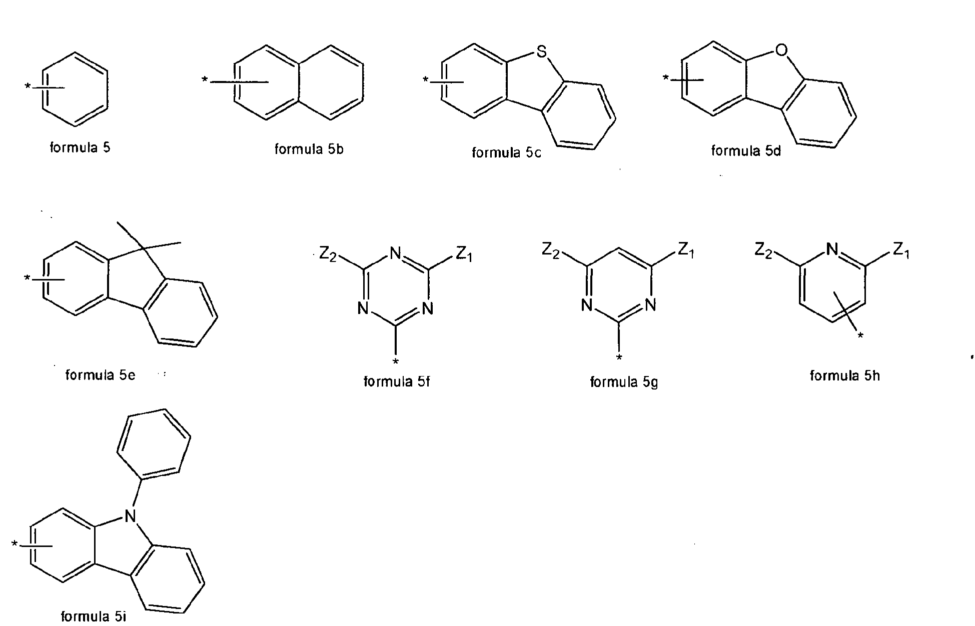 wherein Z₁ and Z₂ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, or a substituted or unsubstituted C₆-C₂₀ condensed polycyclic group; and * indicates a binding site.

Ar₃ for X in Formula 1 may be a substituted or unsubstituted C₅-C₂₀ arylene group, a substituted or unsubstituted C₃-C₂₀ heteroarylene group, or a substituted or unsubstituted C₆-C₂₀ condensed polycyclic group.

Ar₃ for X in Formula 1 may include a group represented by one of Formulae 6a to 6e below: 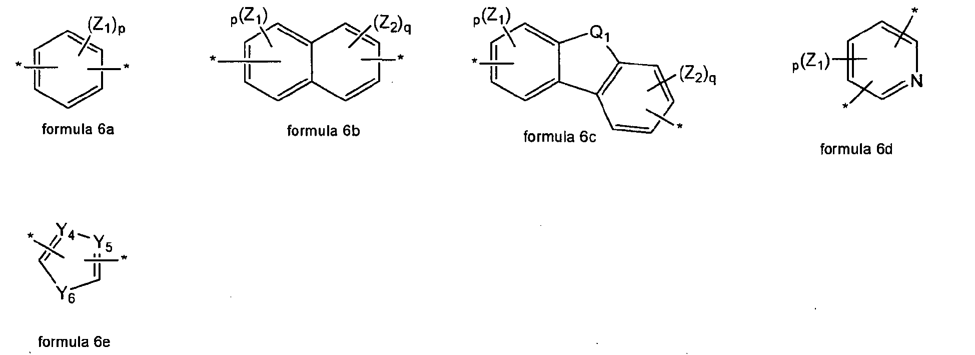 wherein, in Formula 6a to 6e, Q₁ is a linking group represented by -C(R₆)(R₇)-, -N(R₆)-,or -S-;
Y₄, Y₅, and Y₆ are each independently a linking group represented by -N= or -C(R₈)=, -S-, or -O-;
Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
p is an integer from 1 to 8;
q is an integer from 1 to 8; and
* indicates a binding site.
In Formula 1 above, n may be 1 or 2.

In Formula 1, X may include a group represented by one of Formulae 7a to 7j: 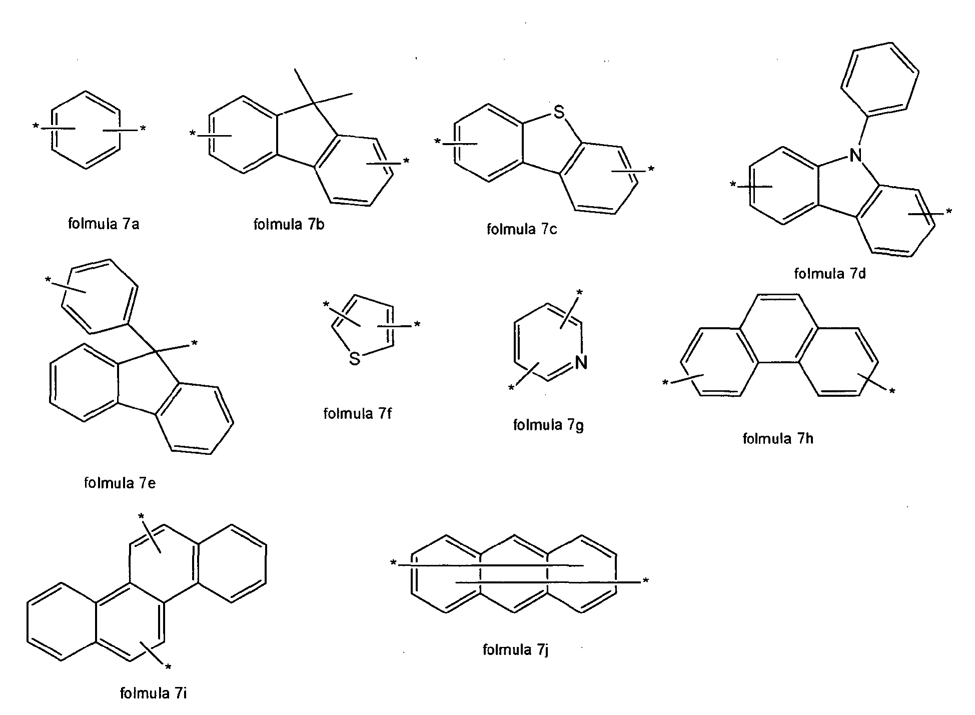 wherein, in Formula 7a to 7j, * indicates a binding site.

In Formula 1 above, R₁, R₃, and R₄ may be each independently a hydrogen atom, a deuterium atom, a halogen atom, a cyano group, a methyl group, an ethyl group, a propyl group, a butyl group, a pentyl group, or groups represented by Formulae 3a to 3h below: 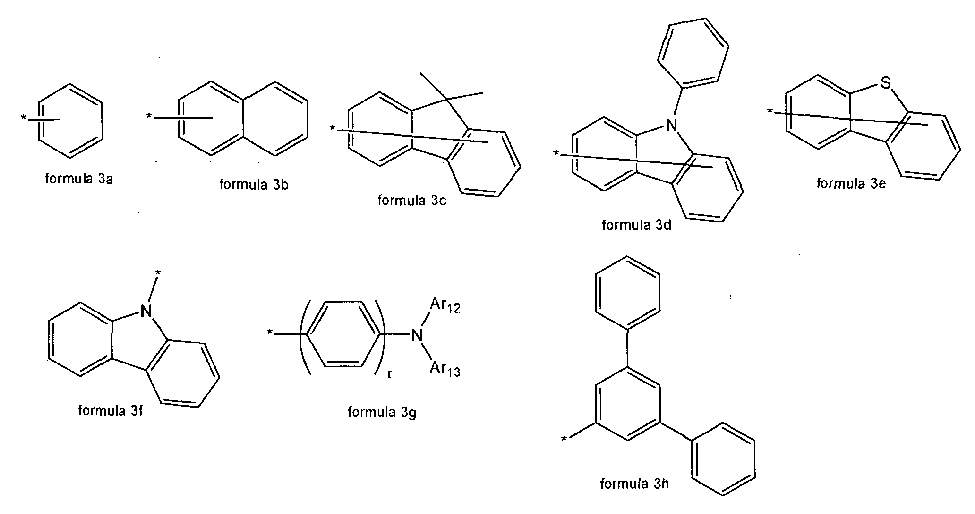 wherein, in Formula 3a to 3h, Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group; r is an integer from 0 to 2, and * indicates a binding site;
R₂ and R₅ are hydrogen atoms;
Ar₃ comprises a group represented by one of Formulae 6a to 6e below: 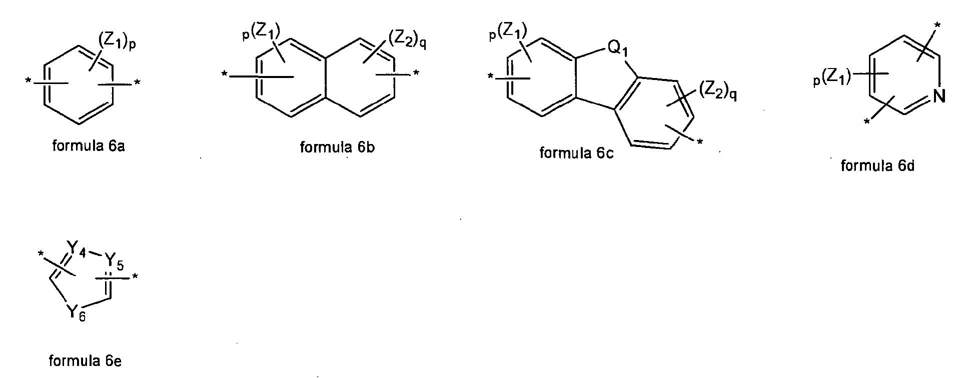 wherein, in Formulae 6a to 6e, Q₁ is a linking group represented by -C(R₆)(R₇)-, -N(R₆)-, or -S-; Y₄, Y₅ and Y₆ are each independently a linking group represented by -N=, -C(R₈)=, -S-, or -O-; Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group; p is an integer from 1 to 8; q is an integer from 1 to 8; and * indicates a binding site;
n in Formula 1 is 1 or 2; and

Ar₁ and Ar₂ are each independently selected from among groups represented by Formulae 4a to 4d below: 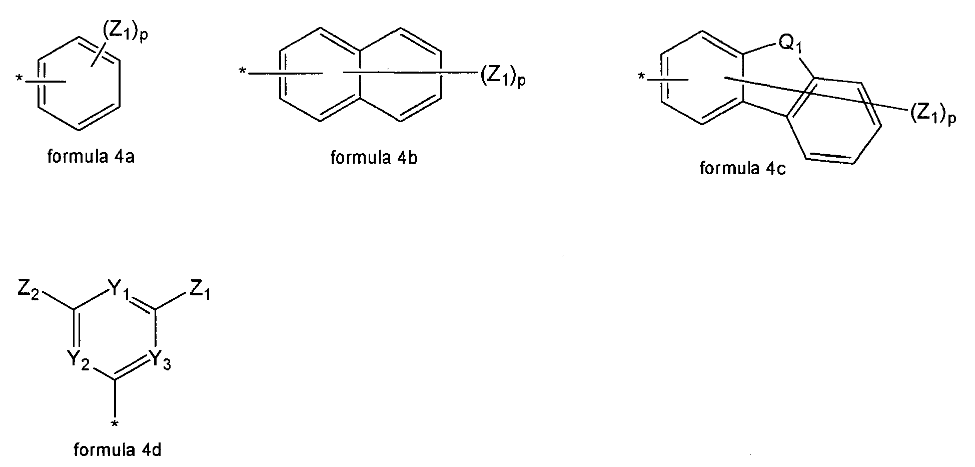 wherein, in Formulae 4a to 4d, Q₁ is a linking group represented by -C(R₆)(R₇)-, -N(R₆)-, -S-, or -O-; Y₁, Y₂ and Y₃ are each independently a linking group represented by -N= or -C(R₈)=; Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group; p is an integer from 1 to 8; and * indicates a binding site.

The heterocyclic compound may include one of the compounds below:

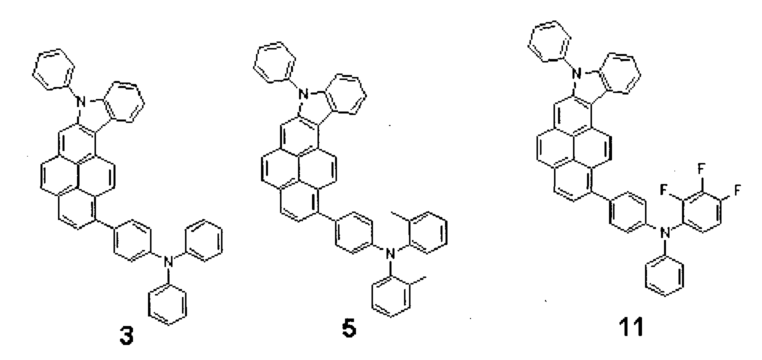

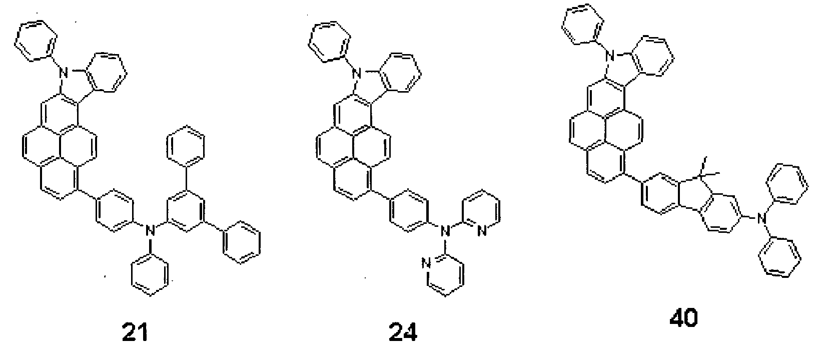

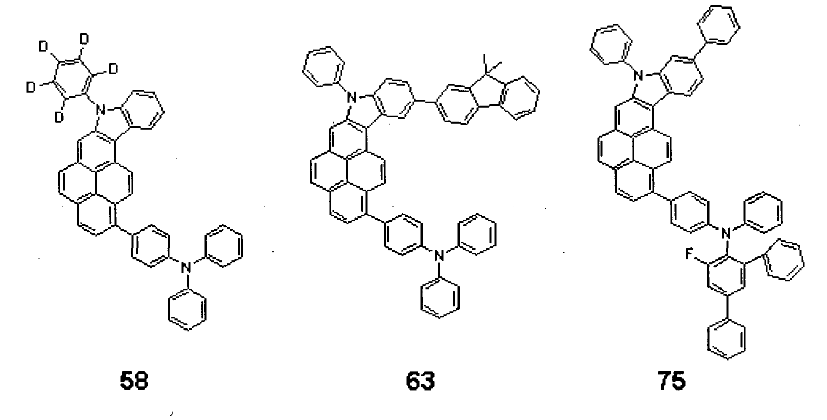

According to another aspect of the present invention, an organic light-emitting device includes: a first electrode; a second electrode; and an organic layer between the first electrode and the second electrode, wherein the organic layer includes a heterocyclic compound represented by Formula 1 above.

The organic layer may include a hole injection layer, a hole transport layer, a functional layer having both hole injection and hole transport capabilities, an electron injection layer, an electron transport layer, a functional layer having both electron injection and electron transport capabilities or any combination thereof.

The organic layer may include an emission layer, and the heterocyclic compound of Formula 1 may be used as a host or a dopant for a fluorescent or phosphorescent device.

The organic layer may include an emission layer, a hole transport layer, and an electron transport layer. The emission layer, the hole transport layer, or the electron transport layer may include the heterocyclic compound of Formula 1. The emission layer may include an anthracene compound, an arylamine compound, or a styryl compound.

The organic layer may include an emission layer, a hole transport layer, and an electron transport layer. The emission layer, the hole transport layer, or the electron transport layer may include the heterocyclic compound of Formula 1. The emission layer may include red, green, blue, and white emission layers, one of which includes a phosphorescent compound.

The organic layer may include a blue emission layer.

The organic layer may include a blue emission layer, and the heterocyclic compound of Formula 1 may be used as a blue dopant.

The organic layer may include at least one organic layer including the heterocyclic compound of Formula 1 above, the at least one organic layer being formed using a wet process.

The organic layer may further comprise at least one of a hole injection layer, a hole transport layer, a functional layer having both hole injection and transport functions, an emission layer, a hole blocking layer, an electron transport layer, an electron injection layer, and a combination of at least two of these layers. At least one of the hole injection layer, the hole transport layer, and the functional layer having both hole injection and transport functions may further comprise a charge-generating material. The electron transport layer may comprise an electron transporting organic material and a metal-containing material. The metal-containing material may comprise a lithium complex.

According to another aspect of the present invention, a flat panel display device includes the organic light-emitting device according to the one or more embodiments described above, wherein the first electrode of the organic light-emitting device may be electrically connected to a source electrode or a drain electrode of a thin-film transistor.

### BRIEF DESCRIPTION OF THE DRAWINGS

The above and other features and advantages of the present invention will become more apparent by a detailed description of exemplary embodiments thereof with reference to the attached drawings in which:
FIG. 1 illustrates the structure of an organic light-emitting device according to an embodiment of the present invention.

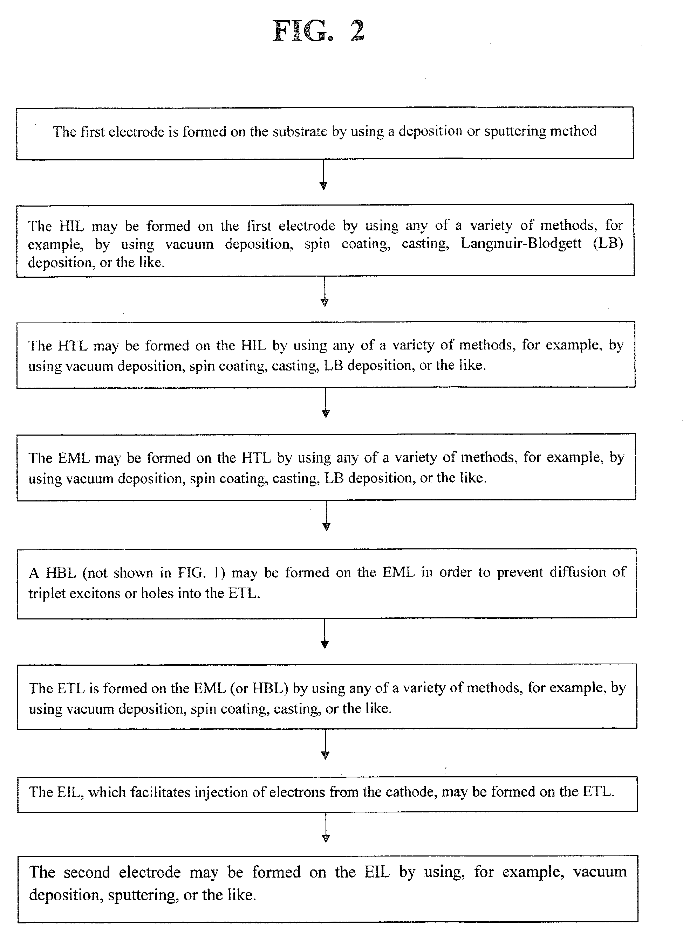

Fig. 2 illustrates the preparation of an organic light emitting device according to an embodiment of the present invention.

### DETAILED DESCRIPTION OF THE INVENTION

Organic light-emitting devices can be roughly classified as either inorganic light-emitting devices that include emission layers containing inorganic compounds, or organic light-emitting devices that include emission layers containing organic compounds.

Specifically, organic light-emitting devices have higher luminance, lower driving voltages, and shorter response times than inorganic light-emitting devices, and can render multi-colored displays. Thus, much research into such organic light-emitting devices has been conducted.

Typically, an organic light-emitting device has a stack structure including an anode, a cathode and an organic emission layer interposed therebetween. However, a hole injection layer, a hole transport layer, an electron transport layer, or an electron injection layer may be further stacked between either the anode or the cathode and the organic emission layer. In other words, an organic light-emitting device may have a stack structure of anode/hole transport layer/organic emission layer/cathode or a stack structure of anode/hole transport layer/organic emission layer/electron transport layer/cathode.

As a material for forming the organic emission layer, naphthalene derivatives can be used. However, organic light-emitting devices including such materials may not have satisfactory life span, efficiency, and power consumption characteristics, therefore improvement in this regard is still necessary.

An organic light-emitting device manufactured using a compound of phenylanthracene dimer or trimer as an organic emission layer material is widely known. However, such organic light-emitting devices have a narrow energy gap and lower blue-light color purity since two or three oligomeric species of anthracene are linked by conjugation. In addition, such compounds are highly vulnerable to oxidation and thus are liable to produce impurities, necessitating purification. In order to overcome these drawbacks, organic light-emitting devices manufactured using an anthracene compound including naphthalene substituted for anthracene at the 1, 9 positions or using a diphenylanthracene compound including an aryl group substituted for a phenyl group at the m-position have been introduced. However, these organic light-emitting devices have a lower light-emission efficiency.

Organic light-emitting devices may also be manufactured using naphthalene-substituted monoanthracene derivatives. However, the light-emission efficiency thereof is low at about 1cd/A, and thus such organic light-emitting devices are not suitable for practical use.

Furthermore, organic light-emitting devices may be manufactured using phenylanthracene compounds including aryl substituents at the m-position. Such a compound has excellent thermal resistance but leads to an unsatisfactorily low light-emission efficiency of about 2cd/A.

The present invention will now be described more fully with reference to the accompanying drawings, in which exemplary embodiments of the invention are shown.

A heterocyclic compound according to an embodiment of the present invention is represented by Formula 1 below:

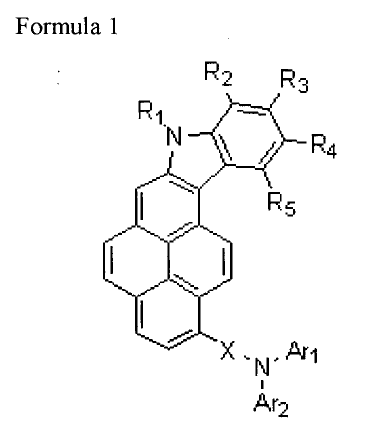

In Formula 1, R₁ through R₅ are each independently a hydrogen atom, a deuterium atom, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₃-C₆₀ cycloalkyl group, a substituted or unsubstituted C₁-C₆₀ alkoxy group, a substituted or unsubstituted C₅-C₆₀ aryloxy group, a substituted or unsubstituted C₅-C₆₀ arylthio group, a substituted or unsubstituted C₅-C₆₀ aryl group, an amino substituted group with a C₅-C₆₀ aryl or C₃-C₆₀ heteroaryl group, a substituted or unsubstituted C₃-C₆₀ heteroaryl group, a substituted or unsubstituted C₆-C₆₀ condensed polycyclic group, a halogen atom, a cyano group, a nitro group, a hydroxyl group, or a carboxyl group;

Ar₁ and Ar₂ are each independently a substituted or unsubstituted C₁-C₆₀ alkyl group, a substituted or unsubstituted C₃-C₅₀ cycloalkyl group, a substituted or unsubstituted C₅-C₆₀ aryloxy group, a substituted or unsubstituted C₅-C₆₀ arylthio group, a substituted or unsubstituted C₅-C₆₀ aryl group, an amino group substituted with a C₅-C₆₀ aryl or C₃-C₆₀ heteroaryl group, a substituted or unsubstituted C₃-C₆₀ heteroaryl group, or a substituted or unsubstituted C₆-C₆₀ condensed polycyclic group; and

X is a divalent linking group represented by -(Ar₃)ₙ- where Ar₃ is a substituted or unsubstituted C₅-C₆₀ arylene group, a substituted or unsubstituted C₃-C₆₀ heteroarylene group, or a substituted or unsubstituted C₆-C₆₀ condensed polycyclic group, and *n* is an integer from 1 to 10, wherein *n* groups of Ar₃ may be identical to or different from each other, and at least two adjacent groups of the *n* Ar₃ groups may be fused or linked to each other by a single bond.

The term "substituted" as used herein refers to the substitution of at least one hydrogen atom of the respective moiety with heavy hydrogen atom, a halogen atom, a hydroxyl group, a nitro group, a cyano group, an amino group, an amidino group, hydrazine, hydrazone, a carboxyl group or a salt thereof, a sulfonic acid group or a salt thereof, a phosphoric acid group or a salt thereof, a C₁-C₁₀ alkyl group, a C₁-C₁₀ alkoxy group, a C₂-C₁₀ alkenyl group, a C₂-C₁₀ alkynyl group, a C₆-C₁₆ aryl group, or a C₃-C₁₆ heteroaryl group.

In some embodiments the heterocyclic compound of Formula 1 may be used as a light-emitting material, a hole transporting material, or an electron transporting material. Having improved performance as blue-emitting material, the compound of Formula 1 may be useful as a deep blue material in a large display having a non-resonant structure. The heterocyclic compound of Formula 1 having a heterocyclic group in the molecules thereof has a high glass transition temperature (Tg) or a high melting point due to the inclusion of the heterocyclic group. Thus, the heterocyclic compound has high heat resistance against Joule's heat generated in an organic layer, between organic layers, or between an organic layer and a metal electrode when light emission occurs, and has high durability in high-temperature environments.

An organic light-emitting device manufactured using a heterocyclic compound of Formula 1 has high durability when stored or operated. In addition, due to the inclusion of a substituent such as an aryl group or heteroaryl group, molecular layers formed as thin films may be maintained in good condition, thereby improving the characteristics of the organic light-emitting device.

Substituents in the heterocyclic compound of Formula 1 will now be described in detail.

R₁ to R₅ in Formula 1 above may be each independently a hydrogen atom, a deuterium atom, a cyano group, a halogen atom, an unsubstituted C₁-C₂₀ alkyl group, a substituted C₁-C₂₀ alkyl group with at least one fluorine (-F) substituent, a substituted or unsubstituted C₅-C₂₀ aryl group, or a substituted or unsubstituted C₃-C₂₀ heteroaryl group.

In some embodiments R₁ to R₅ in Formula 1 may be each independently a hydrogen atom, a deuterium atom, a halogen atom, a cyano group, a substituted or unsubstituted C₁-C₂₀ alkyl group, or compounds represented by Formulae 2a to 2f below:

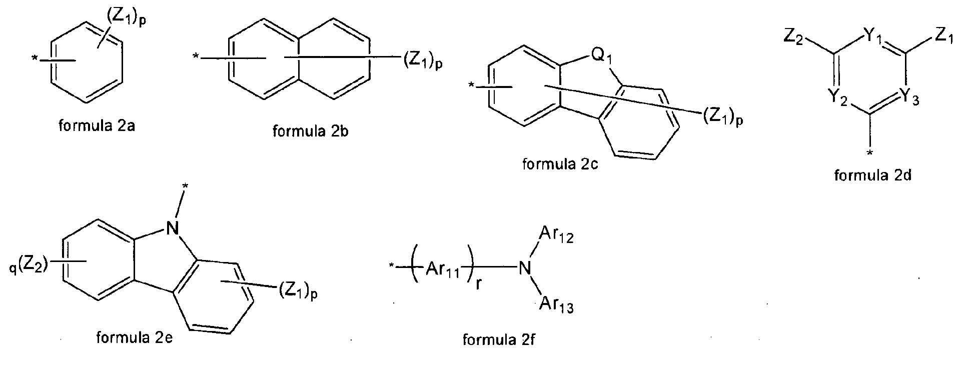

In Formula 2a to 2f above, Q₁ is a linking group represented by -C(R₆)(R₇)-, -N(R₆)-, -S-, or -O-; Y₁, Y₂ and Y₃ are each independently a linking group represented by -N= or -C(R₈)=;
Z₁, Z₂, Ar₁₂, Ar₁₃, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
Ar₁₁ is a substituted or unsubstituted C₁-C₂₀ alkylene group, a substituted or unsubstituted C₅-C₂₀ arylene group, or a substituted or unsubstituted C₃-C₂₀ heteroarylene group;
p is an integer from 1 to 12; q is an integer from 1 to 12; r is an integer from 0 to 5; and * indicates a binding site.

In some embodiments R₁ to R₅ in Formula 1 above may be each independently a hydrogen atom, a deuterium atom, a halogen atom, a cyano group, a methyl group, an ethyl group, a propyl group, a butyl group, a pentyl group, or compounds represented by Formulae 3a to 3h below:

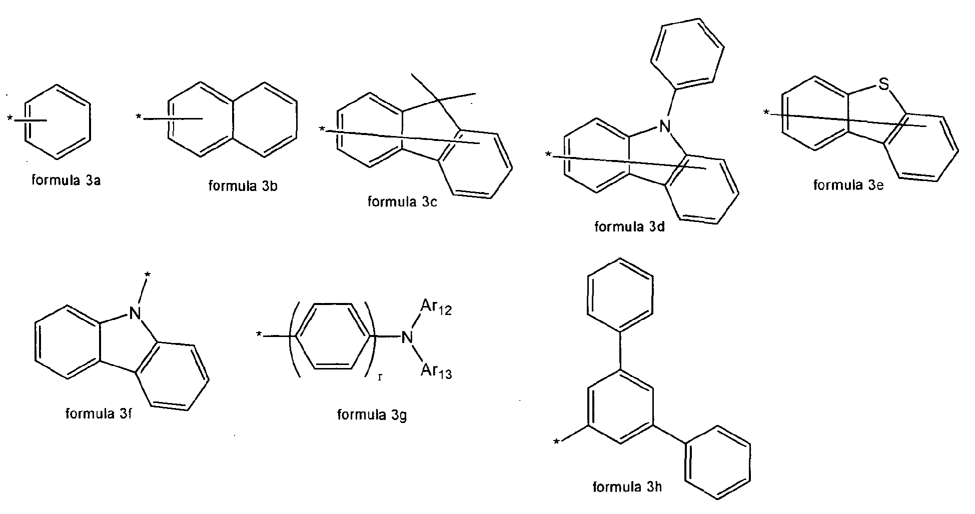

In Formula 3a to 3h above, Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group; r is an integer from 0 to 2; and * indicates a binding site.

In some embodiments R₂ and R₅ in Formula 1 above may be hydrogen atoms; and R₁, R₃ and R₄ may be each independently a hydrogen atom, a deuterium atom, a halogen atom, a cyano group, a methyl group, an ethyl group, a propyl group, a butyl group, a pentyl group, or compounds represented by Formulae 3a to 3h above.

In some embodiments R₂ and R₅ in Formula 1 above may be hydrogen atoms; and R₁, R₃ and R₄ may be each independently a substituted or unsubstituted C₁-C₂₀ alkyl group, a substituted or unsubstituted C₆-C₂₀ aryl group, or a substituted or unsubstituted C₃-C₂₀ heteroaryl group.

In some embodiments Ar₁ and Ar₂ in Formula 1 above may be each independently a substituted or unsubstituted C₁-C₂₀ alkyl group, a substituted or unsubstituted C₅-C₂₀ aryl group, or a substituted or unsubstituted C₃-C₂₀ heteroaryl group.

In some other embodiments Ar₁ and Ar₂ in Formula 1 above may be each independently a compound represented by Formulae 4a to 4d below:

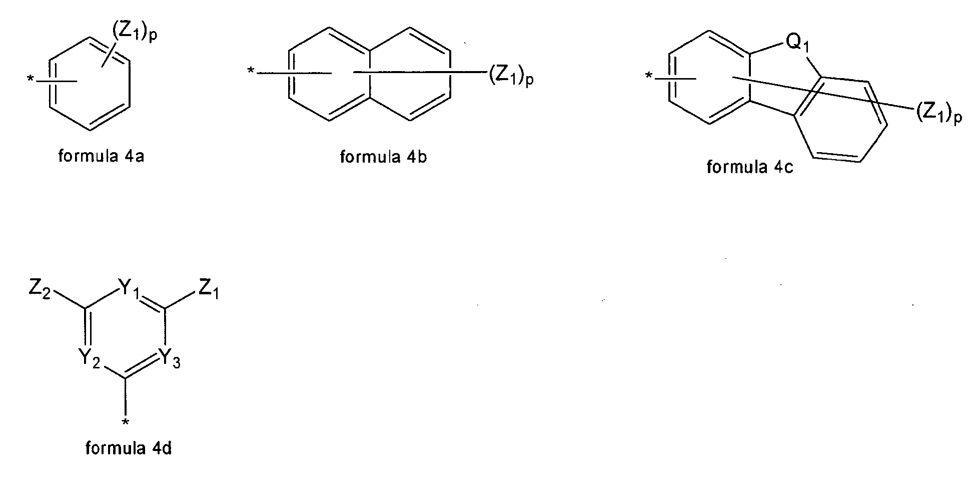

In Formulae 4a to 4d, Q₁ is a linking group represented by -C(R₆)(R₇)-, -N(R₆)-, -S-, or -O-; Y₁, Y₂, and Y₃ are each independently a linking group represented by -N= or -C(R₈)=; Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group; p is an integer from 1 to 8; and * indicates a binding site.

In some embodiments Ar₁ and Ar₂ in Formula 1 above may be each independently a compound represented by one of Formulae 5a to 5i below:

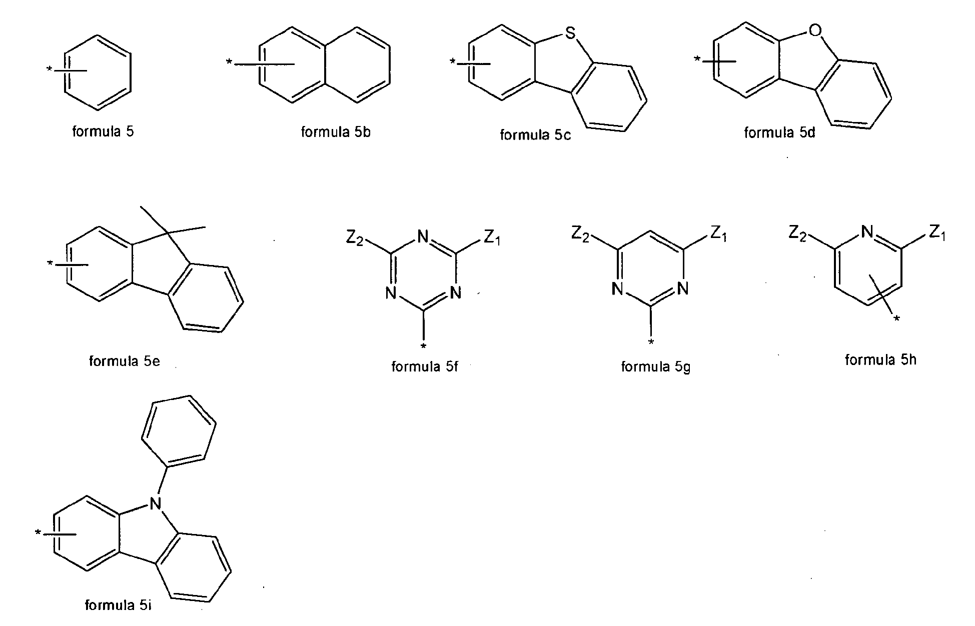

In Formulae 5a to 5i, Z₁ and Z₂ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, or a substituted or unsubstituted C₆-C₂₀ condensed polycyclic group; and * indicates a binding site.

In some embodiments Ar₃ for X in Formula 1 above may be a substituted or unsubstituted C₅-C₂₀ arylene group, a substituted or unsubstituted C₃-C₂₀ heteroarylene group, or a substituted or unsubstituted C₆-C₂₀ condensed polycyclic group.

In some other embodiments Ar₃ may be a group represented by one of Formulae 6a to 6e:

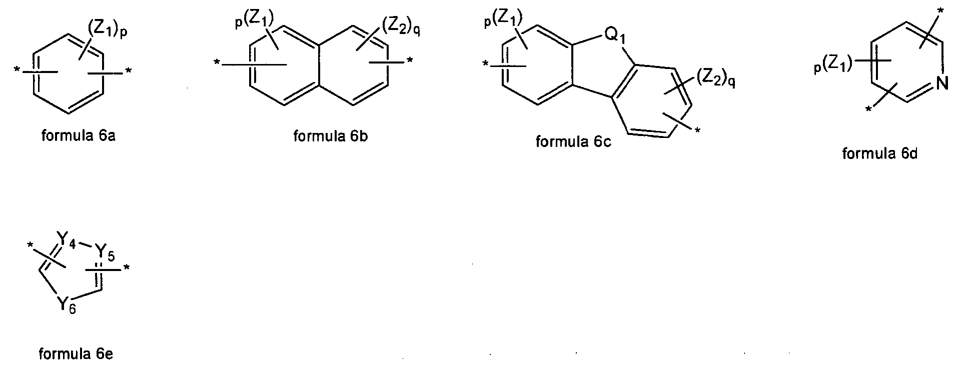

In Formulae 6a to 6e, Q₁ is a linking group represented by -C(R₆)(R₇)-, -N(R₆)-,or -S-;
Y₄, Y₅, and Y₆ are each independently a linking group represented by -N= or -C(R₈)=, -S-, or -O-;
Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
p is an integer from 1 to 8; q is an integer from 1 to 8; and * indicates a binding site.

In some embodiments *n* indicating the number of Ar₃ groups for X in Formula 1 above may be an integer of 1 or 2,

In some embodiments X in Formula 1 above may be a group represented by one of Formulae 7a to 7i:

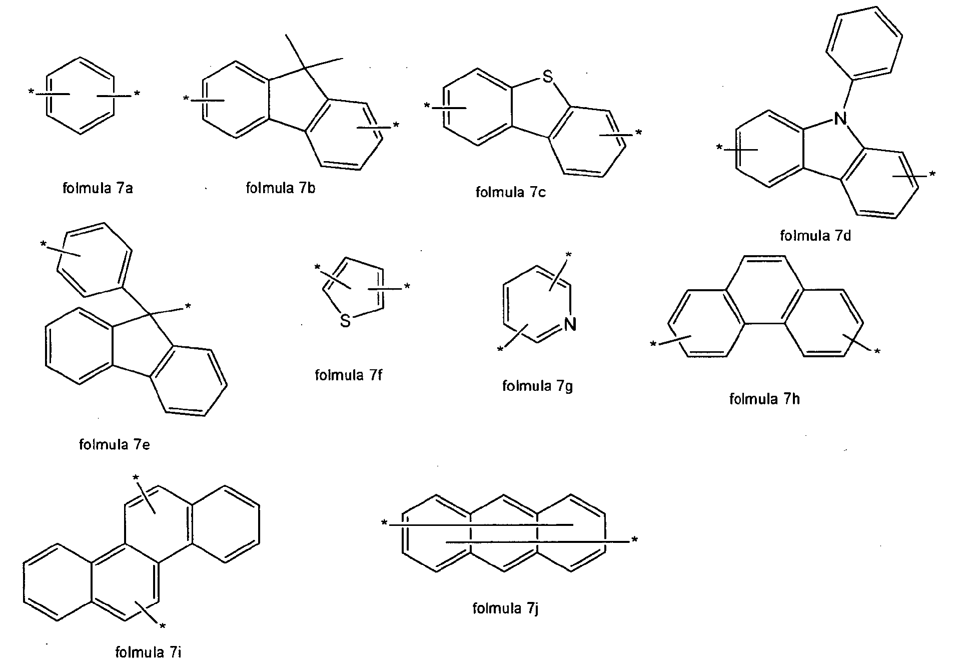

In Formulae 7a to 7j, * indicates a binding site.

In some embodiments R₁, R₃, and R₄ in Formula 1 above may be each independently a hydrogen atom, a deuterium atom, a halogen atom, a cyano group, a methyl group, an ethyl group, a propyl group, a butyl group, a pentyl group, or compounds represented by Formulae 3a to 3h below:

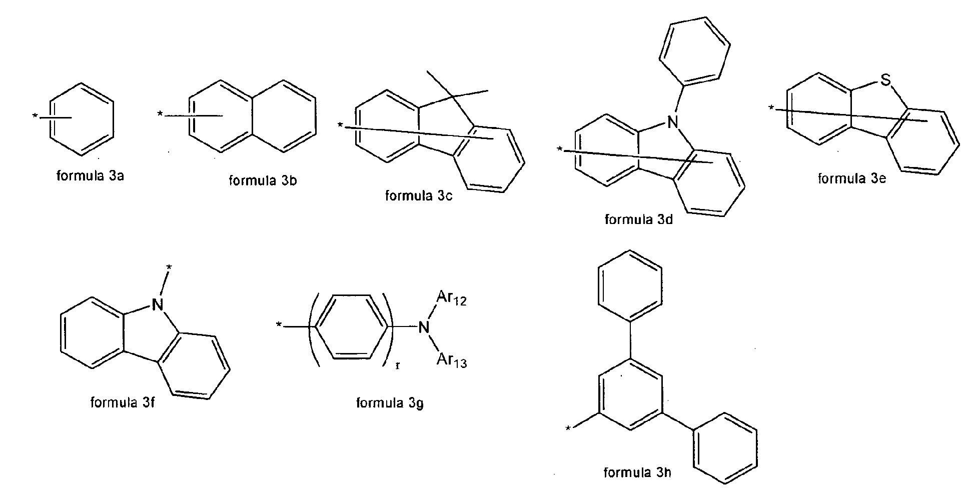

In Formula 3a to 3h above, Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group; r is an integer from 0 to 2, and * indicates a binding site;
R₂ and R₅ in Formula 1 may be hydrogen atoms;
Ar₃ in Formula 1 may be a group represented by one of Formulae 6a to 6e below; 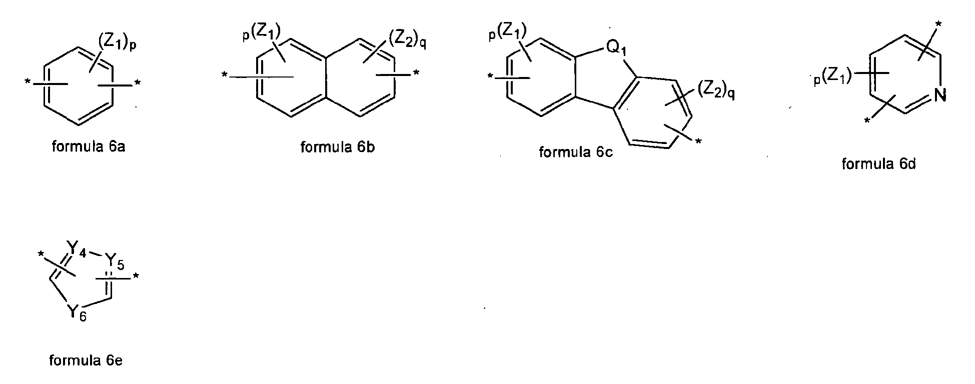 wherein, in Formulae 6a to 6e, Q₁ is a linking group represented by -C(R₆)(R₇)-, -N(R₆)-, or -S-; Y₄, Y₅ and Y₆ are each independently a linking group represented by -N=, -C(R₈)=, -S-, or -O-; Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group; p is an integer from 1 to 8; q is an integer from 1 to 8; and * indicates a binding site;
*n*, which indicates the number of Ar₃ groups for X in Formula 1, may be an integer of 1 or 2;
Ar₁ and Ar₂ in Formula 1 may be each independently a compound represented by one of Formulae 4a to 4d below: 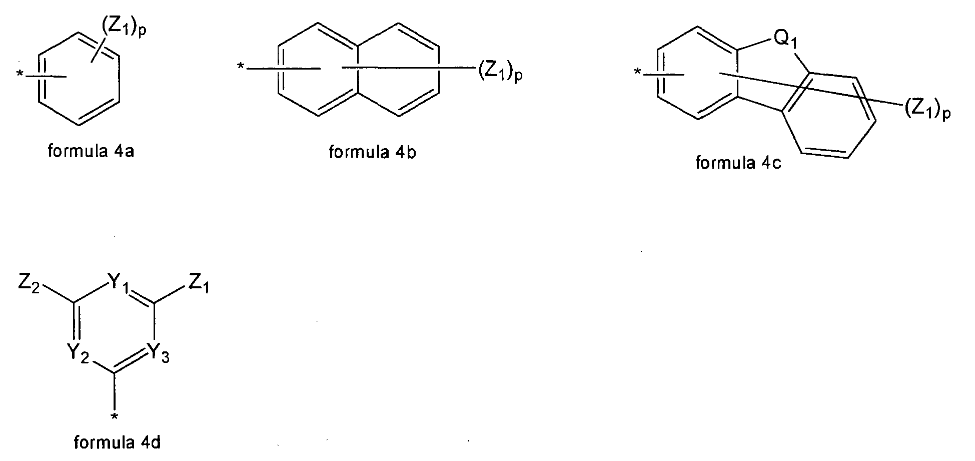 wherein, in Formulae 4a to 4e, Q₁ is a linking group represented by -C(R₆)(R₇)-, -N(R₆)-, -S-, or -O-; Y₁, Y₂ and Y₃ are each independently a linking group represented by -N= or -C(R₈)=; Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group; p is an integer from 1 to 8; and * indicates a binding site.

Hereinafter, substituents described with reference to Formulae 1 to 7J will now be described in detail. In this regard, the numbers of carbons in substituents are presented only for illustrative purposes and do not limit the characteristics of the substituents

The unsubstituted C₁-C₆₀ alkyl group used herein may be linear or branched. Examples of the alkyl group may include, but are not limited to, a methyl group, an ethyl group, a propyl group, an isobutyl group, a sec-butyl group, a pentyl group, an iso-amyl group, a hexyl group, a heptyl group, an octyl group, a nonanyl group, and a dodecyl group.

In the substituted C₁-C₆₀ alkyl group, at least one hydrogen atom of the alkyl group may be substituted with heavy hydrogen atom, a halogen atom, a hydroxyl group, a nitro group, a cyano group, an amino group, an amidino group, hydrazine, hydrazone, a carboxyl group or a salt thereof, a sulfonic acid group or a salt thereof, a phosphoric acid group or a salt thereof, a C₁-C₁₀ alkyl group, a C₁-C₁₀ alkoxy group, a C₂-C₁₀ alkenyl group, a C₂-C₁₀ alkynyl group, a C₆-C₁₆ aryl group, or a C₃-C₁₆ heteroaryl group.

The unsubstituted C₂-C₆₀ alkenyl group indicates an unsaturated alkyl groups having at least one carbon-carbon double bond in the center or at a terminal of the alkyl group. Examples of the alkenyl group include an ethenyl group, a propenyl group, a butenyl group, and the like. In the substituted C₂-C₆₀ alkenyl group, at least one hydrogen atom in the unsubstituted alkenyl group may be substituted with a substituent described above in conjunction with the alkyl group.

The unsubstituted C₂-C₆₀ alkynyl group indicates an alkyl group having at least one carbon-carbon triple bond in the center or at a terminal of the alkyl group. Examples of the unsubstituted C₂-C₂₀ alkynyl group include acetylene, propylene, phenylacetylene, naphthylacetylene, isopropylacetylene, t-butylacetylene, diphenylacetylene, and the like. In the substituted C₂-C₆₀ alkynyl group, at least one hydrogen atom in the alkynyl group may be substituted with a substituent described above in conjunction with the alkyl group.

The unsubstituted C₃-C₆₀ cycloalkyl group indicates a C₃-C₆₀ cyclic alkyl group. In the substituted C₃-C₆₀ cycloalkyl group, at least one hydrogen atom in the cycloalkyl group may be substituted with a substituent described above in connection with the C₁-C₆₀ alkyl group.

The unsubstituted C₁-C₆₀ alkoxy group indicates a group having a structure of -OA wherein A is an unsubstituted C₁-C₆₀ alkyl group as described above. Nonlimiting examples of the unsubstituted C₁-C₆₀ alkoxy group include a methoxy group, an ethoxy group, a propoxy group, an isopropyloxy group, a butoxy group, and a pentoxy group. In the substituted C₁-C₆₀ alkoxy group, at least one hydrogen atom of the alkoxy group may be substituted with a substituent such as those described above in conjunction with the alkyl group.

The unsubstituted C₅-C₆₀ aryl group indicates a carbocyclic aromatic system containing at least one ring. At least two rings may be fused to each other or linked to each other by a single bond. The term 'aryl' refers to an aromatic system, such as phenyl, naphthyl, or anthracenyl. In the substituted C₅-C₆₀ aryl group, at least one hydrogen atom in the aryl group may be substituted as described above with reference to the unsubstituted C₁-C₆₀ alkyl group.

Examples of the substituted or unsubstituted C₅-C₆₀ aryl group include, but are not limited to, a phenyl group, a C₁-C₁₀ alkylphenyl group (for example, ethylphenyl group), a halophenyl group (for example, o-, m-, and p-fluorophenyl group, dichlorophenyl group), a cyanophenyl group, dicyanophenyl group, a trifluoromethoxyphenyl group, a biphenyl group, a halobiphenyl group, a cyanobiphenyl group, a C₁-C₁₀ alkyl biphenyl group, a C₁-C₁₀ alkoxybiphenyl group, a o-, m-, and p-toryl group, an o-, m-, and p-cumenyl group, a mesityl group, a phenoxyphenyl group, a (α,α-dimethylbenzene)phenyl group, a (N,N'-dimethyl)aminophenyl group, a (N,N'-diphenyl)aminophenyl group, a pentalenyl group, an indenyl group, a naphthyl group, a halonaphthyl group (for example, fluoronaphthyl group), a C₁-C₁₀ alkylnaphthyl group (for example, methylnaphthyl group), a C₁-C₁₀ alkoxynaphthyl group (for example, methoxynaphthyl group), a cyanonaphthyl group, an anthracenyl group, an azulenyl group, a heptalenyl group, an acenaphthylenyl group, a phenalenyl group, a fluorenyl group, an anthraquinolyl group, a methylanthryl group, a phenanthryl group, a triphenylene group, a pyrenyl group, a chrycenyl group, an ethyl-chrysenyl group, a picenyl group, a perylenyl group, a chloroperylenyl group, a pentaphenyl group, a pentacenyl group, a tetraphenylenyl group, a hexaphenyl group, a hexacenyl group, a rubicenyl group, a coronelyl group, a trinaphthylenyl group, a heptaphenyl group, a heptacenyl group, a pyranthrenyl group, and an ovalenyl group.

The unsubstituted C₃-C₆₀ heteroaryl group includes one, two or three hetero atoms selected from N, O, P and S. At least two rings may be fused to each other or linked to each other by a single bond. Examples of the unsubstituted C₃-C₆₀ heteroaryl group include a pyrazolyl group, an imidazolyl group, an oxazolyl group, a thiazolyl group, a triazolyl group, a tetrazolyl group, an oxadiazolyl group, a pyridinyl group, a pyridazinyl group, a pyrimidinyl group, a triazinyl group, a carbazol group, an indol group, a quinolyl group, an isoquinolyl group, and a dibenzothiophene group. In addition, at least one hydrogen atom in the heteroaryl group may be substituted with a substituent described above in conjunction with the unsubstituted C₁-C₆₀ alkyl group.

The unsubstituted C₅-C₆₀ aryloxy group is a group represented by -OA₁ wherein A₁ may be a C₅-C₅₀ aryl group. An example of the aryloxy group is a phenoxy group. At least one hydrogen atom in the aryloxy group may be substituted with a substituent described above in conjunction with the unsubstituted C₁-C₅₀ alkyl group.

The unsubstituted C₅-C₆₀ arylthio group is a group represented by -SA₁ where A₁ may be a C₅-C₆₀ aryl group. Nonlimiting examples of the arylthio group include a benzenethio group and a naphthylthio group. At least one hydrogen atom in the arylthio group may be substituted with a substituent described above in conjunction with the unsubstituted C₁-C₆₀ alkyl group.

The unsubstituted C₆-C₆₀ condensed polycyclic group refers to a substituent including at least two rings wherein at least one aromatic ring and/or at least one non-aromatic ring are fused to each other. The unsubstituted C₆-C₆₀ condensed polycyclic group may include some of the substituents described in conjunction with the aryl group or the heteroaryl group.

The following compound is an example of the condensed polycyclic group.

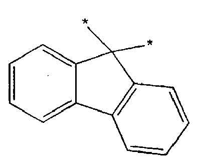

X in the compound of Formula 1 tends to be perpendicular to moiety A (pyrene moiety) in Formula 1 (see below), interfering with lone pair electron of moiety B (arylamine moiety) forming a resonance structure in the moiety A.

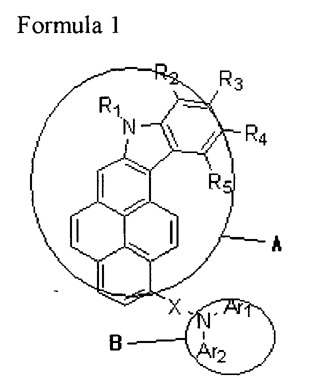

As a result, the compound of Formula 1 may emit high-color purity blue light. The blue light emitted from the compound of Formula 1 is distinct from common longer-wavelength bluish green light.

The compound of Formula 1 may have strong resistance to heat. Thus, when used in an organic light-emitting device, the compound of Formula 1 may improve efficiency and lifespan characteristics of the organic light emitting device.

Examples of the heterocyclic compound represented by Formula 1 may include Compounds 1 through 75 represented by the following formulae. However, the heterocyclic compounds represented by Formula 1 are not limited thereto.

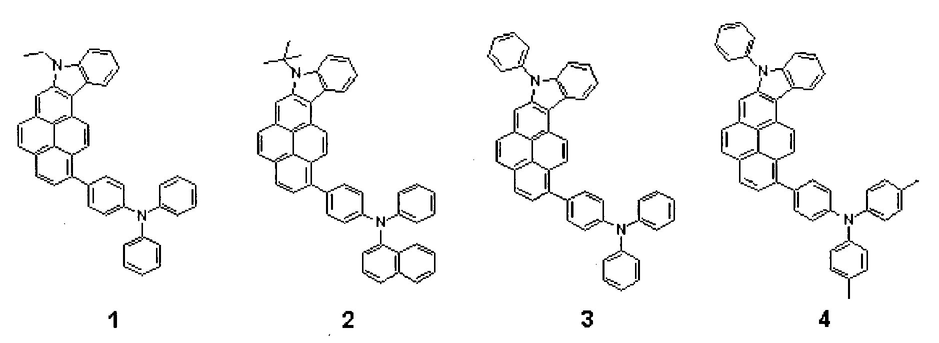

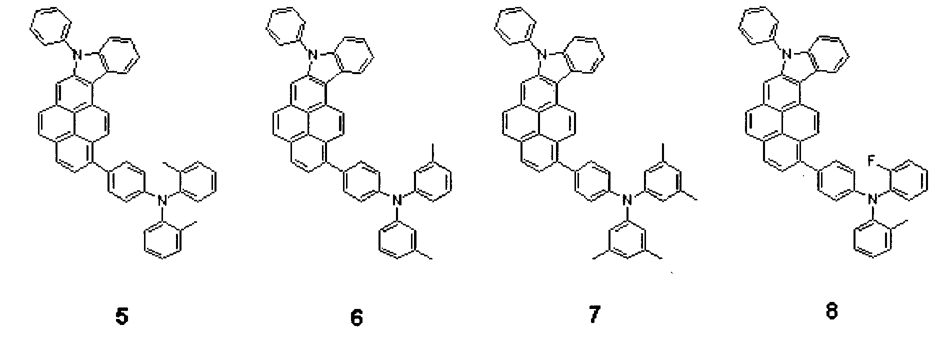

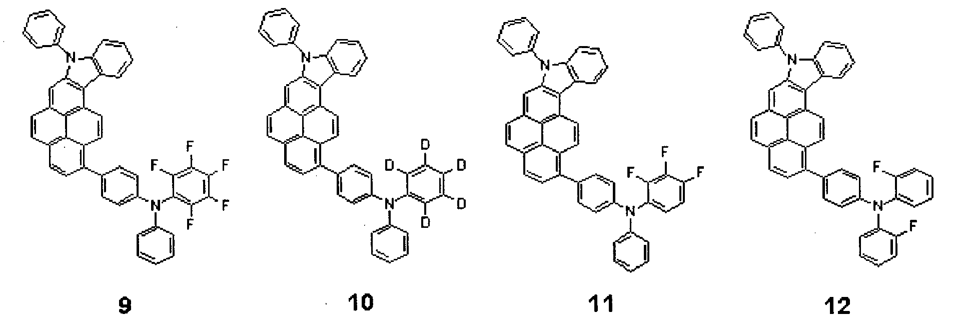

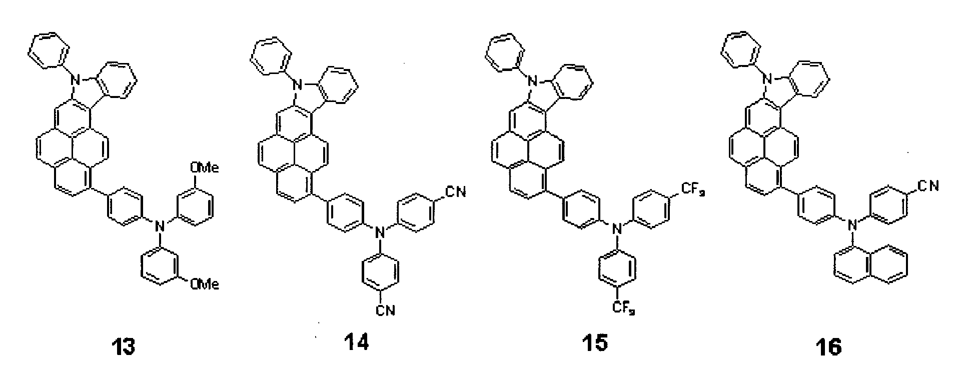

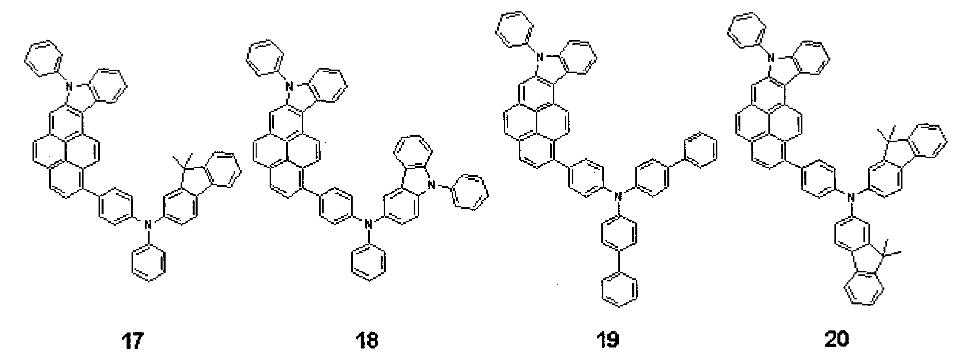

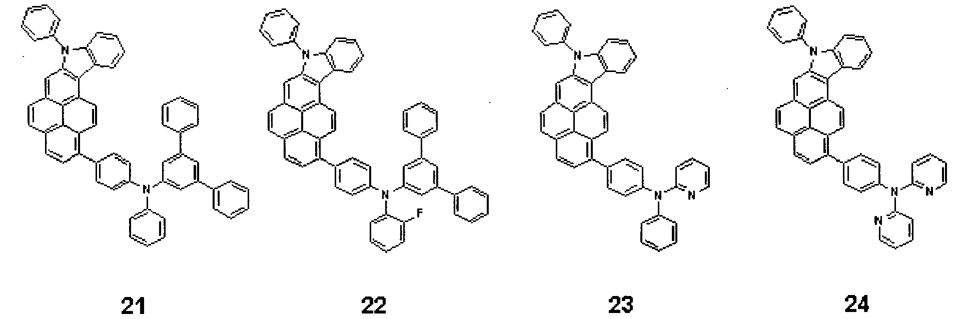

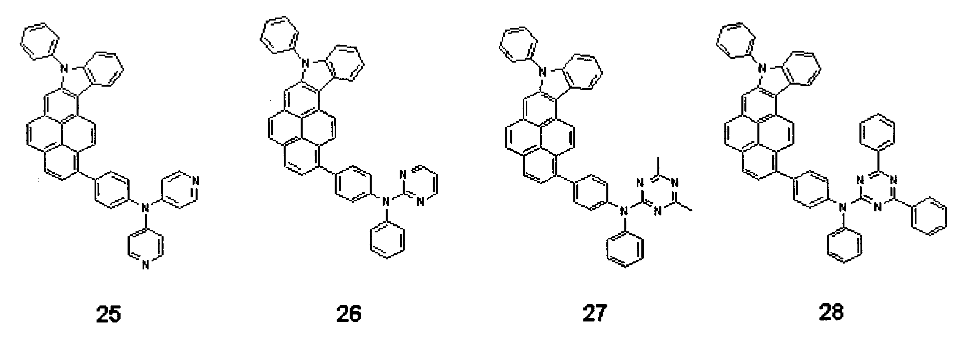

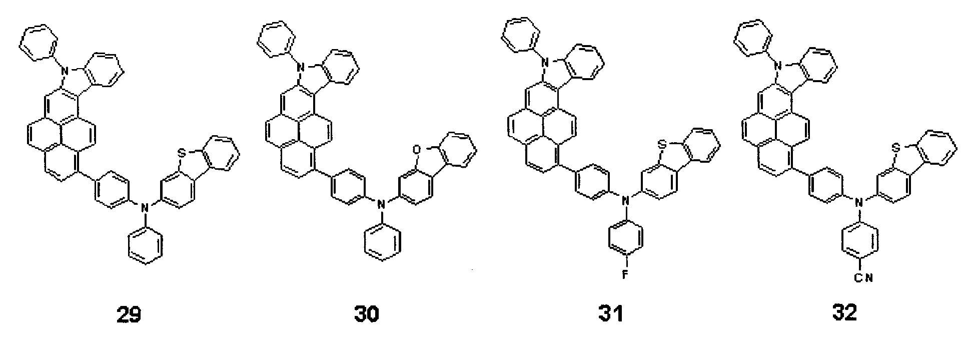

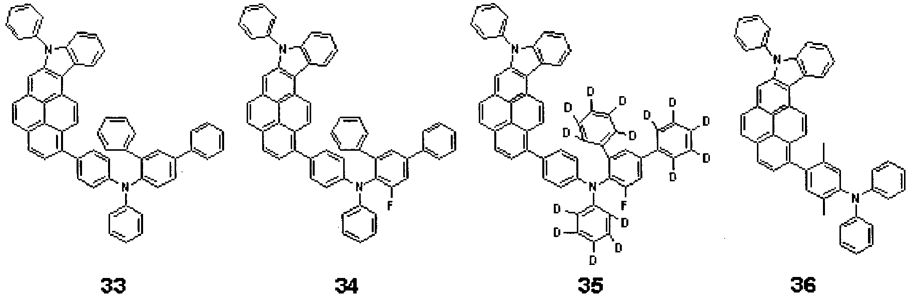

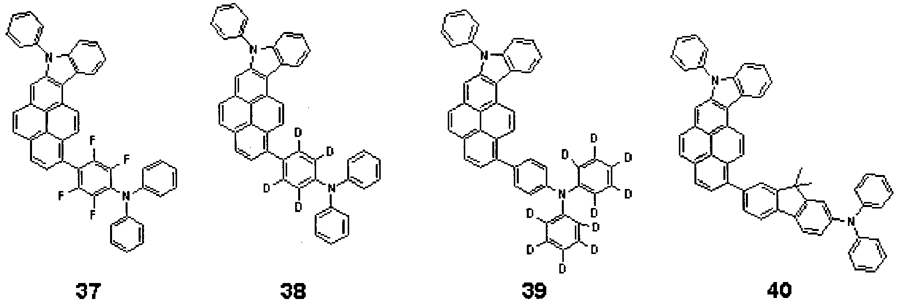

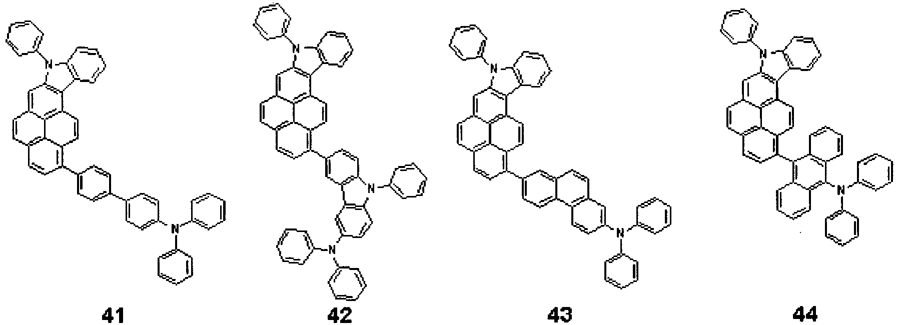

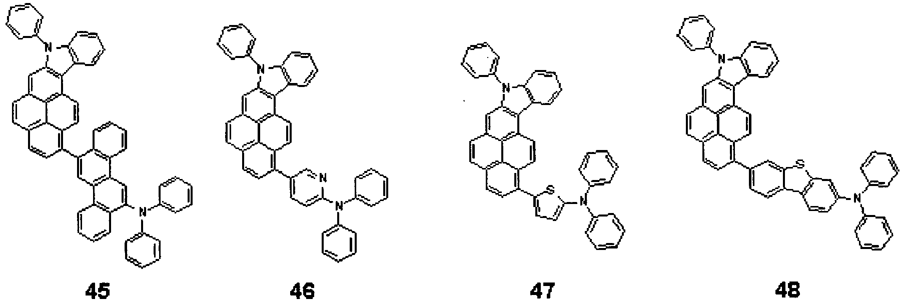

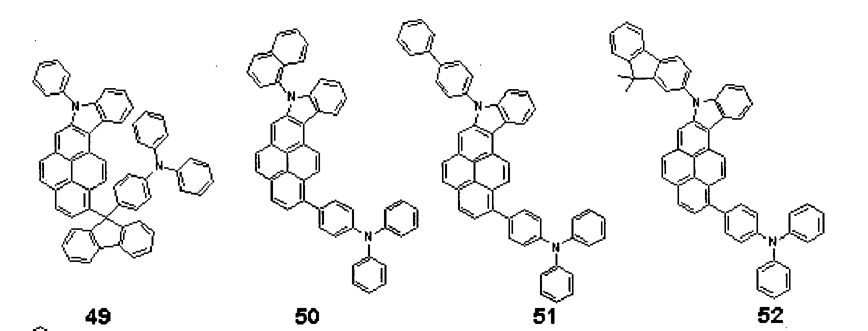

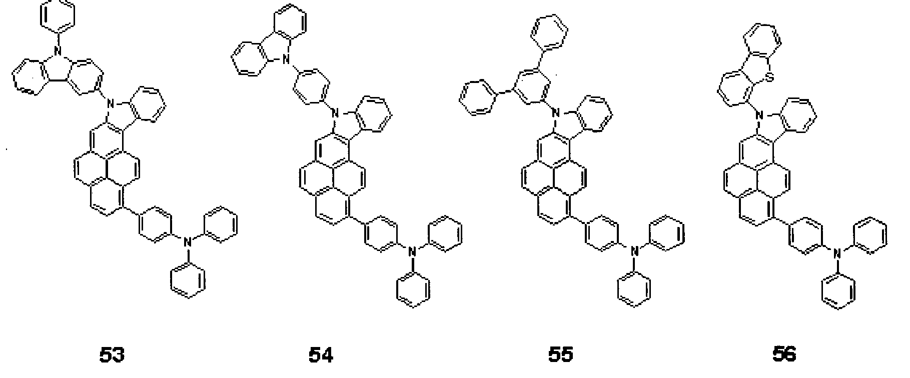

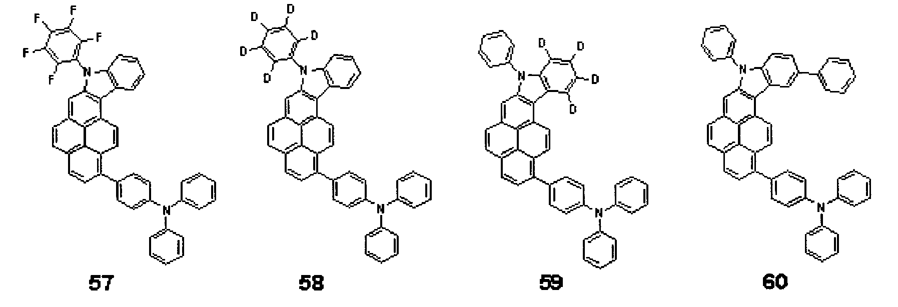

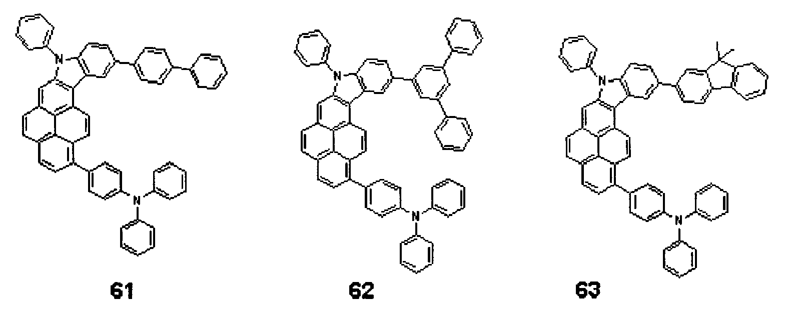

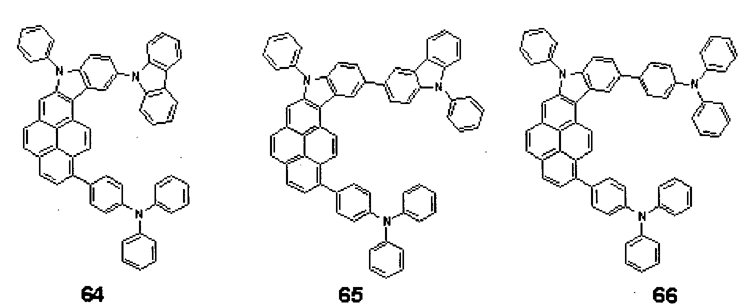

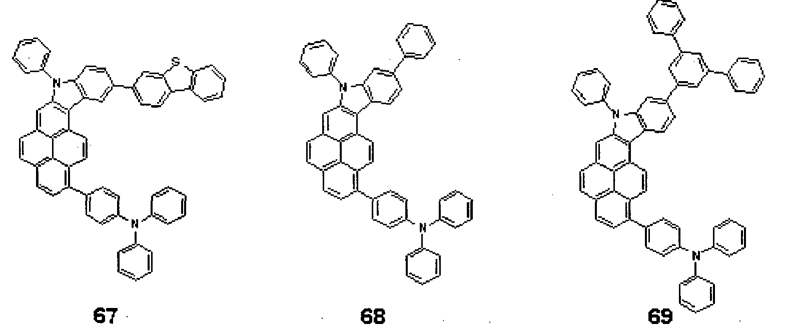

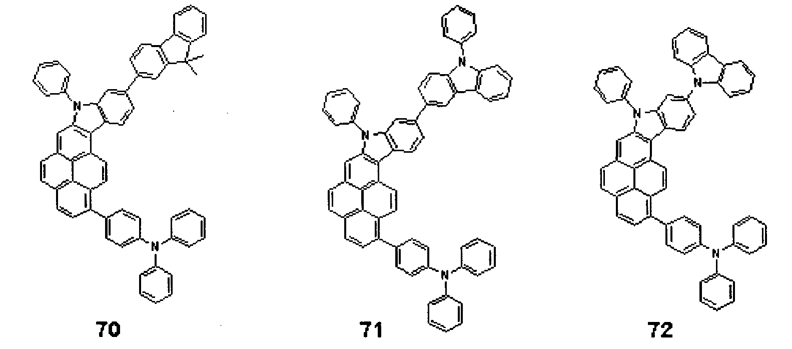

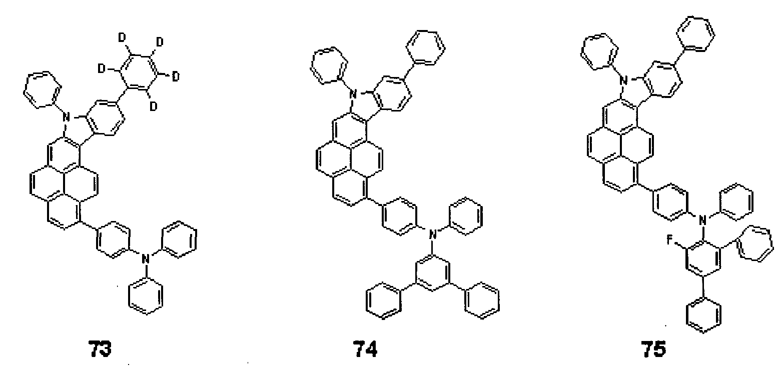

According to an embodiment, an organic light-emitting device includes a first electrode, a second electrode, and an organic layer between the first electrode and the second electrode, wherein the organic layer may include a first layer including the heterocyclic compound of Formula 1 described above.

The first layer including the heterocyclic compound of Formula 1 may include a hole injection layer, a hole transport layer, or a functional layer having both hole injection and hole transport capabilities.

The first layer including the heterocyclic compound of Formula 1 may include an electron injection layer, an electron transport layer, or a functional layer having both electron injection and electron transport capabilities.

When the first layer including the heterocyclic compound of Formula 1 is an emission layer, the heterocyclic compound of Formula 1 may be used as a host or a dopant for a fluorescent or phosphorescent device.

In some embodiments if the first layer of the organic light-emitting device is an emission layer, the emission layer may further include a known anthracene, arylamine or styryl compound.

In addition, at least one hydrogen atom in the anthracene, arylamine or styryl compound may be substituted with a substituent described above in conjunction with the unsubstituted C₁-C₆₀ alkyl group.

The arylamine is a C₅-C₆₀ arylamine group including an amino group with a C₅-C₆₀ aryl or C₃-C₆₀ heteroaryl substituent.

In some embodiments if the first layer of the organic light-emitting device is an emission layer, a red emission layer, a green emission layer, a blue emission layer or a white emission layer of the emission layer may include a widely-known phosphorescent compound.

In some embodiments the first layer of the organic light-emitting device may include a blue emission layer. When the first layer includes a blue emission layer, the heterocyclic compound of Formula 1 may be used as a blue dopant. In some embodiments, the organic layer of the organic light-emitting device may further include, but is not limited to, a hole injection layer, a hole transport layer, a functional layer having both hole injection and transport functions, an emission layer, a hole blocking layer, an electron transport layer, an electron injection layer, of a combination of at least two of these layers. At least one of the hole injection layer, the hole transport layer, and the functional layer having both hole injection and transport functions, may further include, in addition to the heterocyclic compound of Formula 1 and widely-known hole injection and transport materials, a charge-generating material for improving conductivity of the layer.

The charge-generating material may include, for example, a p-dopant. Nonlimiting examples of the p-dopant include quinine derivatives, including tetracyanoquinondimethane (TCNQ) and 2,3,5,6-tetrafluoro-tetracyano-1,4-benzoquininedimethane (F4TCNQ); metal oxides, including tungsten oxide and molybdenium oxide; and cyano group-containing compounds, including a compound represented by Formula 100 below.

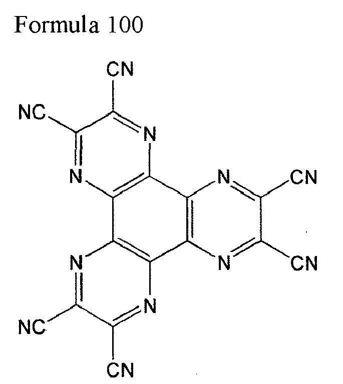

In some embodiments, when the hole injection layer, the hole transport layer, or the functional layer having both hole injection and transport functions further includes the charge-generating material, the charge-generating material may be uniformly or non-uniformly distributed in the layer.

In one embodiment, the electron transport layer of the organic light-emitting device may include an electron transporting organic compound and a metal-containing material. Nonlimiting examples of the electron transporting organic compound include ADN(9,10-di(naphthalene-2-yl)anthracene); and anthracene-based compounds, including a compound of Formula 101 and a compound of Formula 102 below.

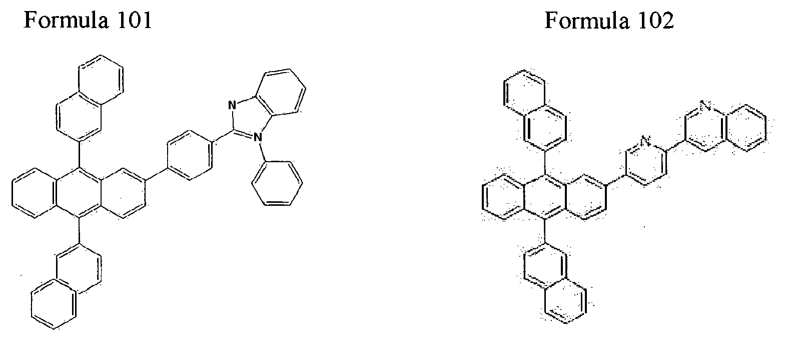

The metal-containing material may include a Li complex. Nonlimiting examples of the Li complex include lithium quinolate (LiQ) and a compound of Formula 103 below.

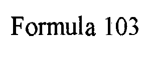

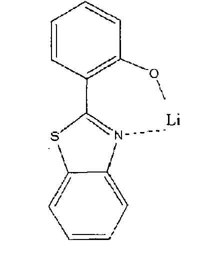

The first electrode may be an anode, and the second electrode may be a cathode, but the reverse is also possible.

In some embodiments, the organic light-emitting device may have a first electrode/hole injection layer/emission layer/second electrode structure, a first electrode/hole injection layer/hole transport layer/emission layer/electron transport layer/second electrode structure, or a first electrode/hole injection layer/hole transport layer/emission layer/electron transport layer/electron injection layer/second electrode structure. In some other embodiments, the organic light-emitting device may have a first electrode/functional layer having both hole injection and hole transport capabilities/emission layer/electron transport layer/second electrode structure, or a first electrode/functional layer having both hole injection and hole transport capabilities/emission layer/electron transport layer/electron injection layer/second electrode structure. Alternatively, the organic light-emitting device may have a first electrode/hole transport layer/emission layer/functional layer having both electron injection and electron transport capabilities/second electrode structure, a first electrode/hole injection layer/emission layer/functional layer having both electron injection and electron transport capabilities/second electrode structure, or a first electrode/hole injection layer/hole transport layer/emission layer/functional layer having both electron injection and electron transport capabilities/second electrode structure.

In some embodiments the organic light-emitting device may be either a top-emission organic light-emitting device or a bottom-emission organic light-emitting device.

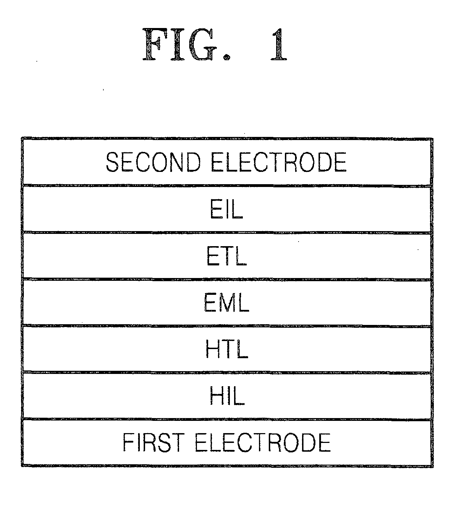

Hereinafter, a method of manufacturing an organic light-emitting device according to an embodiment of the present invention will be described with reference to FIG. 1. FIG. 1 illustrates the structure of an organic light-emitting device according to an embodiment of the present invention. Referring to FIG. 1, the organic light-emitting device according to the present embodiment includes a substrate (not shown), a first electrode (anode), a hole injection layer (HIL), a hole transport layer (HTL), an emission layer (EML), an electron transport layer (ETL), an electron injection layer (EIL), and a second electrode (cathode).

First, the first electrode is formed on the substrate by using a deposition or sputtering method. The first electrode may be formed of a first electrode material having a high work function. The first electrode may constitute an anode or a cathode. The substrate may be a substrate conventionally used in organic light-emitting devices, and may include, for example, a glass substrate or a transparent plastic substrate with excellent mechanical strength, thermal stability, transparency, surface smoothness, ease of handling, and water resistance. Examples of the first electrode material include materials, such as indium tin oxide (ITO), indium zinc oxide (IZO), tin oxide (SnO₂), zinc oxide (ZnO), aluminum (Al), silver (Ag), and magnesium (Mg), which have excellent conductivity. The first electrode may be formed as a transparent or reflective electrode.

An organic layer(s) is formed on the first electrode. The term "organic layer" used herein indicates any layer interposed between the first electrode and the second electrode. The organic layer may not be formed of pure organic materials, and may also include a metal complex.

The organic layer may include a first layer including the heterocyclic compound of Formula 1. The organic layer may further include at least one of a HIL, a HTL, an EML, a hole blocking layer (HBL), an ETL and an EIL. The first layer may include an emission layer.

The HIL may be formed on the first electrode by using any of a variety of methods, and in some embodiments, by using vacuum deposition, spin coating, casting, Langmuir-Blodgett (LB) deposition, or the like.

When the HIL is formed using vacuum deposition, the deposition conditions may vary according to the material that is used to form the HIL, and the structure and thermal characteristics of the HIL. For example, the deposition conditions may include a deposition temperature of about 100 to about 500°C, a vacuum pressure of about 10⁻⁸ to about 10⁻³ torr, and a deposition rate of about 0.01 to about 100 Å/sec.

When the HIL, is formed using spin coating, coating conditions may vary according to the material used to form the HIL, and the structure and thermal properties of the HIL. For example, the coating conditions may include a coating speed of about 2000 rpm to about 5000 rpm, and a thermal treatment temperature of about 80°C to about 200°C at which the solvent remaining after coating may be removed.

The HIL may be formed of the heterocyclic compound of Formula 1 or any material that is commonly used to form a HIL. Nonlimiting examples of the material that can be used to form the HIL include a phthalocyanine compound such as copperphthalocyanine, 4,4',4"-tris (3-methylphenylphenylamino) triphenylamine (m-MTDATA), N,N'-di(1-naphthyl)-N,N'-diphenylbenzidine (NPB), TDATA, 2T-NATA, polyaniline/dodecylbenzenesulfonic acid (Pani/DBSA), poly(3,4-ethylenedioxythio-phene)/poly(4-styrenesulfonate) (PEDOT/PSS), polyaniline/camphor sulfonic acid (Pani/CSA), and polyaniline)/poly(4-styrenesulfonate (PANI/PSS).

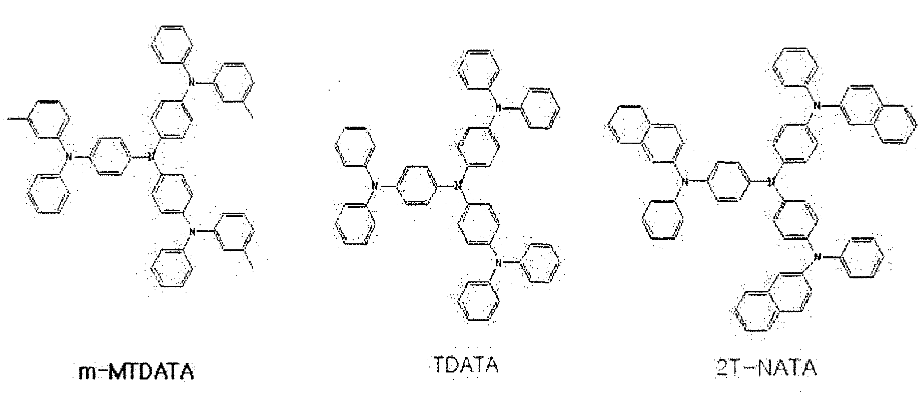

The HIL may have a thickness of about 100 Å to about 10000 Å, and in some embodiments, a thickness of about 100 Å to about 1000 Å. When the thickness of the HIL is within these ranges, the HIL may have good hole injection characteristics without an increase in driving voltage.

Next, the HTL may be formed on the HIL by using any of a variety of methods, and in some embodiments, by using vacuum deposition, spin coating, casting, LB deposition, or the like. When the HTL is formed using vacuum deposition or spin coating, the deposition or coating conditions may be similar to those applied to form the HIL, though the deposition or coating conditions may vary according to the material that is used to form the HTL.

The HTL may be formed of the heterocyclic compound of Formula 1 or any known HTL material. Nonlimiting examples of such HTL materials include carbazole derivatives such as N-phenylcarbazole or polyvinylcarbazole, and amine derivatives having an aromatic condensed ring, such as NPB, N,N'-bis(3-methylphenyl)- N,N'-diphenyl-[1,1-biphenyl]-4,4'-diamine (TPD).

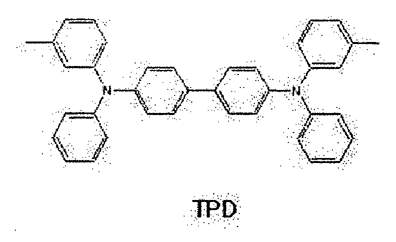

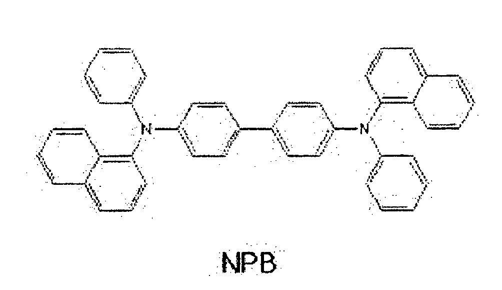

The HTL may have a thickness of about 50 Å to about 1000 Å, and in some embodiments, a thickness of about 100 Å to about 600 Å. When the thickness of the HTL is within these ranges, the HTL may have good hole transport characteristics without a substantial increase in driving voltage.

Next, the EML may be formed on the HTL by using any of a variety of methods, and in some embodiments, by using vacuum deposition, spin coating, casting, LB deposition, or the like. When the EML is formed using vacuum deposition or spin coating, the deposition or coating conditions may be similar to those applied to form the HIL, though the deposition or coating conditions may vary according to the material that is used to form the EML.

The EML may include the heterocyclic compound of Formula 1 described above. For example, the heterocyclic compound of Formula 1 may be used as a host or a dopant. The EML may be formed using a variety of well-known light-emitting materials, in addition to the heterocyclic compound of Formula 1. Alternatively, the EML may also be formed using a well-known host and a dopant. Dopants that may be used to form the EML may include either a fluorescent dopant or a phosphorescent dopant, which are widely known in the art.

Examples of the host include Alq3, 4,4'-N,N'- dicarbazole-biphenyl (CBP), poly(n-vinylcarbazole) (PVK), 9,10-di(naphthalene-2-yl)anthracene (ADN), TCTA, 1,3,5-tris(N-phenylbenzimidazole-2-yl)benzene (TPBI), 3-tert-butyl-9,10-di-2-naphthyl-anthracene (TBADN), E3, and distyrylarylene (DSA), bur are not limited thereto.

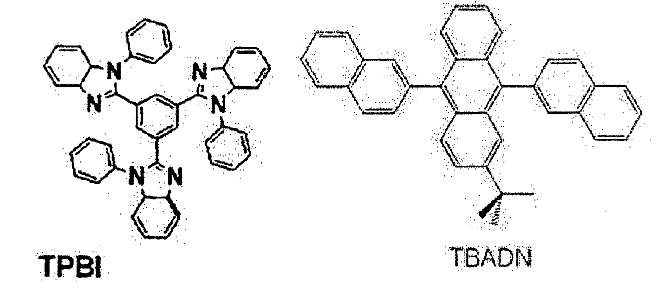

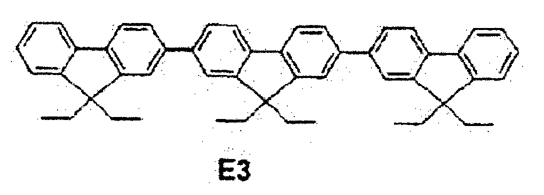

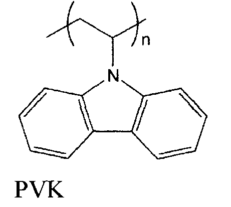

Nonlimiting examples of red dopants include platinum(II)octaethylporphyrin (PtOEP), Ir(piq)₃, Btp₂Ir(acac), and DCJTB.

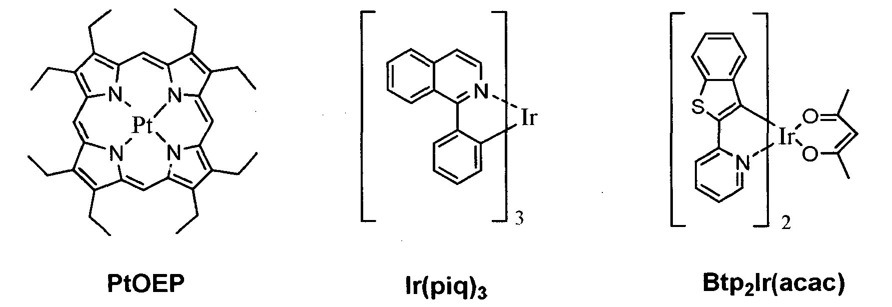

Nonlimiting examples of green dopants include Ir(ppy)₃ (where "ppy" denotes phenylpyridine), Ir(ppy)₂(acac), Ir(mpyp)₃, and C545T.

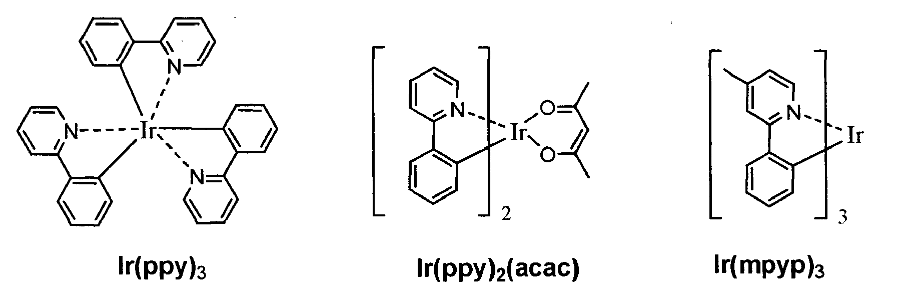

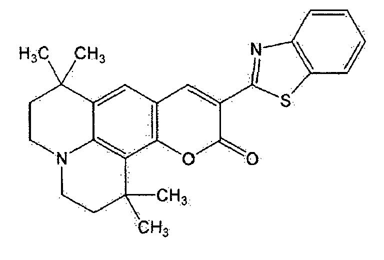

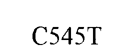

Nonlimiting examples of blue dopants include the heterocyclic compound of Formula 1, F₂Irpic, (F₂ppy)₂Ir(tmd), Ir(dfppz)₃, ter-fluorene, 4,4'-bis(4-diphenylamino-styryl)biphenyl (DPAVBi), and 2,5,8,11-tetra-t-butyl phenylene (TBP).

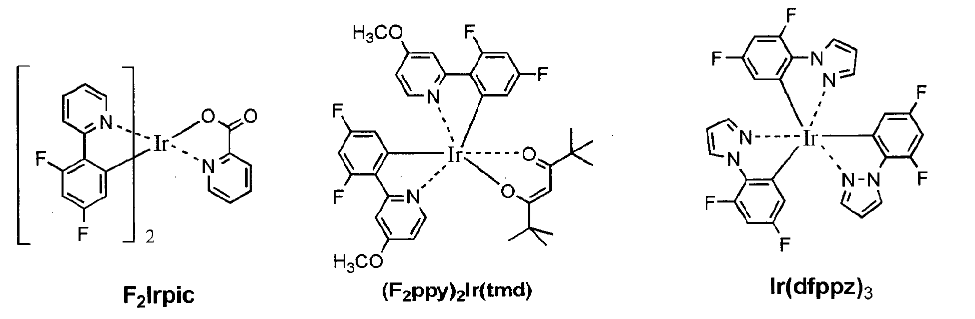

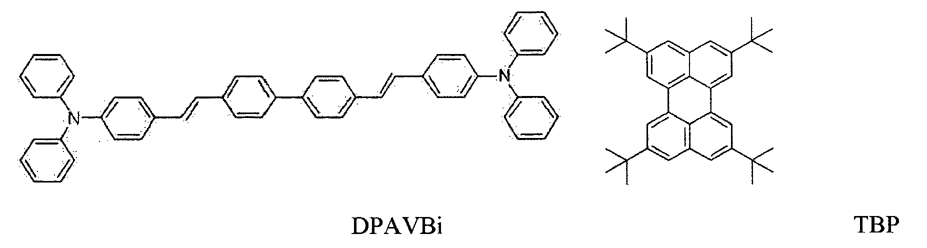

The amount of the dopant may be from about 0.1 to about 20 parts by weight, and in some embodiments, from about 0.5 to about 12 parts by weight, based on 100 parts by weight of the EML material, which is equivalent to the total weight of the host and the dopant. When the amount of the dopant is within these ranges, concentration quenching may be substantially prevented.

The EML may have a thickness of about 100 Å to about 1,000 Å, and in some embodiments, about 200 Å to about 600 Å. When the thickness of the EML is within these ranges, the EML may have good light-emitting characteristics without a substantial increase in driving voltage.

When the EML includes a phosphorescent dopant, a HBL (not shown in FIG. 1) may be formed on the EML in order to prevent diffusion of triplet excitons or holes into the ETL. In this case, the HBL may be formed of any material commonly used to form a HBL. Nonlimiting examples of such HBL materials include oxadiazole derivatives, triazole derivatives, phenathroline derivatives, Balq, and BCP.

The HBL may have a thickness of about 50 Å to about 1,000 Å, and in some embodiments, about 100 Å to about 300 Å. When the thickness of the HBL is within these ranges, the HBL may have good hole blocking characteristics without a substantial increase in driving voltage.

Next, the ETL is formed on the EML (or HBL) by using any of a variety of methods, and in some embodiments, by using vacuum deposition, spin coating, casting, or the like. When the ETL is formed using vacuum deposition or spin coating, the deposition or coating conditions may be similar to those applied to form the HIL, though the deposition or coating conditions may vary according to the material that is used to form the ETL.

The ETL material may include the heterocyclic compound of Formula 1 described above. Alternatively, the ETL may be formed of any material that is widely known in the art. Nonlimiting examples of the ETL material include quinoline derivatives, such as tris(8-quinolinolate)aluminum (Alq3), TAZ, and BAlq.

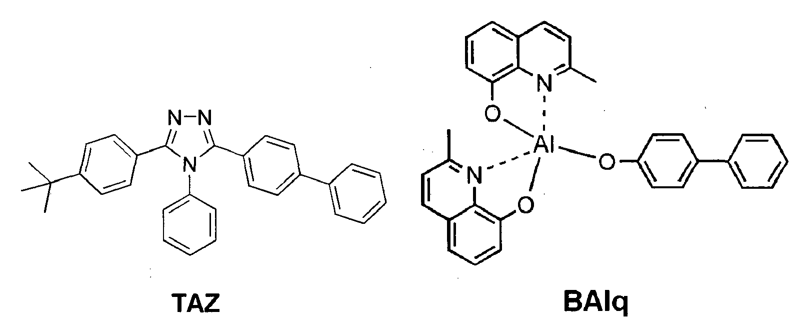

The ETL may have a thickness of about 100 Å to about 1,000 Å, and in some embodiments, about 100 Å to about 500 Å. When the thickness of the ETL is within these ranges, the ETL may have good electron transport characteristics without a substantial increase in driving voltage.

In addition, the EIL, which facilitates injection of electrons from the cathode, may be formed on the ETL.

The EIL may include the heterocyclic compound of Formula 1 described above. In some embodiments well-known EIL materials, such as LiF, NaCl, CsF, Li₂O, or BaO, may be used to form the EIL. The deposition or coating conditions may be similar to those applied to form the HIL, although the deposition and coating conditions may vary according to the material that is used to form the EIL.

The EIL may have a thickness of about 1 Å to 100 Å, and in some embodiments, about 5 Å to about 90 Å. When the thickness of the EIL is within these ranges, the EIL may have good electron injection characteristics without a substantial increase in driving voltage.

Finally, the second electrode may be formed on the EIL by using, for example, vacuum deposition, sputtering, or the like. The second electrode may constitute a cathode or an anode. The material for forming the second electrode may include a metal, an alloy, or an electrically conductive compound, which are materials having a low work function, or a mixture thereof. Nonlimiting examples of such materials include lithium (Li), magnesium (Mg), aluminum (Al), aluminum-lithium (Al-Li), calcium (Ca), magnesium-indium(Mg-In), and magnesium-silver (Mg-Ag). In addition, in order to manufacture a top-emission organic light-emitting device, a transparent cathode formed of a transparent material such as ITO or IZO may be used as the second electrode.

According to embodiments of the present invention, the organic light-emitting device may be included in various types of flat panel display devices, such as in a passive matrix organic light-emitting display device or in an active matrix organic light-emitting display device. In particular, when the organic light-emitting device is included in an active matrix organic light-emitting display device including a thin-film transistor, the first electrode on the substrate may function as a pixel electrode, electrically connected to a source electrode or a drain electrode of the thin-film transistor. Moreover, the organic light-emitting device may also be included in flat panel display devices having double-sided screens.

According to embodiments, the first layer of the organic light-emitting device may be formed of the heterocyclic compound according to the embodiments described above by using a deposition method or a wet method of coating a solution of the heterocyclic compound according to the embodiments described above.

Hereinafter, the present invention will be described in detail with reference to synthesis examples of Compounds 3, 5, 11, 21, 24, 40, 58, 63 and 75 and other examples. However, these examples are for illustrative purposes only and are not intended to limit the scope of the present invention.

### Examples

### Synthesis Example 1: Synthesis of Compound 3

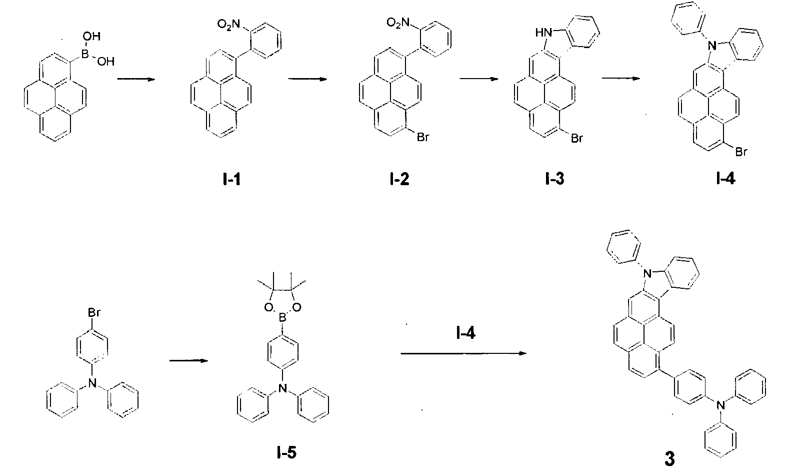

### Synthesis of Intermediate I-1

4.93 g (20.0 mmol) of pyren-1-yl-1-boronic acid, 4.04 g (20.0 mmol) of 2-bromonitrobenzene, 1.15 g (1.0 mmol) of Pd(PPh₃)₄, and 8.29 g (60.0 mmol) of K₂CO₃ were dissolved in 60 mL of a mixed tetrahydrofuran (THF) and H₂O (2:1) solution to obtain a solution, which was then stirred at about 70°C for about 5 hours. The reaction solution was cooled to room temperature, and 100 mL of water was added thereto, followed by three times of extraction with 50 mL of ethylether. The organic phase was collected, and was dried using magnesium sulfate to evaporate the solvent. The residue was separated and purified using silica gel column chromatography to obtain 5.95 g of Intermediate I-1 (Yield: 92 %). This compound was identified using liquid chromatography-mass spectroscopy (LC-MS) and nuclear magnetic resonance (NMR). C₂₂H₁₃NO₂ : M+ 323.1

¹H NMR (CDCl₃, 400MHz) δ (ppm) 8.22-8.20 (d, 2H), 8.17-8.10 (m, 4H), 8.03-8.01 (d, 1H), 8.01-7.98 (d, 1H), 7.88-7.85 (d, 1H), 7.69-7.67 (m, 2H), 7.66-7.56 (m, 2H)

### Synthesis of IntermediateI-2

4.85 g (15.0 mmol) of Intermediate I-1 was dissolved in 100 mL of dichloromethane 100 ml to obtain a solution, and 1.75 ml (15.0 mmol) of bromine (Br₂) was slowly dropwise added to the solution at about 0°C to obtain a reaction solution. The reaction solution was stirred at room temperature for about 12 hours. 60 mL of water and 30 mL of a 20% aqueous thiosodium sulfate solution were added to the reaction solution, followed by three times of extraction with 80 mL of dichloromethane. The organic phase was collected, and was dried using magnesium sulfate to evaporate the solvent. The residue was separated and purified by silica gel column chromatography, followed by recrystallization with a dichloromethane/EtOAc/Et2O solution to obtain 2.53 g of Intemediate I-2 (Yield 42 %). This compound was identified using LC-MS and NMR C₂₂H₁₂BrNO₂ : M+ 401.0

¹H NMR (CDCl₃, 400MHz) δ (ppm) 8.50-8.47 (d, 1H), 8.27-8.22 (dd, 2H), 8.21-8.18 (dd, 1H), 8.16-8.13 (dd, 1H), 8.00-7.97 (d, 1H), 7.96-7.88 (dd, 2H), 7.78-7.73 (dt, 1H), 7.72-7.69 (d, 1H), 7.68-7.63 (dt, 1H), 7.59-7.56 (dd, 1H)

### Synthesis of Intermediate I-3

4.02 g (10.0 mmol) of Intermediate I-2 and 5.77 g (22 mmol) of triphenylphosphine (PPh₃) were dissolved in 30 mL of 1, 2-dichlorobenzene to obtain a solution, which was then stirred at about 170°C for about 12 hours. The reaction solution was cooled to room temperature, and the solvent was removed therefrom under vacuum conditions, followed by three times of extraction with 50 mL of water and 50 mL of dichloromethane. The organic phase was collected, and was dried using magnesium sulfate to evaporate the solvent. The residue was separated and purified using silica gel column chromatography to obtain 2.41 g of Intermediate I-3 (Yield: 65 %). This compound was identified using LC-CM and NMR. C₂₂H₁₂BrN : M+ 369.0

¹H NMR (CDCl₃, 400MHz) δ (ppm) 9.16-9.13 (d, 1H), 8.80-8.78 (d, 1H), 8.60 (s, 1H), 8.43-8.40 (d, 1H), 8.31-8.28 (d, 1H), 8.23-8.21 (d, 1H), 8.18 (s, 1H), 8.10-8.07 (d, 1H), 7.67-7.65 (d, 1H), 7.61-7.57 (dt, 1H), 7.50-7.46 (dt, 1H)

### Synthesis of Intermediate I-4

3.70 g (10.0 mmol) of Intermediate I-3, 3.06 g (15.0 mmol) of iodobenzene, 0.19 g (1.0 mmol) of CuI, 0.05 g (0.2 mmol) of 18-Crown-6, and 4.15 g (30.0 mmol) of K₂CO₃ were dissolved in 30 mL of 1,3-dimethyl-3,4,5,6-tetrahydro-2(1H)-pyrimidinone (DMPU) to obtain a solution, which was then stirred at about 170°C for about 12 hours. The reaction solution was cooled to room temperature, followed by three times of extraction with 50 mL of water and 50 mL of dichloromethane. The organic phase was collected, and was dried using magnesium sulfate to evaporate the solvent. The residue was separated and purified using silica gel column chromatography to obtain 3.39 g of Intermediate I-4 (Yield: 76 %). This compound was identified using LC-MS and NMR. C₂₈H₁₆BrN : M+ 445.0

¹H NMR (CDCl₃, 400MHz) δ (ppm) 9.21-9.17 (dd, 1H), 8.86-8.84 (d, 1H), 8.46-8.35 (dd, 1H), 8.31-8.27 (dd, 1H), 8.25-8.17 (dd, 1H), 8.12-8.08 (d, 2H), 7.94-7.91 (d, 1H), 7.75-7.67 (m, 4H), 7.63-7.49 (m, 4H)

### Synthesis of Intermediate I-5

3.24 g (10.0 mmol) of 4-bromotriphenylamine, 2.54 g (10.0 mmol) of bis(pinacolato)diborone, 0.36 g(0.5 mmol) of [1,1'-bis(diphenylphosphino)ferrocene]dichloro palladium (II) (hereinafter, PdCl₂(dppf)₂), and 2.94 g (30.0 mmol) of KOAc were dissolved in 40 mL of dimethylsulfoxide (DMSO) to obtain a solution, which was then stirred at about 80°C for about 6 hours. The reaction solution was cooled to room temperature, followed by three times of extraction with 50 mL of water and 50 mL of diethylether. The organic phase was collected, and was dried using magnesium sulfate to evaporate the solvent. The residue was separated and purified using silica gel column chromatography to obtain 2.57 g of Intermediate I-5 (Yield: 89 %). This compound was identified using LC-MS and NMR C₂₄H₂₆BNO₂ : M+ 371.2

¹H NMR (CDCl₃, 400MHz) δ (ppm) 7.67-7.63 (m, 2H), 7.30-7.21 (m, 4H), 7.14-7.06 (m, 4H), 7.05-7.00 (m, 4H), 1.32 (s, 12H)

### Synthesis of Compound 3

2.23 g (5.0 mmol) of Intermediate I-4, 1.86 g (5.0 mmol) of Intermediate I-5, 0.29 g (0.25 mmol) of Pd(PPh₃)₄, and 2.07 g (15.0 mmol) of K₂CO₃ were dissolved in 30 mL of a mixed solution THF/H₂O (2:1) to obtain a solution, which was then stirred at about 70°C for about 5 hours. The reaction solution was cooled to room temperature, followed by three times of extraction with 50 mL of water and 50 mL of diethylether. The organic phase was collected, and was dried using magnesium sulfate to evaporate the solvent. The residue was separated and purified using silica gel column chromatography to obtain 2.11 g of Compound 3 (Yield: 72 %). This compound was identified using LC-MS and NMR. C₄₆H₃₀N₂ : M+ 610.2

¹H NMR (CDCl₃, 400MHz) δ (ppm) 9.26-9.22 (d, 1H), 8.92-8.90 (dd, 1H), 8.43-8.41 (d, 1H), 8.34-8.31(d, 1H), 8.27-8.24 (d, 1H), 8.10 (s, 1H), 8.03-7.98 (dd, 2H), 7.74-7.72 (m, 4H), 7.59-7.52 (m, 6H), 7.35-7.22 (m, 10), 7.10-7.05 (dt, 2H)

### Synthesis Example 2: Synthesis of Compound 5

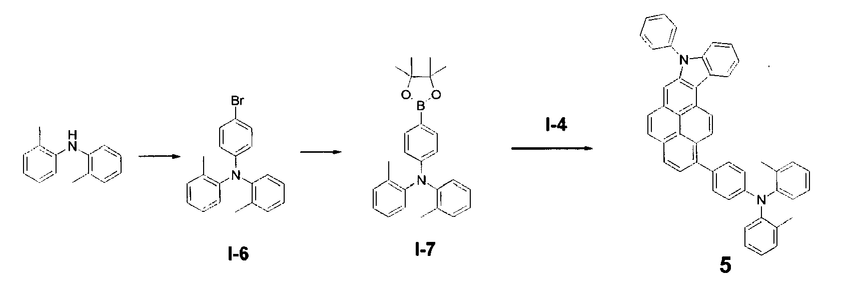

### Synthesis of Intermediate I-6

1.97g (10.0 mmol) of di-ortho-tolylamine, 4.24 g (15.0 mmol) of 4-bromoiodobenzene, 0.18 g (0.2 mmol) of Pd₂(dba)₃, 0.04 g (0.4 mmol) of P*t*Bu₃, and 1.44 g (15.0 mmol) of KOtBu were dissolved in 40 mL of toluene to obtain a solution, which was then stirred at about 85°C for about 4 hours. The reaction solution was cooled to room temperature, followed by three times of extraction with 30 mL of water and 30 mL of diethylether. The organic phase was collected, and was dried using magnesium sulfate to evaporate the solvent. The residue was separated and purified using silica gel column chromatography to obtain 2.18 g of Intermediate I-6 (Yield: 62 %). This compound was identified using LC-MS and NMR. C₂₀H₁₈gBrN : M+ 351.1

¹H NMR (CDCl₃, 400MHz) δ (ppm) 7.29-7.24 (m, 4H), 7.18-7.13 (dt, 2H), 6.92-6.84 (m, 4H), 1.96 (s, 6H)

### Synthesis of Intermediate I-7

3.52 g (10.0 mmol) of Intermediate I-6, 2.54 g (10.0 mmol) of bis(pinacolato)diborone, 0.36 g (0.5 mmol) of PdCl₂(dppf)₂, and 2.94 g (30.0 mmol) of KOAc were dissolved in 40 mL of DMSO to obtain a solution, which was then stirred at about 80°C for about 6 hours. The reaction solution was cooled to room temperature, followed by three times of extraction with 50 mL of water and 50 mL of diethylether. The organic phase was collected, and was dried using magnesium sulfate to evaporate the solvent. The residue was separated and purified using silica gel column chromatography to obtain 3.03 g of Intermediate I-7 (Yield: 76 %). This compound was identified using LC-MS and NMR. C₂₆H₃₀BNO₂ : M+ 399.2

¹H NMR (CDCl₃, 400MHz) δ (ppm) 7.62-7.60 (d, 1H), 7.22-7.20 (dd, 2H), 7.18-7.07(m, 4H), 7.00-6.98 (dd, 2H), 6.63-6.60 (d, 2H), 2.02 (s, 6H), 1.33 (s, 12H)

### Synthesis of Compound 5

2.42 g of Compound 5 was synthesized from Intermediate I-5 and Intermediate I-4 in the same manner as in the synthesis of Compound 3 (Yield: 76 %). This compound was identified using LC-MS and NMR. C₄₈H₃₄N₂ : M+ 638.3

¹H NMR (CDCl₃, 400MHz) δ (ppm) 8.96-8.94 (d, 1H), 8.23-8.17 (m, 2H), 8.09-8.07 (d, 1H), 8.04 (s, 1H), 7.94-7.88 (m, 3H), 7.51-7.47 (m, 4H), 7.37-7.31 (m, 4H), 7.28-7.22 (m, 4H), 7.17-7.13 (dt, 2H), 6.90-6.86 (dt, 2H), 6.78-6.73 (m, 4H), 2.02 (s, 6H)

### Synthesis Example 3: Synthesis of Compound 11

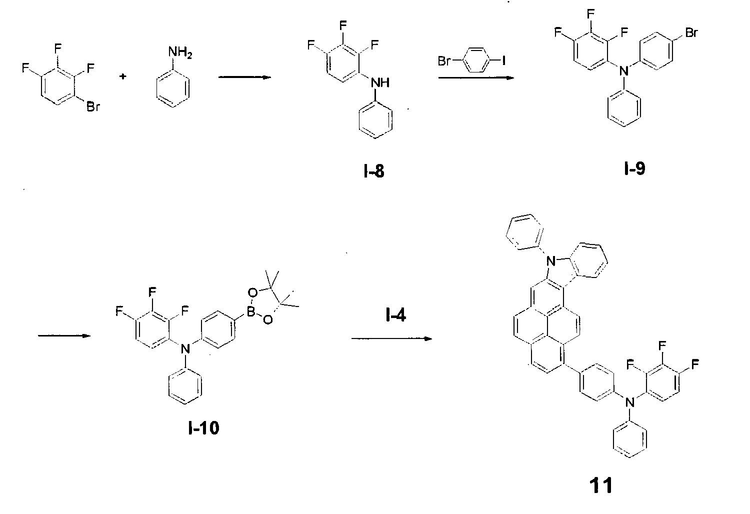

### Synthesis of IntermediateI-8

4.22 g (20.0 mmol) of 1-bromo-2,3,4-trifluorobenzene, 2.79 g (30.0 mmol) of aniline, 0.37 g (0.4 mmol) of Pd₂(dba)₃, 0.08 g (0.4 mmol) of P*t*Bu₃, and 2.88 g (30.0 mmol) of KO*t*Bu were dissolved in 60 mL of toluene to obtain a solution, which was then stirred at about 85°C for about 4 hours. The reaction solution was cooled to room temperature, followed by three times of extraction with 50 mL of water and 50 mL of diethylether. The organic phase was collected, and was dried using magnesium sulfate to evaporate the solvent. The residue was separated and purified using silica gel column chromatography to obtain 3.93 g of Intermediate I-8 (Yield: 88 %). This compound was identified using LC-MS and NMR. C₁₂H₈F₃N : M+ 223.1

¹H NMR (CDCl₃, 400MHz) δ (ppm) 7.34-7.26 (m, 2H), 7.04-7.00 (m, 3H), 6.99-6.92 (m, 1H), 6.87-6.77 (m, 1H), 5.62 (s, 1H)

### Synthesis of Intermediate I-9

2.23 g (10.0 mmol) of Intermediate I-8, 4.23 g (15.0 mmol) of 4-bromoiodobenzene, 0.18 g (0.2 mmol) of Pd₂(dba)₃, 0.04 g (0.4 mmol) of P*t*Bu₃, and 1.44 g (15.0 mmol) of KO*t*Bu were dissolved in 40 mL of toluene to obtain a solution, which was then stirred at about 85°C for about 4 hours. The reaction solution was cooled to room temperature, followed by three times of extraction with 30 mL of water and 30 mL of diethylether. The organic phase was collected, and was dried using magnesium sulfate to evaporate the solvent. The residue was separated and purified using silica gel column chromatography to obtain 2.38 g of Intermediate I-9 (Yield: 63 %). This compound was identified using LC-MS and NMR. C₁₈H₁₁BrF₃N : M+ 377.0

¹H NMR (CDCl₃, 400MHz) δ (ppm) 7.38-7.31 (m, 2H), 7.30-7.25 (m, 2H), 7.11-7.07 (dt, 1H), 7.06-7.01 (m, 2H), 6.98-6.91 (m, 2H), 6.88-6.85 (m, 2H)

### Synthesis of Intermediate I-10

3.78 g (10.0 mmol) of Intermediate I-9, 2.54 g (10.0 mmol) of bis(pinacolato)diborone, 0.36 g (0.5 mmol) of PdCl₂(dppf)₂, and 2.94 g (30.0 mmol) of KOAc were dissolved in 40 mL of DMSO to obtain a solution, which was then stirred at about 80°C for about 6 hours. The reaction solution was cooled to room temperature, followed by three times of extraction with 50 mL of water and 50 mL of diethylether. The organic phase was collected, and was dried using magnesium sulfate to evaporate the solvent. The residue was separated and purified using silica gel column chromatography to obtain 3.40 g of Intermediate I-10 (Yield: 80 %). This compound was identified using LC-MS and NMR. C₂₄H₂₃BF₃NO₂ : M+ 425.2

¹H NMR (CDCl₃, 400MHz) δ (ppm) 7.72-7.68 (m, 2H), 7.32-7.25 (m, 2H), 7.13-7.05 (m, 3H), 6.99-6.85 (m, 4H), 1.35 (s, 12H)

### Synthesis of Compound 11

2.39 g of Compound 11 was synthesized from Intermediate I-10 and Intermediate I-4 in the same manner as in the synthesis of Compound 3 (Yield: 72 %). This compound was identified using LC-MS and NMR. C₄₆H₂₇F₃N₂ : M+ 664.2

¹H NMR (CDCl₃, 400MHz) δ (ppm) 9.25-9.22 (d, 1H), 8.92-8.89 (d, 1H), 8.43-8.40 (d, 1H), 8.33-8.30 (d, 1H), 8.23-8.20 (d, 1H), 8.10 (s, 1H), 7.99 (t, 2H), 7.74-7.72 (m, 4H), 7.62-7.53 (m, 6H), 7.34 (dt, 2H), 7.18-7.15 (d, 4H), 7.12-6.91 (m, 3H)

### Synthesis Example 4: Synthesis of Compound 21

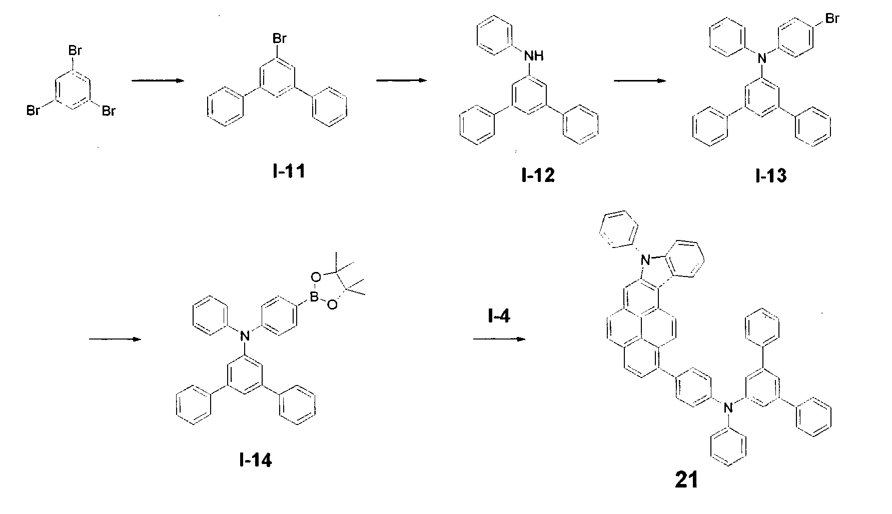

### Synthesis of Intermediate I-11

6.3g (20.0 mmol) of 1,3,5-tribromobenzene, 4.88g (40.0 mmol) of 1-phenylboronic acid, 2.31 g (2.0 mmol) of Pd(PPh₃)₁, and 16.6 g (120.0 mmol) of K₂CO₃ were dissolved in 120 mL of a mixed solution THF/H₂O (2:1) to obtain a solution, which was then stirred at about 70°C for about 5 hours. The reaction solution was cooled to room temperature, followed by three times of extraction with 120 mL of water and 100 mL of diethylether. The organic phase was collected, and was dried using magnesium sulfate to evaporate the solvent. The residue was separated and purified using silica gel column chromatography to obtain 3.83 g of Intermediate I-11 (Yield: 62 %). This compound was identified using LC-MS and NMR. C₁₈H₁₃Br : M+ 308.2

¹H NMR (CDCl₃, 400MHz) δ (ppm) 7.70 (s, 3H), 7.62-7.58 (m, 4H), 7.48-7.47 (t, 1H), 7.46-7.42 (m, 3H), 7.41-7.35 (m, 2H)

### Synthesis of Intermediate I-12

2.73 g of Intermediate I-12 was synthesized from Intermediate I-11 and aniline in the same manner as in the synthesis of Intermediate I-8 (Yield: 85 %). This compound was identified using LC-MS and NMR. C₂₄H₁₉N : M+ 321.2

¹H NMR (CDCl₃, 400MHz) δ (ppm) 7.76-7.75 (t, 1H), 7.54-7.50 (m, 6H), 7.34-7.22 (m, 8H), 7.13-7.11 (m, 2H), 7.10-7.06 (dt, 1H), 6.52 (s, 1H)

### Synthesis of Intermediate I-13

3.14g of Intermediate I-13 was synthesized from Intermediate I-12 in the same manner as in the synthesis of Intermediate I-9 (Yield: 66 %). This compound was identified using LC-MS and NMR. C₃₀H₂₂BrN : M+ 475.1

¹H NMR (CDCl₃, 400MHz) δ (ppm) 7.90 (t, 1H), 7.50 (d, 2H), 7.44-7.43 (d, 8H), 7.34-7.29 (m, 6H), 7.12-7.10 (d, 2H), 7.04-7.00 (m, 1H), 6.91-6.89 (m, 2H)

### Synthesis of Intermediate I-14

4.08g of Intermediate I-14 was synthesized from Intermediate I-13 in the same manner as in the synthesis of Intermediate I-10 (Yield: 78 %). This compound was identified using LC-MS and NMR. C₃₆H₃₄BNO₂ : M+ 523.3

¹H NMR (CDCl₃, 400MHz) δ (ppm) 7.91 (t, 1H), 7.71-7.67 (m, 2H), 7.50 (d, 2H), 7.44-7.42 (d, 8H), 7.34-7.29 (m, 4H), 7.24-7.22 (d, 2H), 7.14-7.10 (m, 1H), 6.81-6.79 (m, 2H), 1.35 (s, 12H)

### Synthesis of Compound 21

2.86 g of Compound 21 was synthesized from Intermediate I-14 and Intermediate I-4 in the same manner as in the synthesis of Compound 3 (Yield: 75 %). This compound was identified using LC-MS and NMR. C₅₈H₃₈N₂ : M+ 762.3

¹H NMR (CDCl₃, 400MHz) δ (ppm) 9.16-9.14 (d, 1H), 8.73-8.67 (m, 2H), 8.46-8.44 (d, 1H), 8.24 (s, 1H), 7.94-7.90 (m, 4H), 7.62-7.58 (m, 6H), 7.54-7.53 (d, 8H), 7.45-7.39 (m, 10H), 7.24-7.20 (m, 1H), 7.15-7.12 (m, 2H), 7.01-6.99 (d, 2H)

### Synthesis Example 5: Synthesis of Compound 24

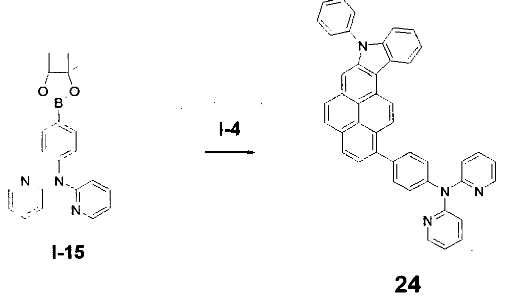

2.18 g of Compound 24 was synthesized from Intermediate I-4 and Intermediate I-15, which was synthesized using a known method, in the same manner as in the synthesis of Compound 3 (Yield: 71 %). This compound was identified using LC-MS and NMR C₄₄H₂₈N₄ : M+ 612.2

¹H NMR (CDCl₃, 400MHz) δ (ppm) 9.23-9.21 (d, 1H), 8.63-8.49 (m, 4H), 8.29-8.27 (d, 1H), 8.14 (s, 1H), 7.94-7.88 (m, 3H), 7.78-7.68 (m, 8H), 7.47-7.40 (m, 6H), 7.11-7.08 (m, 2H), 7.02-6.99(m, 2H)

### Synthesis Example 6: Synthesis of Compound 40

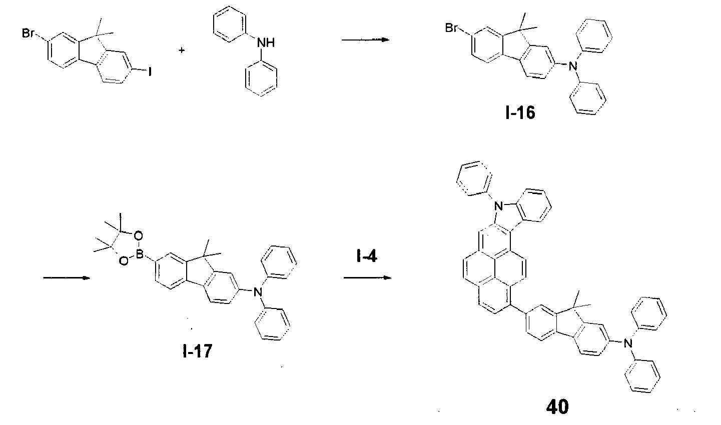

### Synthesis of Intermediate I-16

3.04g of Intermediate I-16 was synthesized from 2-bromo-7-iodo-9,9-dimethylfluorene and diphenylamine in the same manner as in the synthesis of Intermediate I-13 (Yield: 69 %). This compound was identified using LC-MS and NMR. C₂₇H₂₂BrN : M+ 439.1

¹H NMR (CDCl₃, 400MHz) δ (ppm) 8.09-8.07 (d, 1H), 7.94-7.92 (d, 1H), 7.74-7.68 (m, 2H), 7.53-7.49 (t, 4H), 7.30 (s, 1H), 7.24-7.20 (m, 2H), 7.04-7.02 (m, 1H), 6.98-6.96 (d, 4H), 1.86 (s, 6H)

### Synthesis of Intermediate I-17

3.99 g of Intermediate I-17 was synthesized from Intermediate I-16 in the same manner as in the synthesis of Intermediate I-14 (Yield: 82 %). This compound was identified using LC-MS and NMR. C₃₃H₃₄BNO₂ : M+ 487.3

¹H NMR (CDCl₃, 400MHz) δ (ppm) 8.10-8.08 (d, 1H), 7.95-7.92 (m, 2H), 7.79-7.77 (d, 1H), 7.53-7.49 (m, 4H), 7.36-7.35 (m, 1H), 7.24-7.20 (m, 2H), 7.14-7.12 (dd, 1H), 6.98-6.96 (m, 4H), 1.86 (s, 6H), 1.36 (s, 12H)

### Synthesis of Compound 40

2.65 g of Compound 40 was synthesized from Intermediate I-17 and Intermediate I-4 in the same manner as in the synthesis of Compound 3 (Yield: 73 %). This compound was identified using LC-MS and NMR. C₅₅H₃₈N₂ : M+ 726.3

1H NMR (CDCl₃, 400MHz) δ (ppm) 9.17-9.15 (d, 1H), 8.73-8.67 (m, 2H), 8.57-8.54 (m, 2H), 8.34-8.32 (d, 1H), 8.28-8.26 (d, 1H), 8.03-8.01 (d, 1H), 7.85-7.83 (d, 1H), 7.69-7.67 (m, 4H), 7.55-7.49 (m, 8H), 7.35-7.33 (d, 1H), 7.30-7.29 (d, 1H), 7.24 (d, 1H), 7.13-7.11 (dd, 1H), 7.04-7.00 (dt, 2H), 6.94-6.92 (dd, 1H), 6.88-6.86 (dd, 4H), 1.85 (s, 6H)

### Synthesis Example 7: Synthesis of Compound 58

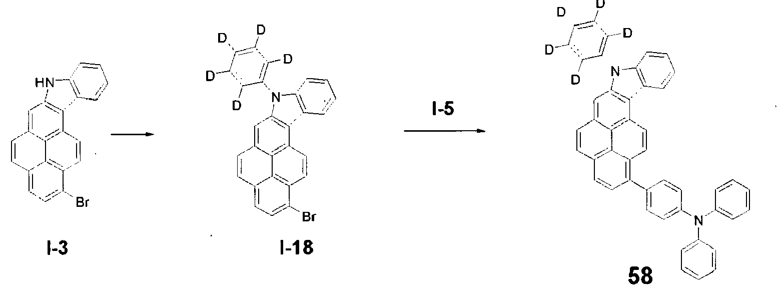

### Synthesis of Intermediate I-18

3.48 g of Intermediate I-18 was synthesized from Intermediate I-3 and chlorobenzene-d₅ in the same manner as in the synthesis of Intermediate I-4 (Yield: 77 %). This compound was identified using LC-MS and NMR. C₂₈H₁₁D₅BrN : M+ 450.1

¹H NMR (CDCl₃, 400MHz) δ (ppm) 8.89-8.87 (d, 1H), 8.85-8.83 (d, 1H), 8.44-8.42 (d, 1H), 8.23-8.19 (m, 2H), 8.14 (s, 1), 8.03-7.97 (dd, 2H), 7.37-7.29 (m, 3H)

### Synthesis of Compound 58

2.52 g of Compound 58 was synthesized from Intermediate I-18 and Intermediate I-5 in the same manner as in the synthesis of Compound 3 (Yield: 82 %). This compound was identified using LC-MS and NMR. C₄₆H₂₅D₅N₂ : M+ 615.3

¹H NMR (CDCl₃, 400MHz) δ (ppm) 9.26-9.24 (d, 1H), 8.63-8.57 (m, 2H), 8.49-8.47 (d, 1H), 8.44 (s, 1H), 8.14-8.10 (t, 3H), 7.65-7.59 (m, 9H), 7.34-7.32 (m, 2H), 7.25-7.21 (m, 2H), 7.10-7.08 (dd, 4H)

### Synthesis Example 8: Synthesis of Compound 63

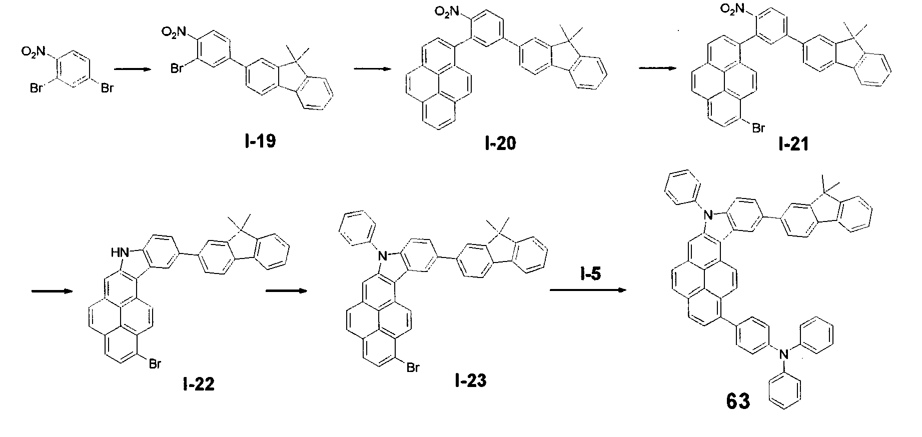

### Synthesis of Intermediate I-19

5.62g (20.0 mmol) of 2,4-dibromo-1-nitrobenzene, 4.52 g (19.0 mmol) of dimethylfluorene-2-boronic acid, 1.15 g (1.0 mmol) of Pd(PPh₃)₄, and 8.29 g (60.0 mmol) of K₂CO₃ were dissolved in 80 mL of a mixed solution THY/H₂O (2:1) to obtain a solution, which was then stirred at about 70°C for about 5 hours. The reaction solution was cooled to room temperature, followed by three times of extraction with 60 mL of water and 60 mL of diethylether. The organic phase was collected, and was dried using magnesium sulfate to evaporate the solvent. The residue was separated and purified using silica gel column chromatography to obtain 4.57 g of Intermediate I-19 (Yield: 58 %) This compound was identified using LC-MS and NMR. C₂₁H₁₆BrNO₂ : M+ 393.0

¹H NMR (CDCl₃, 400MHz) δ (ppm) 8.06-8.04 (d, 1H), 7.79-7.77 (m, 1H), 7.65-7.64 (d, 1H), 7.60-7.57 (dd, 1H), 7.50-7.48 (dd, 1H), 7.44-7.41 (m, 1H), 7.28-7.26 (dd, 1H), 7.10-7.03 (m, 3H), 1.90 (s, 6H)

### Synthesis of Intermediate I-20

4.28 g of Intermediate I-20 was synthesized from Intermediate I-19 and pyren-1-yl-boronic acid in the same manner as in the synthesis of Intermediate I-1 (Yield: 83 %). This compound was identified using LC-MS and NMR. C₃₇H₂₅NO₂: M+ 515.2

¹H NMR (CDCl₃, 400MHz) δ (ppm) 8.67-8.66 (d, 1H), 8.38-8.36 (d, 1H), 8.21-8.06 (m, 8H), 7.80-7.77 (m, 2H), 7.50-7.48 (m, 1H), 7.34-7.31 (m, 1H), 7.21-7.18 (dd, 1H), 7.09-7.03 (m, 4H), 1.89 (s, 6H)

### Synthesis of Intermediate I-21

2.79 g of Intermediate I-21 was synthesized from Intermediate I-20 in the same manner as in the synthesis of Intermediate I-2 (Yield: 47 %). This compound was identified using LC-MS and NMR. C₃₇H₂₄BrNO₂: M+ 593.1

¹H NMR (CDCl₃, 400MHz) δ (ppm) 8.85-8.83 (d, 1H), 8.47-8.46 (dd, 1H), 8.34-8.32 (d, 1H), 8.13-8.04 (m, 4H), 7.98-7.93 (m, 2H), 7.79-7.77 (m, 2H), 7.50-7.48 (dd, 1H), 7.24-7.21 (dt, 1H), 7.11-7.08 (m, 1H), 6.99-6.93 (m, 4H), 1.87 (s, 6H)

### Synthesis of Intermediate I-22

3.21 g of Intermediate I-22 was synthesized from Intermediate I-21 in the same manner as in the synthesis of Intermediate I-3 (Yield: 57 %). This compound was identified using LC-MS and NMR. C₃₇H₂₄BrN : M+ 561.1

¹H NMR (CDCl₃, 400MHz) δ (ppm) 10.38 (s, 1H), 8.85-8.77 (dd, 2H), 8.49-8.47 (d, 1H), 8.26-8.24 (d, 1H), 8.02-7.96 (m, 4H), 7.79-7.77 (dd, 1H), 7.50-7.48 (d, 1H), 7.44-7.42 (d, 1H), 7.34-7.32 (dd, 1H), 7.24-7.20 (dt, 1H), 7.02-6.93 (m, 4H), 1.87 (s, 6H)

### Synthesis of Intermediate I-23

4.98 g of Intermediate I-23 was synthesized from Intermediate I-22 in the same manner as in the synthesis of Intermediate I-4 (Yield: 78 %). This compound was identified using LC-MS and NMR. C₄₃H₂₈BrN : M+ 637.1

¹H NMR (CDCl₃, 400MHz) δ (ppm) 9.05-9.02 (dd, 2H), 8.54-8.52 (d, 1H), 8.31-8.29 (d, 1H), 8.19 (s, 1H), 8.13-8.07 (dd, 2H), 7.93-7.91 (d, 1H), 7.79-7.77 (dd, 1H), 7.67 (dd, 1H), 7.51-7.47 (m, 5H), 7.37-7.31 (m, 1H), 7.24-7.21 (dt, 1H), 7.02-6.93 (m, 5H), 1.86 (d, 6H)

### Synthesis of Compound 63

2.93 g of Compound 63 was synthesized from Intermediate I-23 and Intermediate I-5 in the same manner as in the synthesis of Compound 3 (Yield: 73 %). This compound was identified using LC-MS and NMR. C₆₁H₄₂N₂ : M+ 802.3

¹H NMR (CDCl₃, 400MHz) δ (ppm) 9.11-9.09 (d, 1H), 8.60-8.57 (d, 1H), 8.49-8.48 (d, 1H), 8.29 (s, 1H), 8.23-8.18 (m, 4H), 7.99-7.97 (dd, 1H), 7.77 (dd, 1H), 7.61-7.58 (m, 5H), 7.46-7.39 (m, 7H), 7.34-7.31 (dt, 1H), 7.22-7.13 (m, 5H), 7.04-7.00 (m, 2H), 6.95-6.91 (m, 2H), 6.80-6.78 (m, 4H), 1.86 (s, 6H)

### Synthesis Example 9: Synthesis of Compound 75

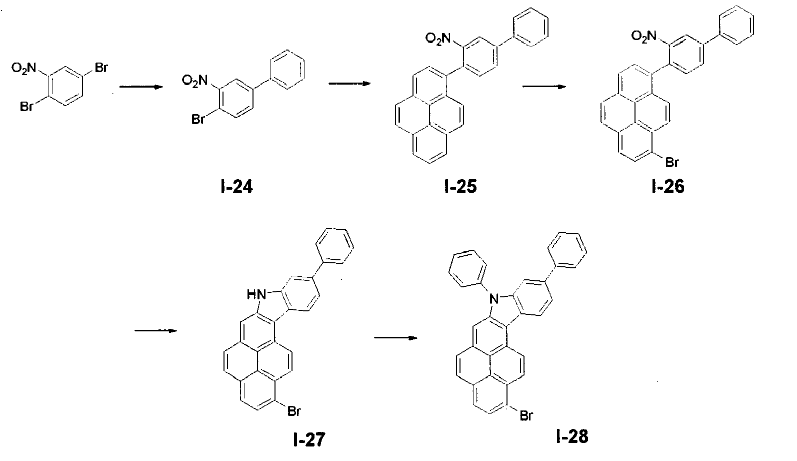

### Synthesis of Intermediate I-24

3.39 g of Intermediate I-24 was synthesized from 1,4-dibromo-2-nitrobenzene and phenylboronic acid in the same manner as in the synthesis of Intermediate I-19 (Yield: 61 %). This compound was identified using LC-MS and NMR. C₁₂H₈BrNO₂ : M+ 277.0

¹H NMR (CDCl₃, 400MHz) δ (ppm) 8.15-8.14 (dd, 1H), 7.80-7.78 (m, 2H), 7.49-7.47 (d, 1H), 7.34-7.30 (m, 1H), 7.19-7.15 (m, 2H), 7.11-7.08 (dd, 1H)

### Synthesis of Intermediate I-25

3.16 g of Intermediate I-25 was synthesized from Intermediate I-24 in the same manner as in the synthesis of Intermediate I-20 (Yield: 79 %). This compound was identified using LC-MS and NMR. C₂gH₁₇NO₂ : M+ 399.1

¹H NMR (CDCl₃, 400MHz) δ (ppm) 8.68-8.66 (d, 1H), 8.49-8.42 (m, 6H), 8.29-8.27 (d, 1H), 8.09-8.06 (d, 1H), 7.91-7.89 (m, 2H), 7.60-7.59 (dd, 1H), 7.54-7.50 (m, 1H), 7.29-7.25 (t, 2H), 7.14-7.12 (d, 1H), 6.92-6.89 (dd, 1H)

### Synthesis of Intermediate I-26

1.96 g of Intermediate I-26 was synthesized from Intermediate I-25 in the same manner as in the synthesis of Intermediate I-21 (Yield: 41 %). This compound was identified using LC-MS and NMR. C₂₈H₁₆BrNO₂ : M+ 477.0

¹H NMR (CDCl₃, 400MHz) δ (ppm) 8.95-8.93 (d, 1H), 8.54-8.52 (d, 1H), 8.23-8.18 (m, 4H), 7.95-7.92 (d, 1H), 7.89-7.86 (d, 1H), 7.71-7.68 (m, 2H), 7.52-7.51 (dd, 1H), 7.44-7.40 (m, 1H), 7.29-7.25 (t, 2H), 7.14-7.12 (d, 1H), 6.96-6.97 (dd, 1H)

### Synthesis of Intermediate I-27

2.72 g of Intermediate I-27 was synthesized from Intermediate I-26 in the same manner as in the synthesis of Intermediate I-22 (Yield: 61 %). This compound was identified using LC-MS and NMR. C₂₈H₁₆BrN : M+ 445.0

¹H NMR (CDCl₃, 400MHz) δ (ppm) 10.29 (s, 1H), 9.05-9.01 (dd, 2H), 8.49-8.47 (d, 1H), 8.36-8.33 (m, 2H), 8.15-8.06 (m, 4H), 7.70-7.64 (m, 3H), 7.50-7.46 (t, 2H), 7.35-7.32 (m, 1H)

### Synthesis of Intermediate I-28

3.97 g of Intermediate I-28 was synthesized from Intermediate I-27 in the same manner as in the synthesis of Intermediate I-23 (Yield: 76 %). This compound was identified using LC-MS and NMR. C₃₄H₂₀BrN : M+ 521.1

¹H NMR (CDCl₃, 400MHz) δ (ppm) 9.09-9.03 (dd, 2H), 8.64-8.62 (d, 1H), 8.50-8.48 (dd, 1H), 8.31-8.29 (d, 1H), 8.23-8.21 (d, 1H), 8.19 (s, 1H), 8.13-8.07 (dd, 2H), 7.72-7.70 (m, 2H), 7.61-7.50 (m, 7H), 7.46-7.39 (m, 2H)

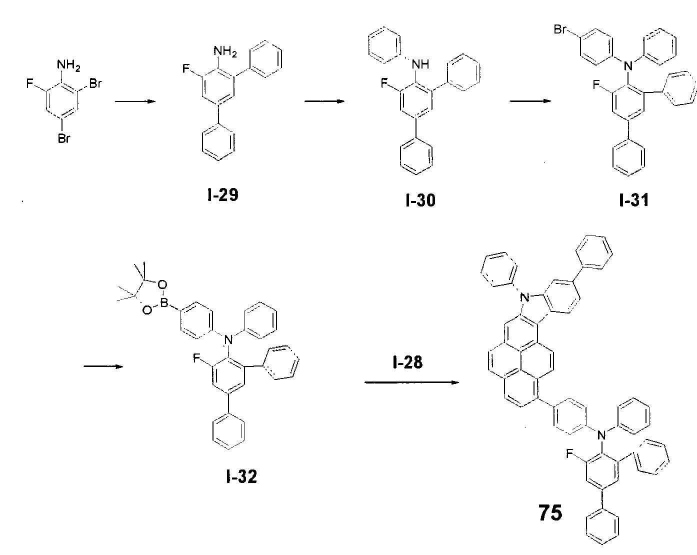

### Synthesis of Intermediate I-29

5.38g (20.0 mmol) of 2,4-dibromo-6-fluoro-phenylamine, 5.36 g (44.0 mmol) of phenylboronic acid, 1.15 g (1.0 mmol) of Pd(PPh₃)₄, and 8.29 g (60.0 mmol) of K₂CO₃ were dissolved in 80 mL of a mixed solution THF/H₂O (2:1) to obtain a solution, which was then stirred at about 70°C for about 5 hours. The reaction solution was cooled to room temperature, followed by three times of extraction with 60 mL of water and 60 mL of diethylether. The organic phase was collected, and was dried using magnesium sulfate to evaporate the solvent. The residue was separated and purified using silica gel column chromatography to obtain 4.11 g of Intermediate I-29 (Yield: 78 %) This compound was identified using LC-MS and NMR. C₁₈H₁₄FN : M+ 263.1

¹H NMR (CD₂Cl₂, 400MHz) δ (ppm) 7.62-7.57 (t, 1H), 7.56-7.54 (m, 1H), 7.51-7.47 (m, 4H), 7.44-7.37 (m, 3H), 7.31-7.30 (m, 1H), 7.29-7.26 (m, 1H), 7.23-7.22 (m, 1H), 3.94 (s, 2H)

### Synthesis of Intermediate I-30

2.44 g of Intermediate I-30 was synthesized from Intermediate I-29 in the same manner as in the synthesis of Intermediate I-8 (Yield: 72 %). This compound was identified using LC-MS and NMR. C₂₄H₁₈FN : M+ 339.1

¹H NMR (CDCl₃, 400MHz) δ (ppm) 7.79-7.78 (m, 1H), 7.63-7.61 (m, 2H), 7.57-7.54 (m, 4H), 7.42-7.38 (m, 2H), 7.28-7.21 (m, 3H), 7.12-7.10 (dt, 2H), 7.03-7.02 (d, 2H), 6.89-6.85 (dt, 1H), 4.23 (s, 1H)

### Synthesis of Intermediate I-31

3.31 g of Intermediate I-31 was synthesized from Intermediate I-30 in the same manner as in the synthesis of Intermediate I-9 (Yield: 67 %). This compound was identified using LC-MS and NMR. C₃₀H₂₁BrFN : M+ 493.1

¹H NMR (CDCl₃, 400MHz) δ (ppm) 7.77-7.63 (m, 2H), 7.50-7.36 (m, 5H), 7.23-7.17 (m, 7H), 7.14-7.09 (dt, 2H), 6.92-6.84 (m, 3H), 6.77-6.72 (m, 2H)

### Synthesis of Intermediate I-32

4.28 g of Intermediate I-32 was synthesized from Intermediate I-31 in the same manner as in the synthesis of Intermediate I-10 (Yield: 79 %). This compound was identified using LC-MS and NMR. C₃₆H₃₃BFNO₂ : M+ 541.3

¹H NMR (CDCl₃, 400MHz) δ (ppm) 7.63-7.58 (m, 4H), 7.46-7.33 (m, 5H), 7.17 -7.13 (m, 5H), 7.07-7.02 (m, 2H), 6.89-6.86 (dd, 3H), 6.82-6.78 (m, 2H)

### Synthesis of Compound 75

3.26 g of Compound 75 was synthesized from Intermediate I-32 and Intermediate I-28 in the same manner as in the synthesis of Compound 3 (Yield: 76 %). This compound was identified using LC-MS and NMR. C₆₄H₄₁FN₂ : M+ 856.3

¹H NMR (CDCl₃, 400MHz) δ (ppm) 9.06-9.04 (d, 1H), 8.50-8.48 (m, 2H), 8.39-8.37 (d, 1H), 8.33-8.27 (dd, 2H), 8.19-8.10 (m, 6H), 7.92-7.85 (m, 4H), 7.71-7.70 (dd, 2H), 7.61-7.50 (m, 9H), 7.46-7.36 (m, 9H), 7.14-7.10 (dt, 1H), 7.09-7.05 (m, 2H), 6.95-6.93 (dd, 2H)

### Example 1

To manufacture an anode, a corning 15 Ω/cm² (1200 Å) ITO glass substrate was cut to a size of 50 mm x 50 mm x 0.7 mm and then sonicated in isopropyl alcohol and pure water each for five minutes, and then cleaned by irradiation of ultraviolet rays for 30 minutes and exposure to ozone. The resulting glass substrate was loaded into a vacuum deposition device.

Then, 2-TNATA, which is a HIL material, was vacuum-deposited on the glass substrate to form a HIL having a thickness of about 600 Å. Then, 4,4'-bis[N-(1-naphthyl)-N-phenylamino]biphenyl (NPB), which is a hole transporting compound, was vacuum-deposited on the HIL to form a HTL having a thickness of about 300 Å.

A blue fluorescent host 9, 10-di-naphthalene-2-yl-anthracene (ADN) and Compound 3 of Synthesis Example 1, which is a blue fluorescent dopant, were simultaneously deposited on the HTL with a weight ratio of 98:2 to form an EML having a thickness of 300 Å.

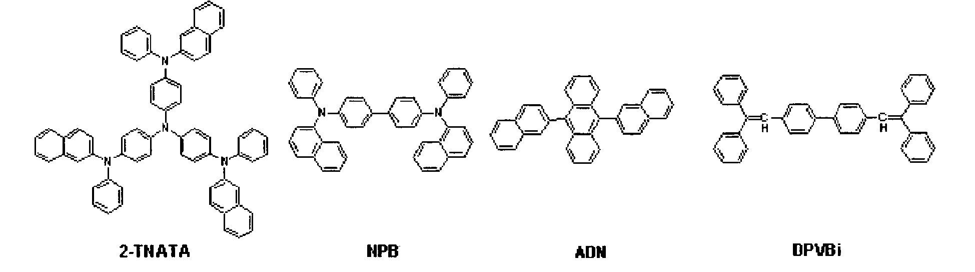

Then, Alq3 was deposited on the EML to form an ETL having a thickness of 300 Å, and then LiF, which is a halogenated alkali metal, was deposited on the ETL to form an EIL having a thickness of 10 Å. Then, Al was vacuum-deposited on the EIL to form a cathode having a thickness of 3000 Å, thereby forming an LiF/Al electrode and completing the manufacture of an organic light-emitting device.

The organic light-emitting device had a driving voltage of 6.45V at a current density of 50 mA/cm², a high luminosity of 2965 cd/m², a luminescent efficiency of 5.93 cd/A, and a half-lifespan of 275 hours at 100mA/cm².

### Example 2

An organic light-emitting device was manufactured in the same manner as in Example 1, except that Compound 5 was used, instead of Compound 3, to form the EML.

The organic light-emitting device had a driving voltage of 6.46V at a current density of 50 mA/cm², a high luminosity of 2940 cd/m², a luminescent efficiency of 5.88 cd/A, and a half-lifespan of 267 hours at 100mA/cm².

### Example 3

An organic light-emitting device was manufactured in the same manner as in Example 1, except that Compound 11 was used, instead of Compound 3, to form the EML.

The organic light-emitting device had a driving voltage of 6.32V at a current density of 50 mA/cm², a high luminosity of 2985 cd/m², a luminescent efficiency of 5.97 cd/A, and a half-lifespan of 180 hours at 100mA/cm².

### Example 4

An organic light-emitting device was manufactured in the same manner as in Example 1, except that Compound 21 was used, instead of Compound 3, to form the EML.

The organic light-emitting device had a driving voltage of 6.45V at a current density of 50 mA/cm², a high luminosity of 2890 cd/m², a luminescent efficiency of 5.78 cd/A, and a half-lifespan of 260 hours at 100mA/cm².

### Example 5

An organic light-emitting device was manufactured in the same manner as in Example 1, except that Compound 24 was used, instead of Compound 3, to form the EML.

The organic light-emitting device had a driving voltage of 6.21 V at a current density of 50 mA/cm², a high luminosity of 2560 cd/m², a luminescent efficiency of 5.12 cd/A, and a half-lifespan of 193 hours at 100mA/cm².

### Example 6

An organic light-emitting device was manufactured in the same manner as in Example 1, except that Compound 40 was used, instead of Compound 3, to form the EML.

The organic light-emitting device had a driving voltage of 6.41 V at a current density of 50 mA/cm², a high luminosity of 2835 cd/m², a luminescent efficiency of 5.67 cd/A, and a half-lifespan of 232 hours at 100mA/cm².

### Example 7

An organic light-emitting device was manufactured in the same manner as in Example 1, except that Compound 58 was used, instead of Compound 3, to form the EML.

The organic light-emitting device had a driving voltage of 6.46V at a current density of 50 mA/cm², a high luminosity of 2745 cd/m², a luminescent efficiency of 5.49 cd/A, and a half-lifespan of 217 hours at 100mA/cm².

### Example 8

An organic light-emitting device was manufactured in the same manner as in Example 1, except that Compound 63 was used, instead of Compound 3, to form the EML.

The organic light-emitting device had a driving voltage of 6.42V at a current density of 50 mA/cm², a high luminosity of 2855 cd/m², a luminescent efficiency of 5.71 cd/A, and a half-lifespan of 225 hours at 100mA/cm².

### Example 9

An organic light-emitting device was manufactured in the same manner as in Example 1, except that Compound 75 was used, instead of Compound 3, to form the EML.

The organic light-emitting device had a driving voltage of 6.47V at a current density of 50 mA/cm², a high luminosity of 2945 cd/m², a luminescent efficiency of 5.89 cd/A, and a half-lifespan of 209 hours at 100mA/cm².

### Comparative Example 1

An organic light-emitting device was manufactured in the same manner as in Example 1, except that a blue fluorescent dopant 1,4-bis-(2,2-diphenylvinyl)biphenyl (DPVBi) was used, instead of Compound 3, to form the EML.

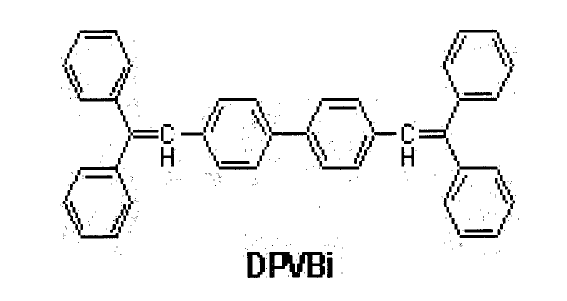

The organic light-emitting device had a driving voltage of 7.85V at a current density of 50 mA/cm², a luminosity of 1560 cd/m², a luminescent efficiency of 3.12 cd/A, and a half-lifespan of 113 hours at 100mA/cm².

The organic light-emitting devices manufactured using the heterocyclic compounds represented by Formula 1 according to embodiments as blue dopants for EML had improved driving voltages and much higher I-V-L characteristics, as compared to those manufactured using DPVBi. In particular, the organic light-emitting devices according to the embodiments had markedly improved lifetimes. These results are shown in Table 1 below.

**Table 1**

| | EML material | Driving voltage (V) | Current density (mA/cm²) | Luminosity (cd/m²) | Luminescent efficiency (cd/A) | Emitting light color | Half life-span (hr@100mA/ cm²) |
|---|---|---|---|---|---|---|---|
| Example 1 | Compound 3 | 6.45 | 50 | 2,965 | 5.93 | blue | 275 hr |
| Example 2 | Compound 5 | 6.46 | 50 | 2,940 | 5.88 | blue | 267 hr |
| Example 3 | Compound 11 | 6.32 | 50 | 2,985 | 5.97 | blue | 180 hr |
| Example 4 | Compound 21 | 6.45 | 50 | 2,890 | 5.78 | blue | 260 hr |
| Example 5 | Compound 24 | 6.21 | 50 | 2,560 | 5.12 | bluish green | 193 hr |
| Example 6 | Compound 40 | 6.41 | 50 | 2,835 | 5.67 | blue | 232 hr |
| Example 7 | Compound 58 | 6.46 | 50 | 2,745 | 5.49 | blue | 217 hr |
| Example 8 | Compound 63 | 6.42 | 50 | 2,855 | 5.71 | blue | 225 hr |
| Example 9 | Compound 75 | 6.47 | 50 | 2,945 | 5.89 | blue | 209 hr |
| Comparative Example 1 | DPVBi | 7.85 | 50 | 1,560 | 3.12 | blue | 113 hr |

As described above, novel heterocyclic compounds according to the one or more of the above embodiments of the present invention have good electrical characteristics, good charge transporting capabilities and good emission characteristics, and may be used to prevent crystallization due to high glass transition temperatures (T_{g}). The heterocyclic compounds may also be used as electron transporting materials for most color-fluorescent and phosphorescent devices, such as red, green, blue, and white fluorescent and phosphorescent devices, or as red, green, blue or white-light emitting materials. Thus, an organic light-emitting device with high-efficiency, low-driving voltage, high luminance and long lifespan may be manufactured using the heterocylic compounds.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A heterocyclic compound represented by Formula 1 below: 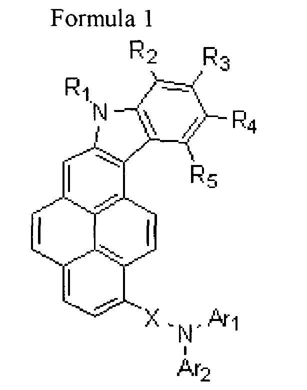 wherein, in Formula 1,
R₁ through R₅ are each independently a hydrogen atom, a deuterium atom, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₃-C₆₀ cycloalkyl group, a substituted or unsubstituted C₁-C₆₀ alkoxy group, a substituted or unsubstituted C₅-C₆₀ aryloxy group, a substituted or unsubstituted C₅-C₆₀ arylthio group, a substituted or unsubstituted C₅-C₆₀ aryl group, an amino group substituted with a C₅-C₆₀ aryl or C₃- C₆₀ heteroaryl group, a substituted or unsubstituted C₃-C₆₀ heteroaryl group, a substituted or unsubstituted C₆-C₆₀ condensed polycyclic group, a halogen atom, a cyano group, a nitro group, a hydroxyl group, or a carboxyl group;
Ar₁ and Ar₂ are each independently a substituted or unsubstituted C₁-C₆₀ alkyl group, a substituted or unsubstituted C₃-C₅₀ cycloalkyl group, a substituted or unsubstituted C₅-C₆₀ aryloxy group, a substituted or unsubstituted C₅-C₆₀ arylthio group, a substituted or unsubstituted C₅-C₆₀ aryl group, a amino group substituted with a C₅-C₆₀ aryl or C₃-C₆₀ heteroaryl group, a substituted or unsubstituted C₃-C₆₀ heteroaryl group, or a substituted or unsubstituted C₆-C₆₀ condensed polycyclic group; and
X is a divalent linking group represented by -(Ar₃)ₙ where Ar₃ is a substituted or unsubstituted C₅-C₆₀ arylene group, a substituted or unsubstituted C₃-C₆₀ heteroarylene group, or a substituted or unsubstituted C₆-C₆₀ condensed polycyclic group, and *n* is an integer from 1 to 10, wherein *n* groups of Ar₃ are identical to or different from each other, and at least two adjacent groups of the *n* Ar₃ groups are fused or linked to each other by a single bond.

2. The heterocyclic compound of claim 1, wherein R₁ to R₅ in Formula 1 are each independently a hydrogen atom, a deuterium atom, a cyano group, a halogen atom, substituted or unsubstituted C₁-C₂₀ alkyl group, a substituted C₁-C₂₀ alkyl group with at least one fluorine (-F) substituent, a substituted or unsubstituted C₅-C₂₀ aryl group, or a substituted or unsubstituted C₃-C₂₀ heteroaryl group.

3. The heterocyclic compound of claim 1 or 2, wherein R₁ to R₅ in Formula 1 are each independently a hydrogen atom, a deuterium atom, a halogen atom, a cyano group, a substituted or unsubstituted C₁-C₂₀alkyl group, or a group represented by Formulae 2a to 2f below: 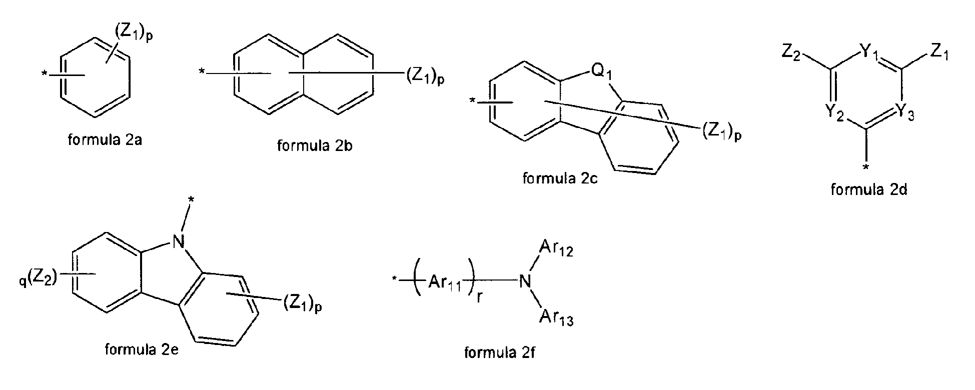 wherein, in Formulae 2a to 2f,
Q₁ is a linking group represented by -C(R₆)(R₇)-, -N(R₆)-, -S-, or -O-;
Y₁, Y₂, and Y₃ are each independently a linking group represented by -N= or -C(R₈)=;
Z₁, Z₂, Ar₁₂, Ar₁₃, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
Ar₁₁ is a substituted or unsubstituted C₁-C₂₀ alkylene group, a substituted or unsubstituted C₅-C₂₀ arylene group, or a substituted or unsubstituted C₃-C₂₀ heteroarylene group;
p is an integer from 1 to 12;
q is an integer from 1 to 12;
r is an integer from 0 to 5; and
* indicates a binding site.

4. The heterocyclic compound of one of the preceding claims, wherein R, to R₅ in Formula 1 are each independently a hydrogen atom, a deuterium atom, a halogen atom, a cyano group, a methyl group, an ethyl group, a propyl group, a butyl group, a pentyl group, or groups represented by Formulae 3a to 3h below: 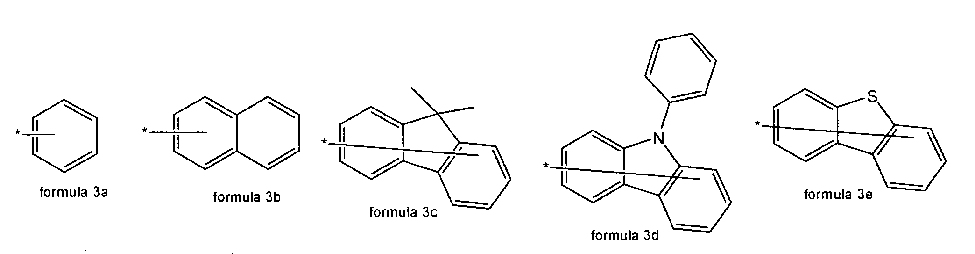 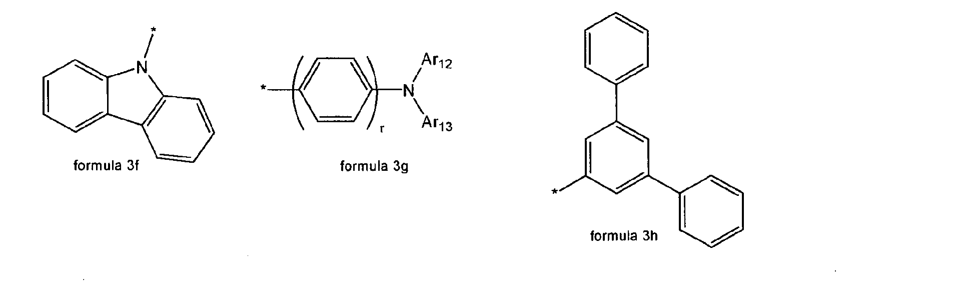 wherein in Formula 3a to 3h,
Ar₁₂ and Ar₁₃ are each independently an unsubstituted aryl group, a substituted or unsubstituted C₃-C₂₀ heteroaryl group, or a substituted or unsubstituted C₆-C₂₀ condensed polycyclic group,
r is an integer from 0 to 2; and
* indicates a binding site.

5. The heterocyclic compound of one of the preceding claims, wherein Ar₁ and Ar₂ in Formula 1 are each independently a substituted or unsubstituted C₁-C₂₀ alkyl group, a substituted or unsubstituted C₅-C₂₀ aryl group, or a substituted or unsubstituted C₃-C₂₀ heteroaryl group.

6. The heterocyclic compound of one of the preceding claims, wherein Ar₁ and Ar₂ in Formula 1 are each independently a group represented by one of Formulae 4a to 4d below: 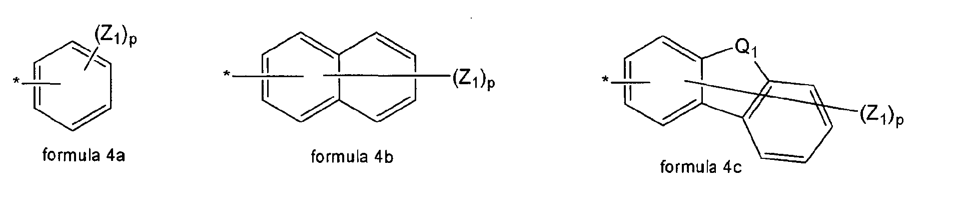 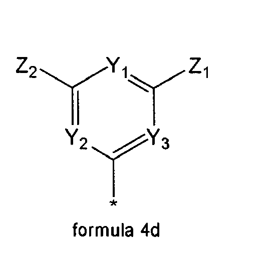 wherein, in Formula 4a to 4d,
Q₁ is a linking group represented by -C(R₆)(R₇)-, -N(R₆)-, -S-, or -O-;
Y₁, Y₂, and Y₃ are each independently a linking group represented by -N= or -C(R₈)=;
Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
p is an integer from 1 to 8; and
*indicates a binding site.

7. The heterocyclic compound of one of the preceding claims, wherein Ar₁ and Ar₂ in Formula 1 are each independently a group represented by one of Formulae 5a to 5i below: 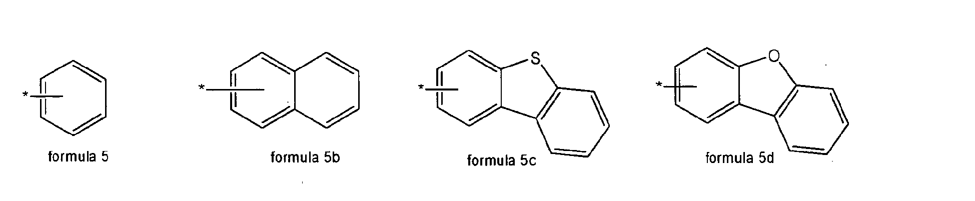 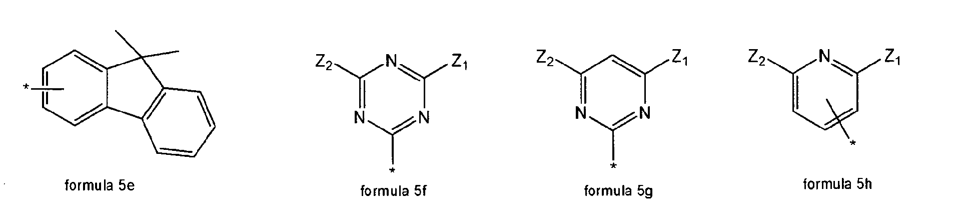 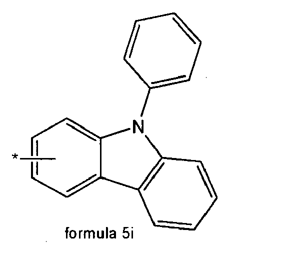 wherein Z₁ and Z₂ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, or a substituted or unsubstituted C₆-C₂₀ condensed polycyclic group; and * indicates a binding site.

8. The heterocyclic compound of one of the preceding claims, wherein Ar₃ for X in Formula 1 is a substituted or unsubstituted C₅-C₂₀ arylene group, a substituted or unsubstituted C₃-C₂₀ heteroarylene group, or a substituted or unsubstituted C₆-C₂₀ condensed polycyclic group.

9. The heterocyclic compound of one of the preceding claims, wherein Ar₃ for X in Formula 1 comprises a group represented by one of Formulae 6a to 6e below: 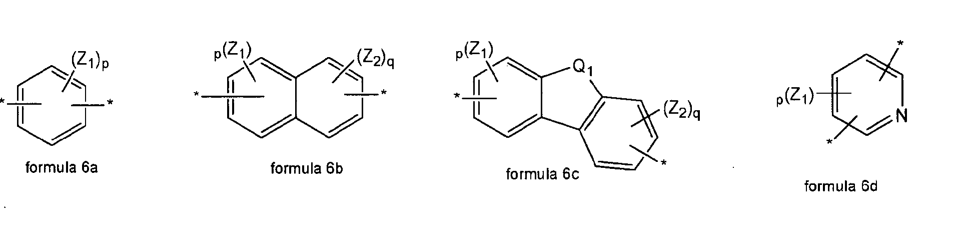 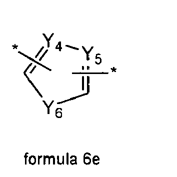 wherein, in Formula 6a to 6e,
Q₁ is a linking group represented by -C(R₆)(R₇)-, -N(R₆)-, or -S-;
Y₄, Y₅, and Y₆ are each independently a linking group represented by -N= or -C(R₈)=, -S-, or -O-;
Z₁, Z₂, R₆, R₇, and R₈ are each independently a hydrogen atom, a deuterium atom, a substituted or unsubstituted C₁-C₂₀ alkyl group, a substituted or unsubstituted C₅-C₂₀ aryl group, a substituted or unsubstituted C₃-C₂₀ heteroaryl group, a substituted or unsubstituted C₆-C₂₀ condensed polycyclic group, a halogen atom, a cyano group, a nitro group, a hydroxyl group, or a carboxy group;
p is an integer from 1 to 8;
q is an integer from 1 to 8; and
* indicates a binding site.

10. The heterocyclic compound of one of the preceding claims, wherein n is 1 or 2.

11. The heterocyclic compound of one of the preceding claims, wherein X in Formula 1 comprises a group represented by one of Formulae 7a to 7j: 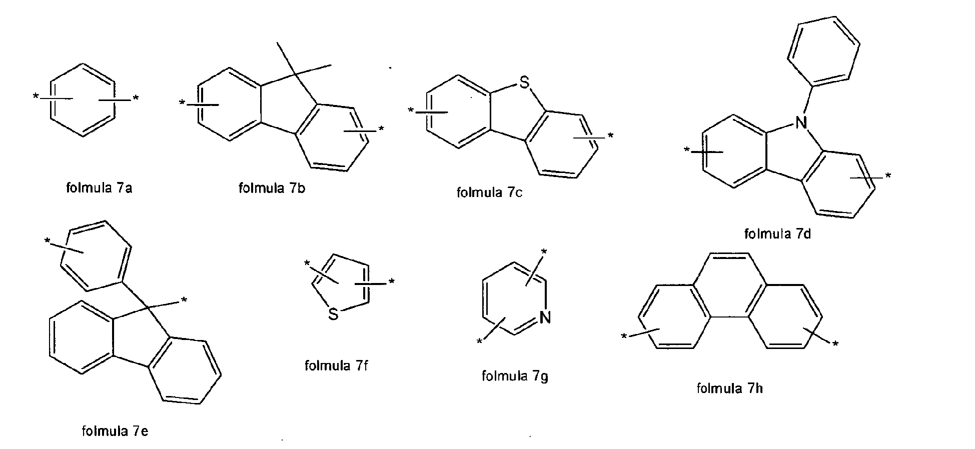 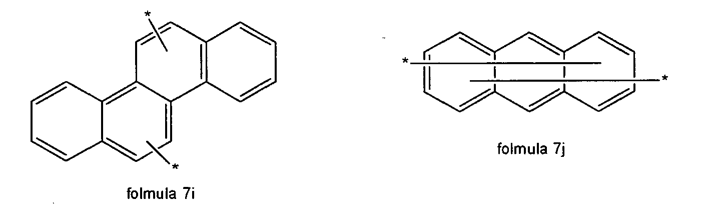 wherein, in Formula 7a to 7j, * indicates a binding site.

12. The heterocyclic compound of one of the preceding claims, represented by one of the compounds below:

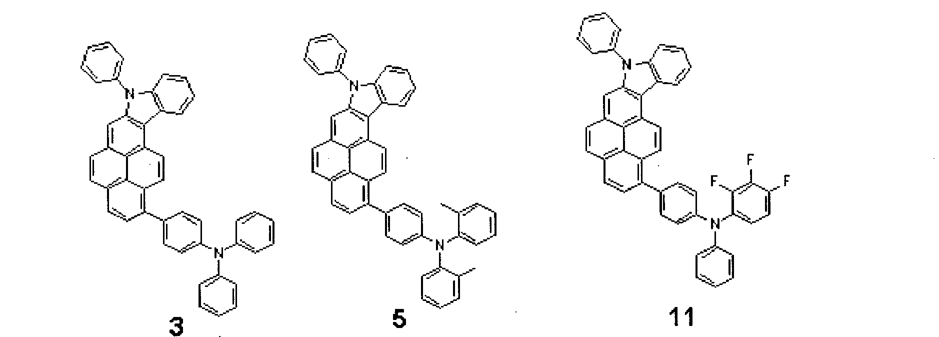

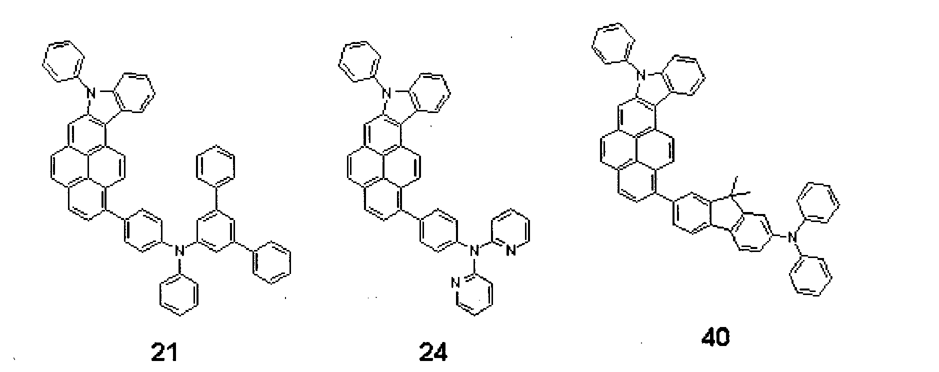

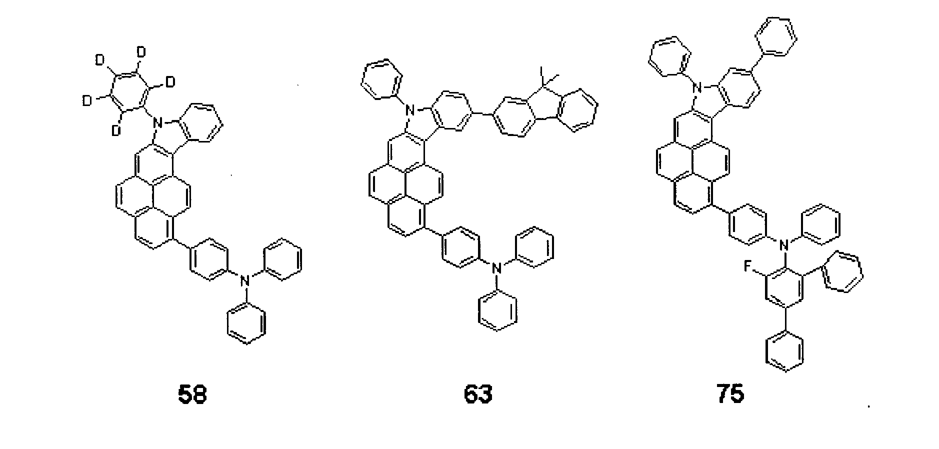

13. An organic light-emitting device comprising:
a first electrode;
a second electrode; and
an organic layer between the first electrode and the second electrode,
wherein the organic layer comprises a first layer including the heterocyclic compound represented by Formula 1 in any one of claims 1-12.

14. The organic light-emitting device of claim 13, wherein the first layer comprises at least one of a hole injection layer, a hole transport layer, a functional layer having both hole injection and hole transport capabilities, an electron injection layer, an electron transport layer, a functional layer having both electron injection and electron transport capabilities or any combination thereof.

15. The organic light-emitting device of claim 13 or 14, wherein the first layer comprises an emission layer, and a heterocyclic compound of Formula 1 is used in the emission layer as a host or a dopant for a fluorescent or phosphorescent device.

16. The organic light-emitting device of any of claims 13 to 15, wherein the first layer comprises a blue emission layer and a heterocyclic compound of Formula 1 as a blue dopant.

17. A flat panel display device comprising the organic light-emitting device of any of claims 13 to 16, wherein the first electrode of the organic light-emitting device is electrically connected to a source electrode or a drain electrode of a thin-film transistor.

## Patentansprüche

1. Heterocyclische Verbindung, die durch die Formel 1
unten dargestellt ist 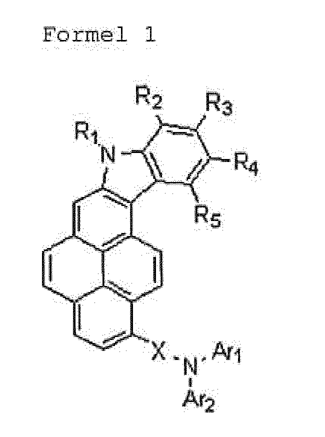 wobei in Formel 1
R₁ bis R₅ jeweils unabhängig ein Wasserstoffatom, ein Deuteriumatom, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₃-C₆₀-Cycloalkylgruppe, eine substituierte oder unsubstituierte C₁-C₆₀-Alkoxygruppe, eine substituierte oder unsubstituierte C₅-C₆₀-Aryloxygruppe, eine substituierte oder unsubstituierte C₅-C₆₀-Arylthiogruppe, eine substituierte oder unsubstituierte C₅-C₆₀-Arylgruppe, eine Aminogruppe, die mit einer C₅-C₆₀-Aryl- oder C₃-C₆₀-Heteroarylgruppe substituiert ist, eine substituierte oder unsubstituierte C₃-C₆₀-Heteroarylgruppe, eine substituierte oder unsubstituierte kondensierte polycyclische C₆-C₆₀-Gruppe, ein Halogenatom, eine Cyanogruppe, eine Nitrogruppe, eine Hydroxylgruppe oder eine Carboxylgruppe sind;
Ar₁ und Ar₂ jeweils unabhängig eine substituierte oder unsubstituierte C₁-C₆₀-Alkylgruppe, eine substituierte oder unsubstituierte C₃-C₅₀-Cycloalkylgruppe, eine substituierte oder unsubstituierte C₅-C₆₀-Aryloxygruppe, eine substituierte oder unsubstituierte C₅-C₆₀-Arylthiogruppe, eine substituierte oder unsubstituierte C₅-C₆₀-Arylgruppe, eine Aminogruppe, die mit einer C₅-C₆₀-Aryl- oder C₃-C₆₀-Hereroarylgruppe substituiert ist, eine substituierte oder unsubstituierte C₃-C₆₀-Heteroarylgruppe oder eine substituierte oder unsubstituierte kondensierte polycyclische C₆-C₆₀-Gruppe sind; und
X eine divalente Verknüpfungsgruppe ist, die durch - (Ar₃)ₙ- dargestellt ist, wobei Ar₃ eine substituierte oder unsubstituierte C₅-C₆₀-Arylengruppe, eine substituierte oder unsubstituierte C₃-C₆₀-Heteroarylengruppe oder eine substituierte oder unsubstituierte kondensierte polycyclische C₆-C₆₀-Gruppe ist und n eine ganze Zahl von 1 bis 10 ist, wobei n Gruppen von Ar₃ identisch oder verschieden voneinander sind und mindestens zwei benachbarte Gruppen der n Ar₃-Gruppen anelliert oder durch eine Einzelbindung miteinander verknüpft sind.

2. Heterocyclische Verbindung nach Anspruch 1, wobei R₁ bis R₅ in Formel 1 jeweils unabhängig ein Wasserstoffatom, ein Deuteriumatom, eine Cyanogruppe, ein Halogenatom, eine substituierte oder unsubstituierte C₁-C₂₀-Alkylgruppe eine substituierte C₁-C₂₀-Alkylgruppe mit mindestens einem Fluor- (- F-) Substituenten, eine substituierte oder unsubstituierte C₅-C₂₀-Arylgruppe oder eine substituierte oder unsubstituierte C₃-C₂₀-Heteroarylgruppe sind.

3. Heterocyclische Verbindung nach Anspruch 1 oder 2, wobei R₁ bis R₅ in Formel 1 jeweils unabhängig ein Wasserstoffatom, ein Deuteriumatom, ein Halogenatom, eine Cyanogruppe, eine substituierte oder unsubstituierte C₁-C₂₀-Alkylgruppe oder eine Gruppe sind, die durch die Formeln 2a bis 2f unten dargestellt ist: 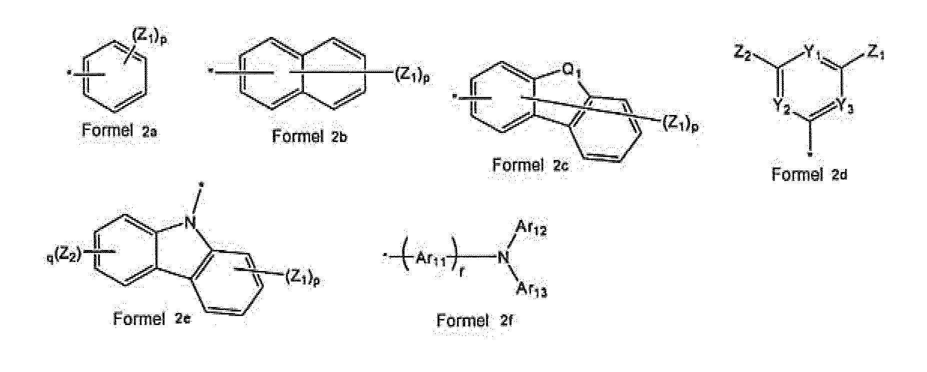 wobei in den Formeln 2a bis 2f
Q₁ eine Verknüpfungsgruppe ist, die durch -C(R₆) (R₇)-, -N(R₆), -S- oder -O- dargestellt ist;
Y₁, Y₂ und Y₃ jeweils unabhängig eine Verknüpfungsgruppe sind, die durch -N= oder -C(R₈)= dargestellt ist;
Z₁, Z₂, Ar₁₂, Ar₁₃, R₆, R₇ und R₈ jeweils unabhängig ein Wasserstoffatom, ein Deuteriumatom, eine substituierte oder unsubstituierte C₁-C₂₀-Alkylgruppe, eine substituierte oder unsubstituierte C₅-C₂₀-Arylgruppe, eine substituierte oder unsubstituierte C₃-C₂₀-Heteroarylgruppe, eine substituierte oder unsubstituierte kondensierte polycyclische C₆-C₂₀-Gruppe, ein Halogenatom, eine Cyanogruppe, eine Nitrogruppe, eine Hydroxylgruppe oder eine Carboxylgruppe sind;
Ar₁₁ eine substituierte oder unsubstituierte C₁-C₂₀-Alkylengruppe, eine substituierte oder unsubstituierte C₅-C₂₀-Arylengruppe oder eine substituierte oder unsubstituierte C₃-C₂₀-Heteroarylengruppe ist;
p eine ganze Zahl von 1 bis 12 ist;
q eine ganze Zahl von 1 bis 12 ist;
r eine ganze Zahl von 0 bis 5 ist; und
* eine Bindungsstelle anzeigt.

4. Heterocyclische Verbindung nach einem der vorhergehenden Ansprüche, wobei R₁ bis R₅ in Formel 1 jeweils unabhängig ein Wasserstoffatom, ein Deuteriumatom, ein Halogenatom, eine Cyanogruppe, eine Methylgruppe, eine Ethylgruppe, eine Propylgruppe eine Butylgruppe, eine Pentylgruppe oder Gruppen sind, die durch die Formeln 3a bis 3h unten dargestellt sind: 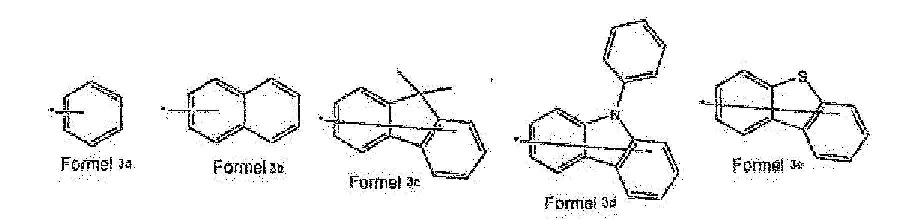 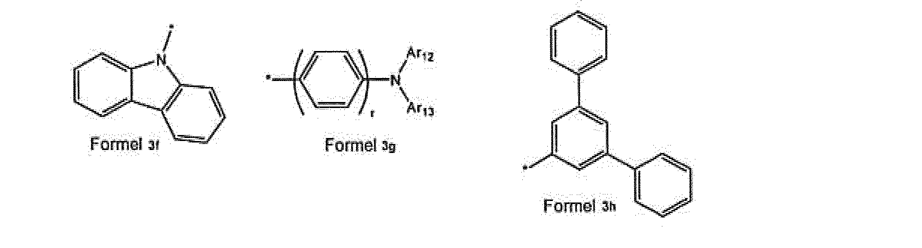 wobei in den Formeln 3a bis 3h
Ar₁₂ und Ar₁₃ jeweils unabhängig eine unsubstituierte Arylgruppe, eine substituierte oder unsubstituierte C₃-C₂₀-Heteroarylgruppe oder eine substituierte oder unsubstituierte kondensierte polycyclische C₆-C₂₀-Gruppe sind;
r eine ganze Zahl von 0 bis 2 ist; und
* eine Bindungsstelle anzeigt.

5. Heterocyclische Verbindung nach einem der vorhergehenden Ansprüche, wobei Ar₁ und Ar₂ in Formel 1 jeweils unabhängig eine substituierte oder unsubstituierte C₁-C₂₀-Alkylgruppe, eine substituierte oder unsubstituierte C₅-C₂₀-Arylgruppe oder eine substituierte oder unsubstituierte C₃-C₂₀-Heteroarylgruppe sind.

6. Heterocyclische Verbindung nach einem der vorhergehenden Ansprüche, wobei Ar₁ und Ar₂ in Formel 1 jeweils unabhängig eine Gruppe sind, die durch eine der Formeln 4a bis 4d unten dargestellt ist: 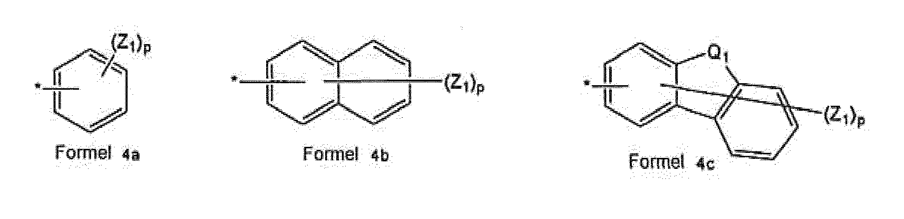 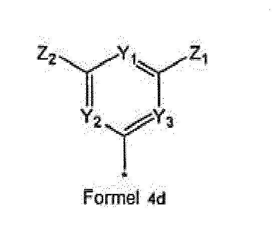 wobei in den Formeln 4a bis 4d
Q₁ eine Verknüpfungsgruppe ist, die durch -C(R₆) (R₇)-, -N(R₆)-, -S- oder -O- dargestellt ist;
Y₁, Y₂ und Y₃ jeweils unabhängig eine Verknüpfungsgruppe sind, die durch -N= oder -C(R₈) = dargestellt ist;
Z₁, Z₂, R₆, R₇ und R₈ jeweils unabhängig ein Wasserstoffatom, ein Deuteriumatom, eine substituierte oder unsubstituierte C₁-C₂₀-Alkylgruppe, eine substituierte oder unsubstituierte C₅-C₂₀-Arylgruppe, eine substituierte oder unsubstituierte C₃-C₂₀-Heteroarylgruppe, eine substituierte oder unsubstituierte kondensierte polycyclische C₆-C₂₀-Gruppe, ein Halogenatom, eine Cyanogruppe, eine Nitrogruppe, eine Hydroxylgruppe oder eine Carboxylgruppe sind;
p eine ganze Zahl von 1 bis 8 ist; und
* eine Bindungsstelle anzeigt.

7. Heterocyclische Verbindung nach einem der vorhergehenden Ansprüche, wobei Ar₁ und Ar₂ in Formel 1 jeweils unabhängig eine Gruppe sind, die durch die Formeln 5a bis 5i unten dargestellt ist: 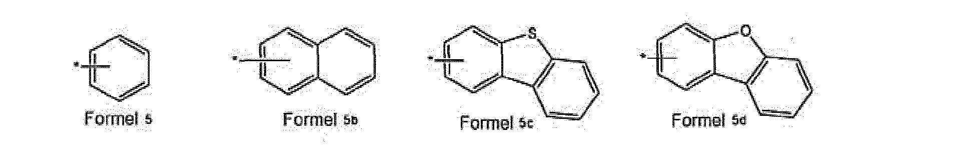 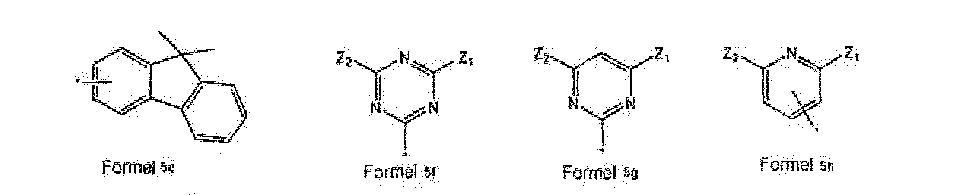 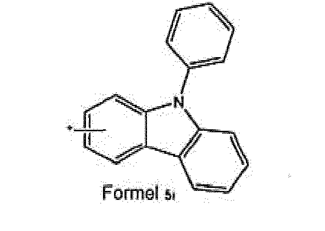 wobei Z₁ und Z₂ jeweils unabhängig ein Wasserstoffatom, ein Deuteriumatom, eine substituierte oder unsubstituierte C₁-C₂₀-Alkylgruppe, eine substituierte oder unsubstituierte C₅-C₂₀-Arylgruppe, eine substituierte oder unsubstituierte C₃-C₂₀-Heteroarylgruppe oder eine substituierte oder unsubstituierte kondensierte polycyclische C₆-C₂₀-Gruppe sind; und * eine Bindungsstelle anzeigt.

8. Heterocyclische Verbindung nach einem der vorhergehenden Ansprüche, wobei Ar₃ für X in Formel 1 eine substituierte oder unsubstituierte C₅-C₂₀-Arylengruppe, eine substituierte oder unsubstituierte C₃-C₂₀-Heteroarylengruppe oder eine substituierte oder unsubstituierte kondensierte polycyclische C₆-C₂₀-Gruppe ist.

9. Heterocyclische Verbindung nach einem der vorhergehenden Ansprüche, wobei Ar₃ für X in Formel 1 eine Gruppe umfasst, die durch die Formeln 6a bis 6e unten dargestellt ist: 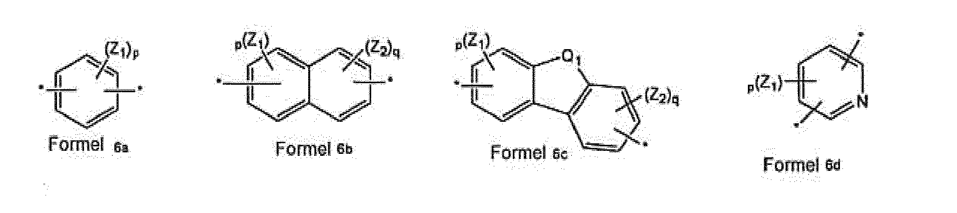 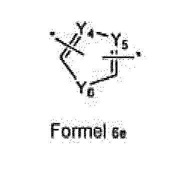 wobei in den Formeln 6a bis 6e
Q₁ eine Verknüpfungsgruppe ist, die durch -C(R₆) (R₇)-. -N(R₆)- oder -S-dargestellt ist;
Y₄, Y₅ und Y₆ jeweils unabhängig eine Verknüpfungsgruppe sind, die durch -N= oder -C(R₈)=, - S- oder -O- dargestellt ist;
Z₁, Z₂, R₆, R₇ und R₈ jeweils unabhängig ein Wasserstoffatom, ein Deuteriumatom, eine substituierte oder unsubstituierte C₁-C₂₀-Alkylgruppe, eine substituierte oder unsubstituierte C₅-C₂₀-Arylgruppe, eine substituierte oder unsubstituierte C₃-C₂₀-Heteroarylgruppe, eine substituierte oder unsubstituierte kondensierte polycyclische C₆-C₂₀-Gruppe, ein Halogenatom, eine Cyanogruppe, eine Nitrogruppe, eine Hydroxylgruppe oder eine Carboxylgruppe sind;
p eine ganze Zahl von 1 bis 8 ist;
q eine ganze Zahl von 1 bis 8 ist; und
* einen Bindungsort anzeigt.

10. Heterocyclische Verbindung nach einem der vorhergehenden Ansprüche, wobei n 1 oder 2 beträgt.

11. Heterocyclische Verbindung nach einem der vorhergehenden Ansprüche, wobei X in Formel 1 eine Gruppe umfasst, die durch eine der Formeln 7a bis 7j dargestellt ist: 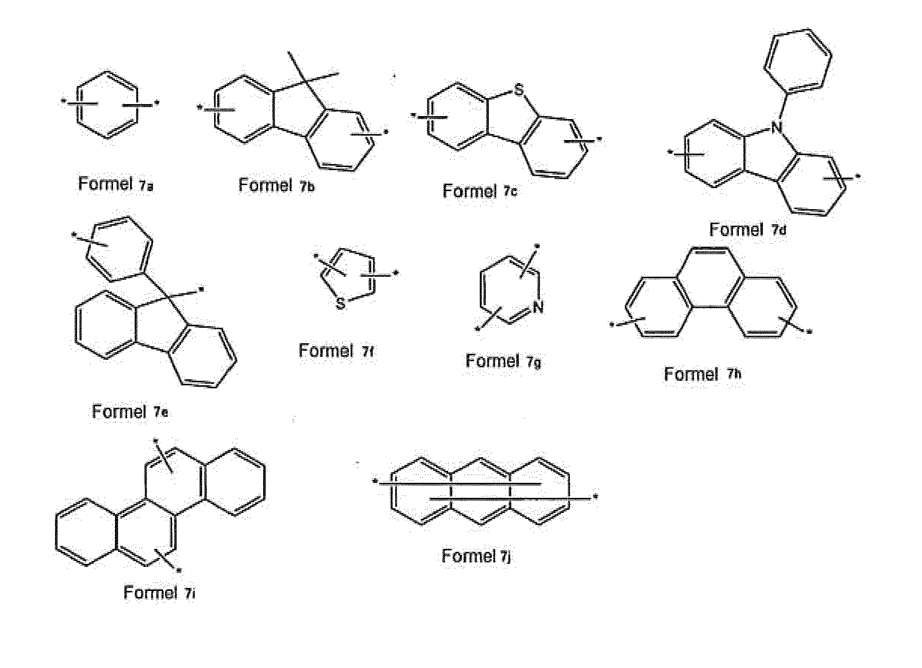 wobei in Formel 7a bis 7j * eine Bindungsstelle anzeigt.

12. Heterocyclische Verbindung nach einem der vorhergehenden Ansprüche, dargestellt durch eine der Verbindungen unten:

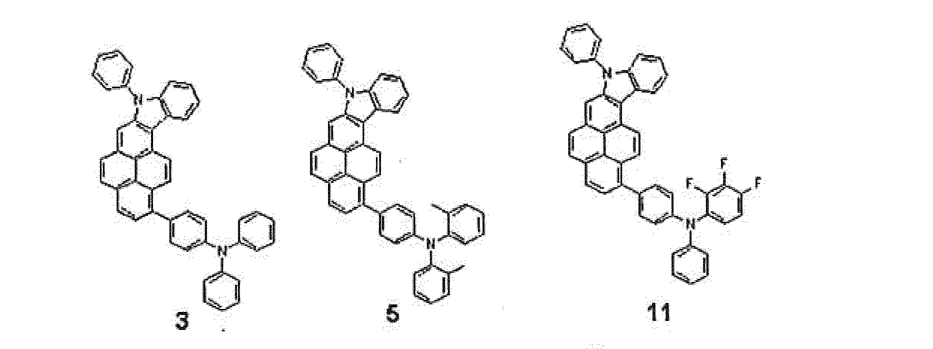

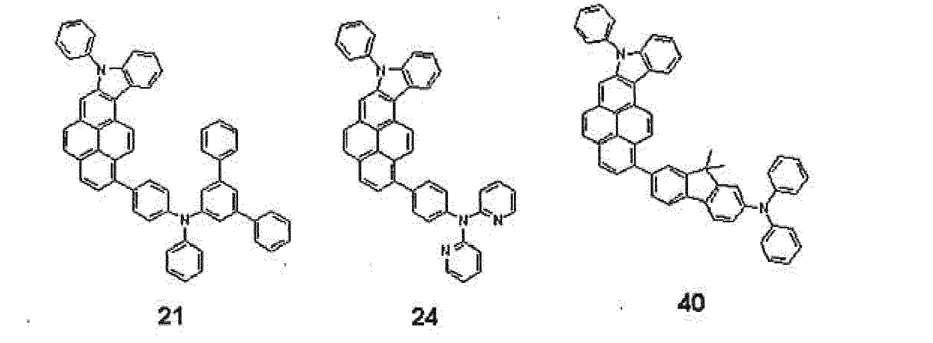

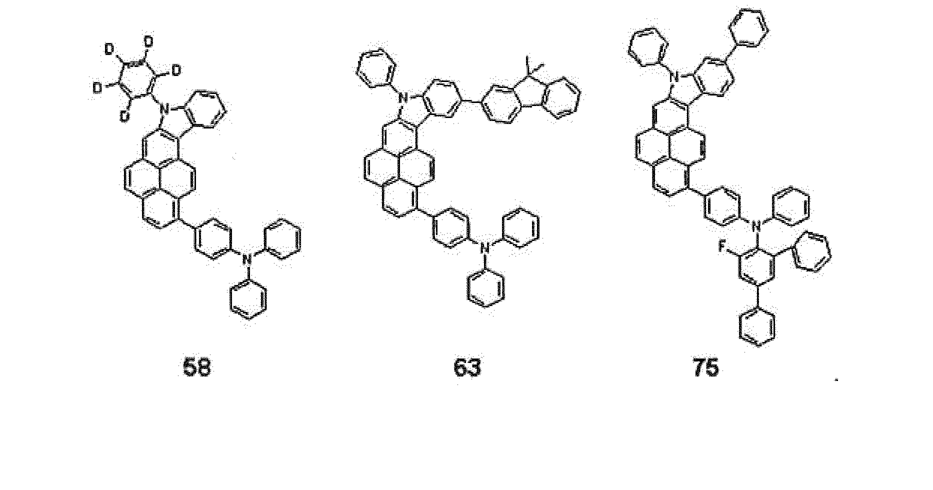

13. Organische lichtemittierende Vorrichtung umfassend:
eine erste Elektrode;
eine zweite Elektrode; und
eine organische Schicht zwischen der ersten Elektrode und der zweiten Elektrode,
wobei die organische Schicht eine erste Schicht umfasst, die die heterocyclische Verbindung umfasst, die durch die Formel 1 in einem der Ansprüche 1-12 dargestellt ist.

14. organische lichtemittierende Vorrichtung nach Anspruch 13, wobei die erste Schicht mindestens eine Lochinjektionsschicht, eine Lochtransportschicht, eine funktionelle Schicht, die sowohl Lichtinjektions- als auch Lochtransportfähigkeiten aufweist, eine Elektroneninjektionsschicht, eine Elektronentransportschicht, eine funktionelle Schicht, die sowohl Elektroneninjektions- als auch Elektronentransportfähigkeiten aufweist, oder irgendeine Kombination davon umfasst.

15. Organische lichtemittierende Vorrichtung nach Anspruch 13 oder 14, wobei die erste Schicht eine Emissionsschicht umfasst, und eine heterocyclische Verbindung der Formel 1 in der Emissionsschicht als Wirt oder Dotiermittel für eine fluoreszierende oder phosphoreszierende Vorrichtung verwendet wird.

16. Organische lichtemittierende Vorrichtung nach einem der Ansprüche 13 bis 15, wobei die erste Schicht eine blaue Emissionsschicht und eine heterocyclische Verbindung der Formel 1 als blaues Dotiermittel umfasst.

17. Flachbildschirmvorrichtung umfassend die organische lichtemittierende Vorrichtung nach einem der Ansprüche 13 bis 16, wobei die erste Elektrode der organischen lichtemittierenden Vorrichtung elektrisch mit einer Quellenelektrode oder einer Drainelektrode eines Dünnfilmtransistors verbunden ist.

## Revendications

1. Composé hétérocyclique représenté par la formule 1 ci-dessous : 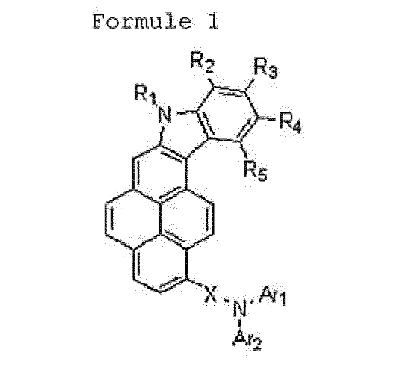 dans lequel dans la formule 1
R₁ à R₅ représentent chacun indépendamment un atome d'hydrogène, un atome de deutérium, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe cycloalkyle en C₃ à C₆₀ substitué ou non substitué, un groupe alkoxy en C₁ à C₆₀ substitué ou non substitué, un groupe aryloxy en C₅ à C₆₀ substitué ou non substitué, un groupe arylthio en C₅ à C₆₀ substitué ou non substitué, un groupe aryle en C₅ à C₆₀ substitué ou non substitué, un groupe amino substitué par un groupe aryle en C₅ à C₆₀ ou un groupe hétéroaryle en C₃ à C₆₀, un groupe hétéroaryle en C₃ à C₆₀ substitué ou non substitué, un groupe polycyclique condensé en C₆ à C₆₀ substitué ou non substitué, un atome d'halogène, un groupe cyano, un groupe nitro, un groupe hydroxyle ou un groupe carboxyle ;
Ar₁ et Ar₂ représentent chacun indépendamment un groupe alkyle en C₁ à C₆₀ substitué ou non substitué, un groupe cycloalkyle en C₃ à C₅₀ substitué ou non substitué, un groupe aryloxy en C₅ à C₆₀ substitué ou non substitué, un groupe arylthio en C₅ à C₆₀ substitué ou non substitué, un groupe aryle en C₅ à C₆₀ substitué ou non substitué, un groupe amino substitué par un groupe aryle en C₅ à C₆₀ ou un groupe hétéroaryle en C₃ à C₆₀, un groupe hétéroaryle en C₃ à C₆₀ substitué ou non substitué ou un groupe polycyclique condensé en C₆ à C₆₀ substitué ou non substitué ; et
X représente un groupe de liaison divalent représenté par -(Ar₃)ₙ- où Ar₃ représente un groupe arylène en C₅ à C₆₀ substitué ou non substitué, un groupe hétéroarylène en C₃ à C₆₀ substitué ou non substitué ou un groupe polycyclique condensé en C₆ à C₆₀ substitué ou non substitué, et n représente un nombre entier de 1 à 10, où n groupes de Ar₃ sont identiques ou différents les uns des autres, et au moins deux groupes adjacents des n groupes Ar₃ sont condensés ou liés les uns aux autres par une liaison simple.

2. Composé hétérocyclique selon la revendication 1, dans lequel R₁ à R₅ dans la formule 1 représentent chacun indépendamment un atome d'hydrogène, un atome de deutérium, un groupe cyano, un atome d'halogène, un groupe alkyle en C₁ à C₂₀ substitué ou non substitué, un groupe alkyle en C₁ à C₂₀ substitué par au moins un substituant fluor (-F), un groupe aryle en C₅ à C₂₀ substitué ou non substitué ou un groupe hétéroaryle en C₃ à C₂₀ substitué ou non substitué.

3. Composé hétérocyclique selon la revendication 1 ou 2, dans lequel R₁ à R₅ dans la formule 1 représentent chacun indépendamment un atome d'hydragéne, un atome de deutérium, un atome d'halogène, un groupe cyano, un groupe alkyle en C₁ à C₂₀ substitué ou non substitué ou un groupe représenté par les formules 2a à 2f ci-dessous : 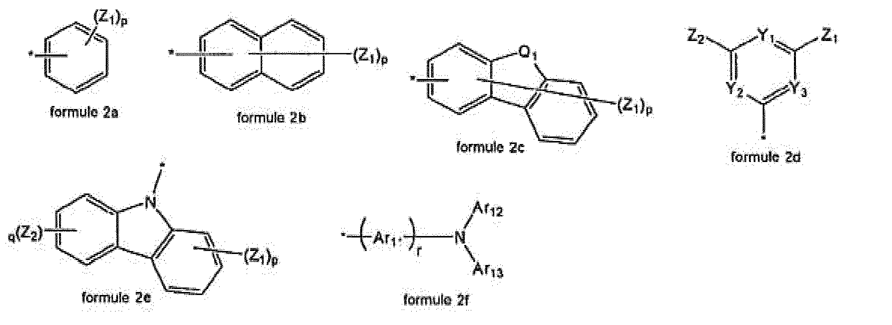 dans lequel dans les formules 2a à 2f Q₁ représente un groupe de liaison représenté par -C(R₆) (R₇)-, -N(R₆)-, -S- ou -O- ;
Y₁, Y₂ et Y₃ représentent chacun indépendamment un groupe de liaison représenté par -N= ou -C(R₈)= ;
Z₁, Z₂, Ar₁₂, Ar₁₃, R₆, R₇ et R₈ représentent chacun indépendamment un atome d'hydrogène, un atome de deutérium, un groupe alkyle en C₁ à C₂₀ substitué ou non substitué, un groupe aryle en C₅ à C₂₀ substitué ou non substitué, un groupe hétéroaryle en C₃ à C₂₀ substitué ou non substitué, un groupe polycyclique condensé en C₆ à C₂₀ substitué ou non substitué, un atome d'halogène, un groupe cyano, un groupe nitro, un groupe hydroxyle ou un groupe carboxy ;
Ar₁₁ représente un groupe alkylène en C₁ à C₂₀ substitué ou non substitué, un groupe arylène en C₅ à C₂₀ substitué ou non substitué ou un groupe hétéroarylène en C₃ à C₂₀ substitué ou non substitué ;
p représente un nombre entier de 1 à 12 ;
q représente un nombre entier de 1 à 12 ;
r représente un nombre entier de 0 à 5 ; et
* indique un site de liaison.

4. Composé hétérocyclique selon l'une quelconque des revendications précédentes, dans lequel R₁ à R₅ dans la formule 1 représentent chacun indépendamment un atome d'hydrogène, un atome de deutérium, un atome d'halogène, un groupe cyano, un groupe méthyle, un groupe éthyle, un groupe propyle, un groupe butyle, un groupe pentyle ou des groupes représentés par les formules 3a à 3h ci-dessous : 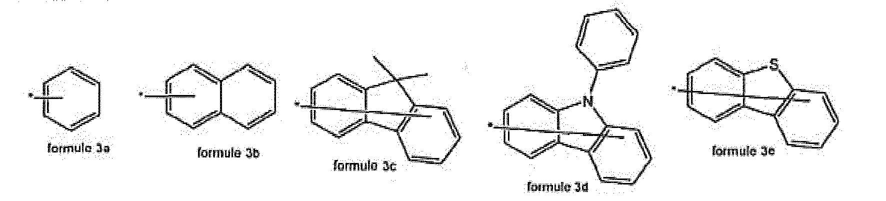 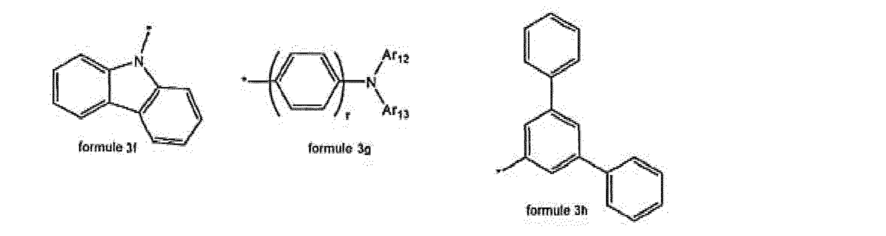 dans lequel dans les formules 3a à 3h
Ar₁₂ et Ar₁₃ représentent chacun indépendamment un groupe aryle non substitué, un groupe hétéroaryle en C₃ à C₂₀ substitué ou non substitué ou un groupe polycyclique condensé en C₆ à C₂₀ substitué ou non substitué ;
r représente un nombre entier de 0 à 2 ; et
* indique un site de liaison.

5. Composé hétérocyclique selon l'une quelconque des revendications précédentes, dans lequel Ar₁ et Ar₂ dans la formule 1 représentent chacun indépendamment un groupe alkyle en C₁ à C₂₀ substitué ou non substitué, un groupe aryle en C₅ à C₂₀ substitué ou non substitué ou un groupe hétéroaryle en C₃ à C₂₀ substitué ou non substitué.

6. Composé hétérocyclique selon l'une quelconque des revendications précédentes, dans lequel Ar₁ et Ar₂ dans la formule 1 représentent chacun indépendamment un groupe représenté par l'un des groupes de formules 4a à 4d ci-dessous : 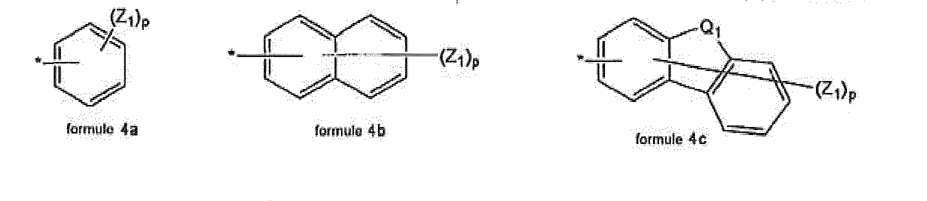 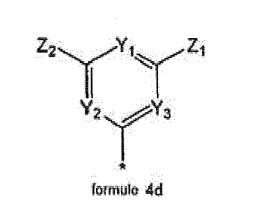 dans lequel dans les formules 4a à 4d,
Q₁ représente un groupe de liaison représenté par -C(R₆) (R₇)-, -N(R₆)-, -S- ou -O- ;
Y₁, Y₂ et Y₃ représentent chacun indépendamment un groupe de liaison représenté par -N= ou -C(R₈) = ;
Z₁, Z₂, R₆, R₇ et R₈ représentent chacun indépendamment un atome d'hydrogène, un atome de deutérium, un groupe alkyle en C₁ à C₂₀ substitué ou non substitué, un groupe aryle en C₅ à C₂₀ substitué ou non substitué, un groupe hétéroaryle en C₃ à C₂₀ substitué ou non substitué, un groupe polycyclique condensé en C₆ à C₂₀ substitué ou non substitué, un atome d'halogène, un groupe cyano, un groupe nitro, un groupe hydroxyle ou un groupe carboxy ;
p représente un nombre entier de 1 à 8 ; et
* indique un site de liaison.

7. Composé hétérocyclique selon l'une quelconque des revendications précédentes, dans lequel Ar₁ et Ar₂ dans la formule 1 représentent chacun indépendamment un groupe représenté par l'un des groupes de formules 5a à 5i ci-dessous : 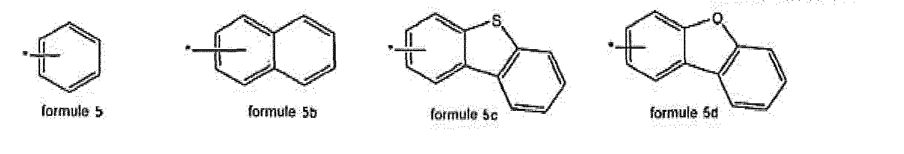 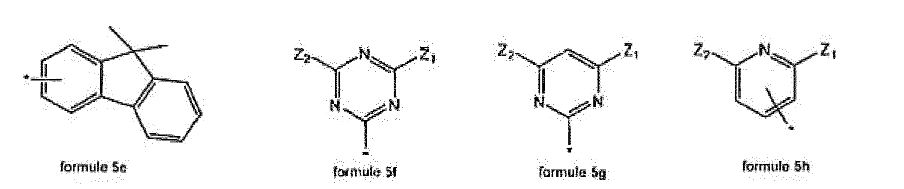 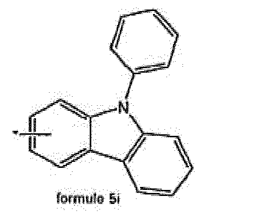 dans lequel Z₁ et Z₂ représentent chacun indépendamment un atome d'hydrogène, un atome de deutérium, un groupe alkyle en C₁ à C₂₀ substitué ou non substitué, un groupe aryle en C₅ à C₂₀ substitué ou non substitué, un groupe hétéroaryle en C₃ à C₂₀ substitué ou non substitué ou un groupe polycyclique condensé en C₆ à C₂₀ substitué ou non substitué ; et * indique un site de liaison.

8. Composé hétérocyclique selon l'une quelconque des revendications précédentes, dans lequel Ar₃ pour X dans la formule 1 représente un groupe arylène en C₅ à C₂₀ substitué ou non substitué, un groupe hétéroarylène en C₃ à C₂₀ substitué ou non substitué ou un groupe polycyclique condensé en C₆ à C₂₀ substitué ou non substitué.

9. Composé hétérocyclique selon l'une quelconque des revendications précédentes, dans lequel Ar₃ pour X dans la formule 1 comprend un groupe représenté par l'une des formules 6a à 6e ci-dessous : 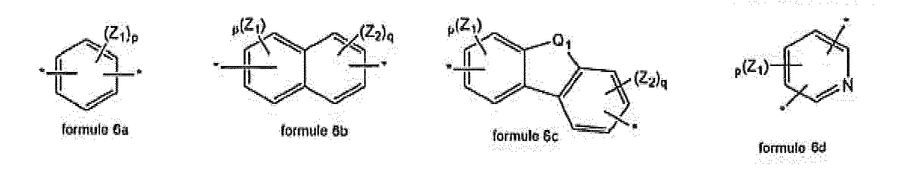 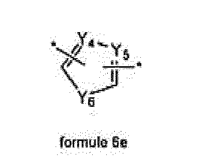 dans lequel dans les formules 6a à 6e Q₁ représente un groupe de liaison représenté par -C(R₆) (R₇)-, -N(R₆)- ou -S- ;
Y₄, Y₅ et Y₆ représentent chacun indépendamment un groupe de liaison représenté par -N= ou -C(R₈)=, -S- ou -O- ;
Z₁, Z₂, R₆, R₇ et R₈ représentent chacun indépendamment un atome d'hydrogène, un atome de deutérium, un groupe alkyle en C₁ à C₂₀ substitué ou non substitué, un groupe aryle en C₅ à C₂₀ substitué ou non substitué, un groupe hétéroaryle en C₃ à C₂₀ substitué ou non substitué, un groupe polycyclique condensé en C₆ à C₂₀ substitué ou non substitué, un atome d'halogène, un groupe cyano, un groupe nitro, un groupe hydroxyle ou un groupe carboxy ;
p représente un nombre entier de 1 à 8 ;
q représente un nombre entier de 1 à 8 ; et
* indique un site de liaison.

10. Composé hétérocyclique selon l'une quelconque des revendications précédentes, dans lequel n est égal à 1 ou 2.

11. Composé hétérocyclique selon l'une quelconque des revendications précédentes, dans lequel X dans la formule 1 comprend un groupe représenté par l'une des formules 7a à 7j : 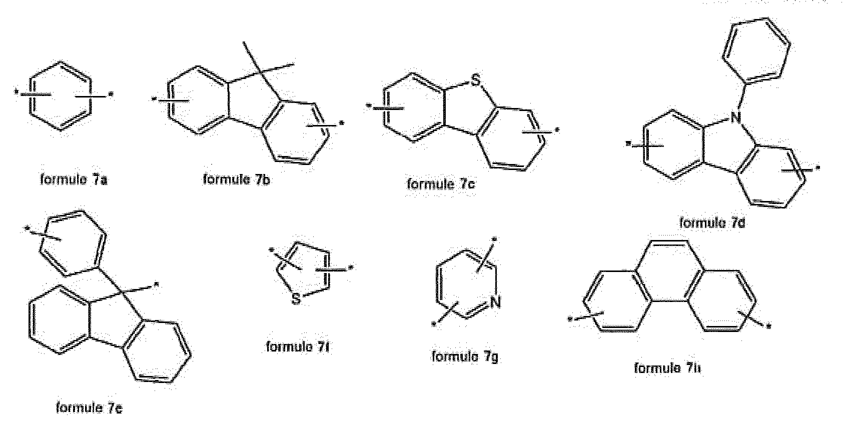 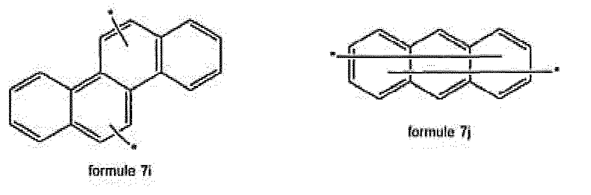 dans lequel dans les formules 7a à 7j, * indique un site de liaison.

12. Composé hétérocyclique selon l'une quelconque des revendications précédentes, représenté par l'un des composés ci-dessous:

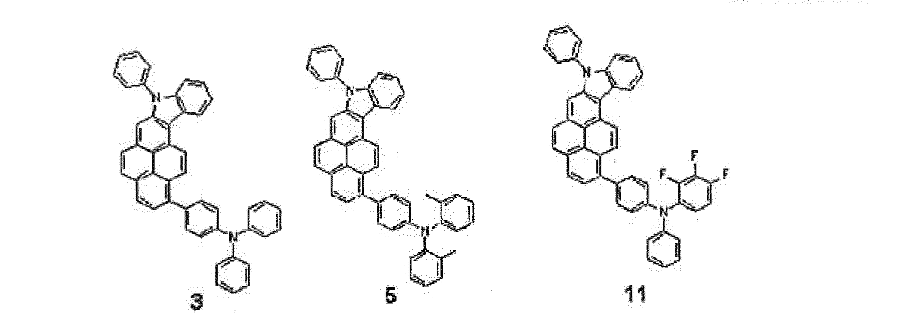

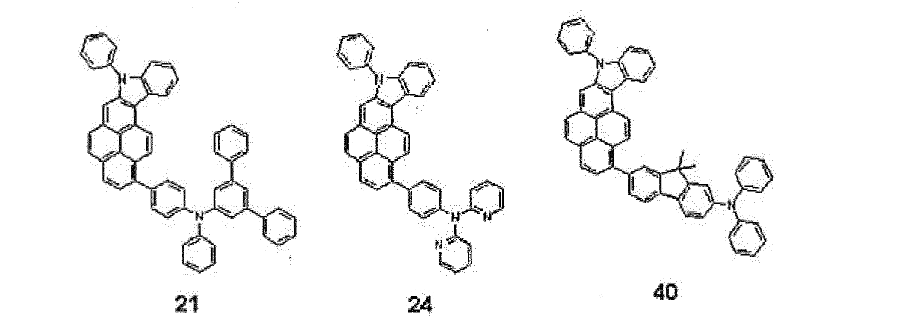

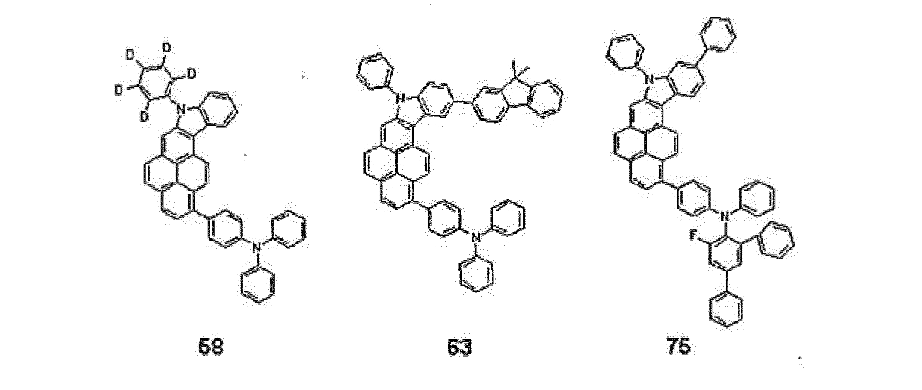

13. Dispositif électroluminescent organique comprenant :
une première électrode ;
une seconde électrode ; et
une couche organique entre la première électrode et la seconde électrode,
la couche organique comprenant une première couche incluant le composé hétérocyclique représenté par la formule 1 selon l'une quelconque des revendications 1 à 12.

14. Dispositif électroluminescent organique selon la revendication 13, dans lequel la première couche comprend au moins une couche d'injection de trous, une couche de transport de trous, une couche fonctionnelle ayant à la fois des capacités d'injection de trous et de transport de trous, une couche d'injection d'électrons, une couche de transport d'électrons, une couche fonctionnelle ayant à la fois des capacités d'injection d'électrons et de transport d'électrons ou des combinaisons de celles-ci.

15. Dispositif électroluminescent organique selon la revendication 13 ou 14, dans lequel la première couche comprend une couche d'émission, et un composé hétérocyclique de formule 1 est utilisé dans la couche d'émission en tant qu'hôte ou dopant pour un dispositif fluorescent ou phosphorescent.

16. Dispositif électroluminescent organique selon l'une quelconque des revendications 13 à 15, dans lequel la première couche comprend une couche d'émission bleue et un composé hétérocyclique de formule 1 en tant que dopant bleu.

17. Dispositif d'affichage à panneau plat comprenant le dispositif électroluminescent organique selon l'une quelconque des revendications 13 à 16, dans lequel la première électrode du dispositif électroluminescent organique est électriquement reliée à une électrode de source ou une électrode de drain d'un transistor à film mince.
